# EUROPEAN PATENT SPECIFICATION

(11) **EP 2 799 557 B1**
(45) Date of publication and mention of the grant of the patent: **07.09.2016**
(21) Application number: 14170625.9
(22) Date of filing: 27.02.2009
(51) Int. Cl.: A61K 31/7105, C12Q 1/68

(54) **MiR-32 antagonists for increasing responsiveness of prostate cancer to apoptosis**
MiR-32 Antagonisten zur Erhöhung der Empfindlichkeit von Prostatakrebszellen für Apoptose
Antagonistes de miR-32 pour augmenter la sensibilité des cellules cancéreuses de la prostate à l'apoptose

(30) Priority: 28.02.2008 US 67518 P
(43) Date of publication of application: 05.11.2014
(62) Divisional of application: 09715064.3
(73) Proprietor: The Ohio State University Research Foundation, Columbus, OH 43201 (US); The Government of The United States of America, as represented by The Secretary, Department of Health and Human Services, Rockville, MD 20852-3804 (US)
(72) Inventor: Croce, Carlo M., Columbus, OH 43221 (US); Ambs, Stefan, Silver String, MD 20901 (US)
(74) Representative: Turner, Craig Robert

(56) References cited:
- WO-A2-2006/137941
- WO-A2-2007/081740
- US-A1- 2007 259 352
- PORKKA KATI P ET AL: "MicroRNA expression profiling in prostate cancer", CANCER RESEARCH, vol. 67, no. 13, July 2007 (2007-07), pages 6130-6135, XP002631732, ISSN: 0008-5472

### SEQUENCE LISTING

<110> THE OHIO STATE UNIVERSITY RESEARCH FOUNDATION
<120> MICRORNA-BASED METHODS AND COMPOSITIONS FOR THE
   DIAGNOSIS, PROGNOSIS AND TREATMENT OF PROSTATE
   RELATED DISORDERS
<130> 53-29927
<140>
   <141>
<150> 61/067,518
   <151> 2008-02-28
<160> 24
<170> PatentIn version 3.5
<210> 1
   <211> 19
   <212> DNA
   <213> Artificial Sequence
<220>
   <223> Description of Artificial Sequence: Synthetic oligonucleotide
<400> 1
   caagagctct ttgtcctgg 19
<210> 2
   <211> 19
   <212> DNA
   <213> Artificial Sequence
<220>
   <223> Description of Artificial Sequence: Synthetic oligonucleotide
<400> 2
   agcccacctt ctgtcctcg 19
<210> 3
   <211> 19
   <212> DNA
   <213> Artificial Sequence
<220>
   <223> Description of Artificial Sequence: Synthetic oligonucleotide
<400> 3
   agacggagtc ttgttctgt 19
<210> 4
   <211> 19
   <212> DNA
   <213> Artificial Sequence
<220>
   <223> Description of Artificial Sequence: Synthetic oligonucleotide
<400> 4
   ctaaataaga ttgttctta 19
<210> 5
   <211> 19
   <212> DNA
   <213> Artificial Sequence
<220>
   <223> Description of Artificial Sequence: Synthetic oligonucleotide
<400> 5
   cagggacaat ttgttattc 19
<210> 6
   <211> 19
   <212> DNA
   <213> Artificial Sequence
<220>
   <223> Description of Artificial Sequence: Synthetic oligonucleotide
<400> 6
   ttacaaatat gtgttcttt 19
<210> 7
   <211> 19
   <212> DNA
   <213> Artificial Sequence
<220>
   <223> Description of Artificial Sequence: Synthetic oligonucleotide
<400> 7
   cttttgttag atgttctgg 19
<210> 8
   <211> 19
   <212> DNA
   <213> Artificial Sequence
<220>
   <223> Description of Artificial Sequence: Synthetic oligonucleotide
<400> 8
   actgtcatga gtgttctga 19
<210> 9
   <211> 19
   <212> DNA
   <213> Artificial Sequence
<220>
   <223> Description of Artificial Sequence: Synthetic oligonucleotide
<400> 9
   caggtcttca gtgtcctcc 19
<210> 10
   <211> 19
   <212> DNA
   <213> Artificial Sequence
<220>
   <223> Description of Artificial Sequence: Synthetic oligonucleotide
<400> 10
   gtggtgcttt ttgttgttg 19
<210> 11
   <211> 19
   <212> DNA
   <213> Artificial Sequence
<220>
   <223> Description of Artificial Sequence: Synthetic oligonucleotide
<400> 11
   tgggaactta gagtcctca 19
<210> 12
   <211> 19
   <212> DNA
   <213> Artificial Sequence
<220>
   <223> Description of Artificial Sequence: Synthetic oligonucleotide
<400> 12
   gatgggcatc ttgtccccg 19
<210> 13
   <211> 19
   <212> DNA
   <213> Artificial Sequence
<220>
   <223> Description of Artificial Sequence: Synthetic oligonucleotide
<400> 13
   ggacctaggt gtgttctca 19
<210> 14
   <211> 19
   <212> DNA
   <213> Artificial Sequence
<220>
   <223> Description of Artificial Sequence: Synthetic oligonucleotide
<400> 14
   agctttcttg cggtccttt 19
<210> 15
   <211> 19
   <212> DNA
   <213> Artificial Sequence
<220>
   <223> Description of Artificial Sequence: Synthetic oligonucleotide
<400> 15
   cagtttattt ttgttctcc 19
<210> 16
   <211> 19
   <212> DNA
   <213> Artificial Sequence
<220>
   <223> Description of Artificial Sequence: Synthetic oligonucleotide
<400> 16
   aaagattgta ctgttctct 19
<210> 17
   <211> 19
   <212> DNA
   <213> Artificial Sequence
<220>
   <223> Description of Artificial Sequence: Synthetic oligonucleotide
<400> 17
   cttacttctg atgttcttt 19
<210> 18
   <211> 19
   <212> DNA
   <213> Artificial Sequence
<220>
   <223> Description of Artificial Sequence: Synthetic oligonucleotide
<400> 18
   acggggccac gtgtccttc 19
<210> 19
   <211> 19
   <212> DNA
   <213> Artificial Sequence
<220>
   <223> Description of Artificial Sequence: Synthetic oligonucleotide
<400> 19
   acagggccat gtgtcctgg 19
<210> 20
   <211> 15
   <212> DNA
   <213> Artificial Sequence
<220>
   <223> Description of Artificial Sequence: Synthetic oligonucleotide
<220>
   <221> modified_base
   <222> (3)..(3)
   <223> a, c, g, t, unknown or other
<220>
   <221> modified_base
   <222> (7)..(8)
   <223> a, c, g, t, unknown or other
<400> 20
   rgnacrnngt gttct 15
<210> 21
   <211> 14
   <212> DNA
   <213> Artificial Sequence
<220>
   <223> Description of Artificial Sequence: Synthetic oligonucleotide
<220>
   <221> modified_base
   <222> (2)..(2)
   <223> a, c, g, t, unknown or other
<220>
   <221> modified_base
   <222> (7)..(8)
   <223> a, c, g, t, unknown or other
<400> 21
   dnwcwtnntg tyct 14
<210> 22
   <211> 15
   <212> DNA
   <213> Artificial Sequence
<220>
   <223> Description of Artificial Sequence: Synthetic oligonucleotide
<220>
   <221> modified_base
   <222> (3)..(3)
   <223> a, c, g, t, unknown or other
<220>
   <221> modified_base
   <222> (6)..(7)
   <223> a, c, g, t, unknown or other
<220>
   <221> modified_base
   <222> (9)..(9)
   <223> a, c, g, t, unknown or other
<400> 22
   rgnacnnknt gttct 15
<210> 23
   <211> 15
   <212> DNA
   <213> Artificial Sequence
<220>
   <223> Description of Artificial Sequence: Synthetic oligonucleotide
<220>
   <221> modified_base
   <222> (7)..(9)
   <223> a, c, g, t, unknown or other
<400> 23
   ggtwcwnnnt gttct 15
<210> 24
   <211> 15
   <212> DNA
   <213> Artificial Sequence
<220>
   <223> Description of Artificial Sequence: Synthetic oligonucleotide
<220>
   <221> modified_base
   <222> (8)..(9)
   <223> a, c, g, t, unknown or other
<400> 24
   rgaacasnnt gttct 15

## Description

### TECHNICAL FIELD

This invention relates generally to the field of molecular biology. Certain aspects of the invention include application in diagnostics, therapeutics, and prognostics of prostate related disorders.

### BACKGROUND

Expression profiles derived from gene microarrays have provided new insights into the biology of prostate cancer. Patterns of mRNA expression in prostate tumors have been associated with Gleason score, aggressive tumor subtypes, and disease recurrence. Additionally, mRNA expression signatures derived from primary tumors have lead to the discovery of novel candidate diagnostic markers, e.g., a-methylacyl-CoA racemase, for the early detection of prostate cancer. These findings demonstrate that gene expression profiles of resected tumors offer the opportunity to identify new diagnostic and prognostic markers for prostate cancer.

Recently, a new class of small RNAs has been described, termed microRNAs, which was found to regulate mRNA function by modulating both mRNA stability and the translation of mRNA into protein. MicroRNA genes are expressed as large precursor RNAs, called pri-mRNAs, which may encode multiple microRNAs in a polycistronic arrangement. These precursors are converted into a mature microRNA of 19 to 25 nucleotides by the nuclear RNase III enzyme, Drosha, and the cytosolic RNase III enzyme, Dicer. These two enzymes and their cofactors, e.g., DGCR8/Pasha, TRBP, and EIF2C2/argonaute-2, are key components of microRNA processing activity. Changes to their expression levels can alter cell function and induce cellular transformation.

A crucial role of microRNAs in cancer has been demonstrated. Their expression is commonly altered in solid human tumors. MicroRNA expression profiles also classify tumors by developmental lineage and differentiation state. Multiple microRNAs have been shown to have oncogenic properties, or act like tumor suppressor genes. These microRNAs have been termed oncomiRs. An alteration in their expression is causatively linked to cancer development.

In spite of considerable research into therapies to treat these diseases, they remain difficult to diagnose and treat effectively, and the mortality observed in patients indicates that improvements are needed in the diagnosis, treatment and prevention of prostate cancer.

WO 2007/081740 discloses miRNAs differentially expressed in prostate cancer, among them miR-32.

### SUMMARY

Accordingly the present invention provides use of an isolated miR-32 gene product comprising a miR antagonist for increasing the responsiveness of prostate cancer cells to apoptotic stimuli as set out in claim 1. Preferred features are defined in claims 2 to 4.

Disclosed herein is a method of diagnosing whether a subject has, or is at risk for developing a prostate-related disorder, determining a prognosis of a subject with prostate related disorder, and/or treating a prostate related disorder in a subject who has the prostate related disorder, comprising: measuring the level of at least one biomarker in a test sample from the subject, wherein an alteration in the level of the biomarker in the test sample, relative to the level of a corresponding biomarker in a control sample, is indicative of the subject either having, or being at risk for developing, the disorder.

In certain embodiments, the level of the at least one biomarker in the test sample is less than the level of the corresponding biomarker in the control sample.

In certain embodiments, the level of the at least one biomarker in the test sample is greater than the level of the corresponding biomarker in the control sample.

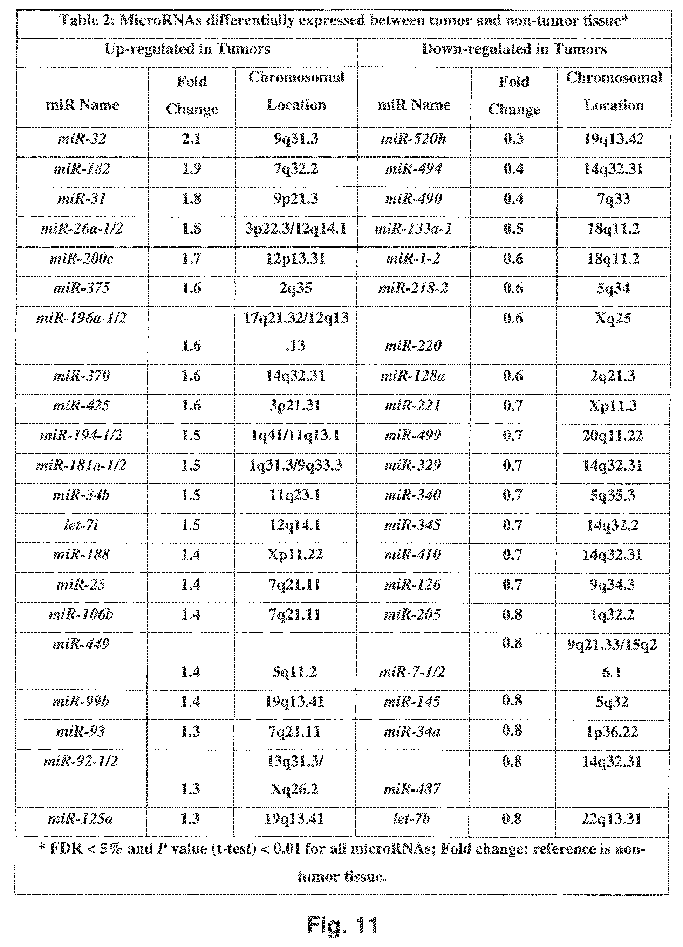

In certain embodiments, the at least one biomarker differentially expressed between tumor tissue and non-tumor tissue, and is one or more of the miRs, or functional variants thereof, listed in **Figure 11** **- Table 2.**

In certain embodiments, the at least one biomarker is selected from one or more miRs or functional variants thereof, listed in **Figure 11** **- Table 2,** that are upregulated in prostate tumors: miR-32, miR-182, miR-31, miR-26a-1/2, miR-200c, miR-375, miR-196a-1/2, miR-370, miR-425, miR-194-1/2, miR-181a-1/2, miR-34b, let-7i, miR-188, miR-25, miR-106b, miR-449, miR-99b, miR-93, miR-92-1/2, miR-125a.

In certain embodiments, the at least one biomarker is selected from one or more miRs, or functional variants thereof, listed in **Figure 11** **- Table 2,** that are down-regulated in prostate tumors: miR-520h, miR-494, miR-490, miR-133a-1, miR-1-2, miR-218-2,miR-220, miR-128a, miR-221, miR-499, miR-329, miR-340, miR-345, miR-410, miR-126, miR-205, miR-7-1/2, miR-145, miR-34a, miR-487, let-7b.

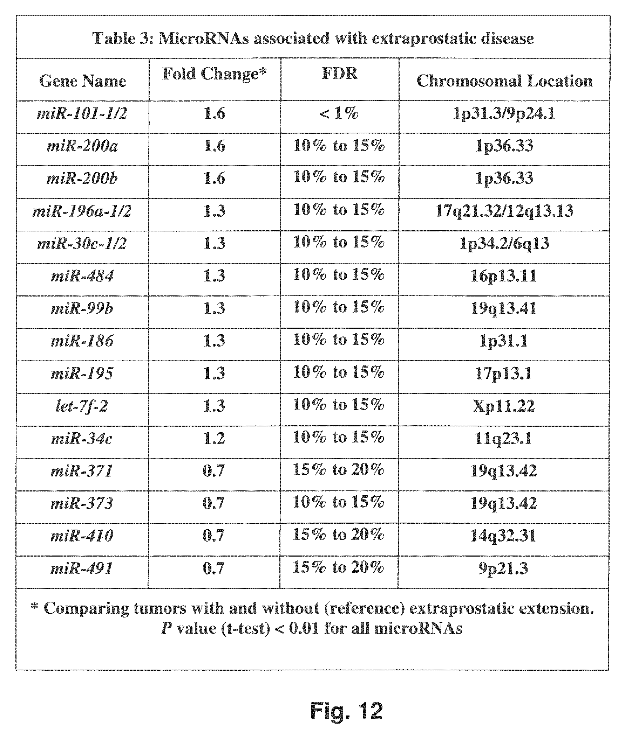

In certain embodiments, the at least one biomarker is associated with extraprostatic disease, and is selected from one or more of the miRs, or functional variants thereof, listed in **Figure 12** **- Table 3:** miR-101-1/2, miR-200a, miR-200b, miR-196a-1/2, miR-30c-1/2, miR-484, miR-99b, miR-186, miR-195, let- 7f-2, miR-34c, miR-371, miR-373, miR-410 and miR-491.

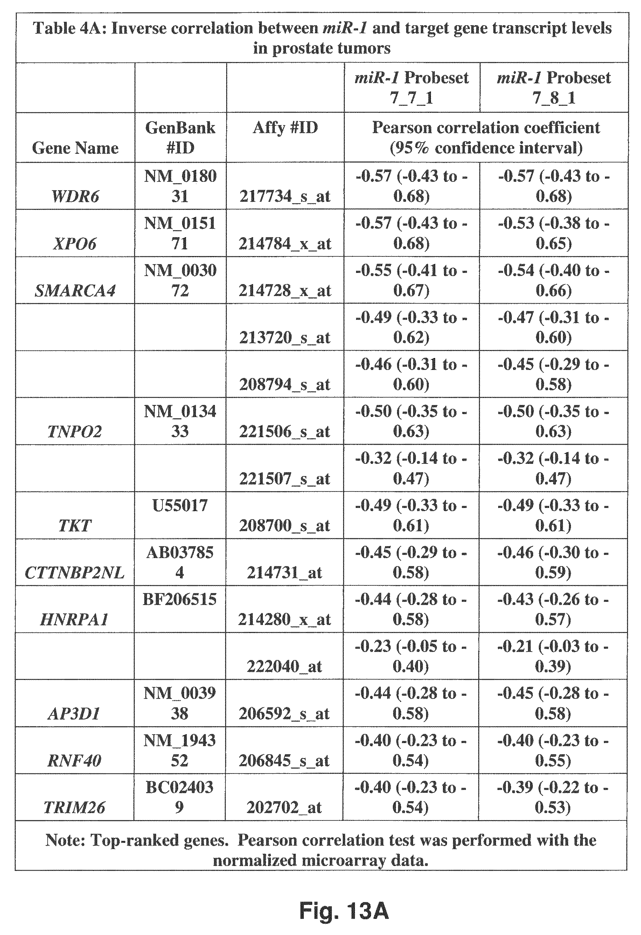

In certain embodiments, the at least one biomarker shows an inverse correlation between miR-1 and target gene transcript levels in prostate tumors, and is selected from is one or more of the genes, or functional variants thereof, listed in **Figure 13A** **- Table 4A.**

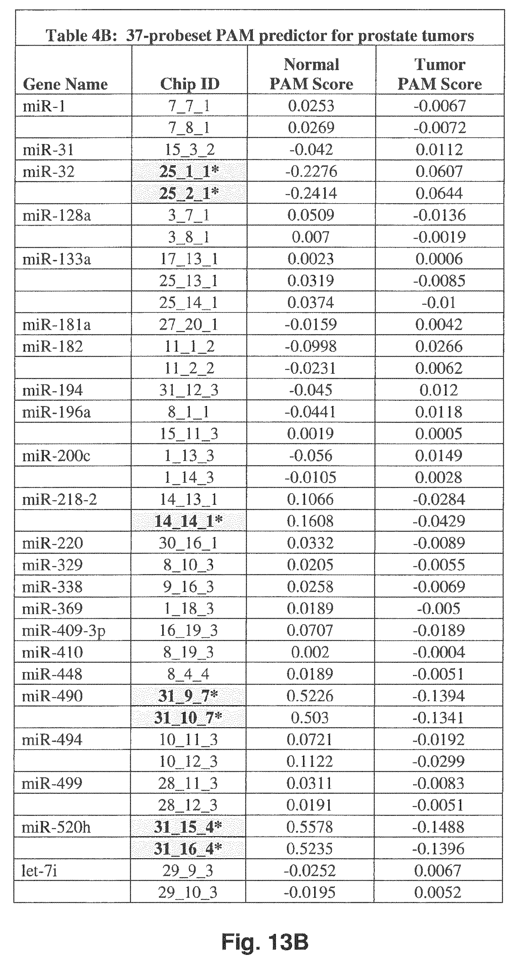

In certain embodiments, the the at least one biomarker is selected from one or more of the miRs, or functional variants thereof, listed in **Figure 13B****- Table 4B:** miR-1, miR-31, miR-32, miR-128a, miR-133a, miR-181a, miR-182, miR-194, miR-196a, miR-200c, miR-218-2, miR-220, miR-329, miR-338, miR-369, miR-409-3p, miR-410, miR-448, miR-490, miR-494, miR-499, miR-520h, and let-7i.

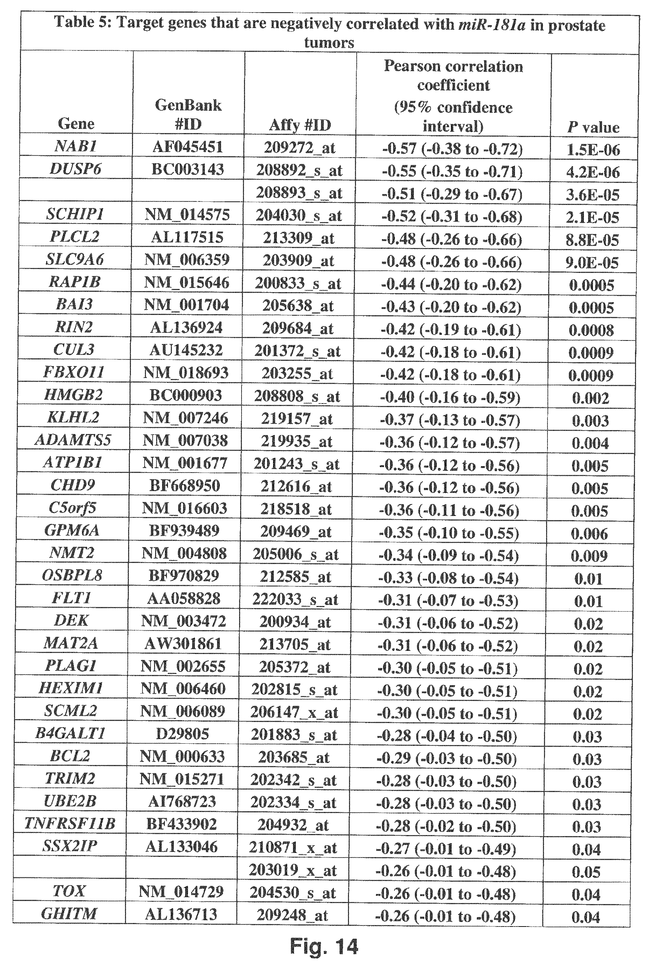

The method disclosed herein comprises a probe set showing a negative correlation with miR-181a in prostate tumors, wherein the probe set includes one or more of the genes, or functional variants thereof, listed in **Figure 14** **- Table 5.**

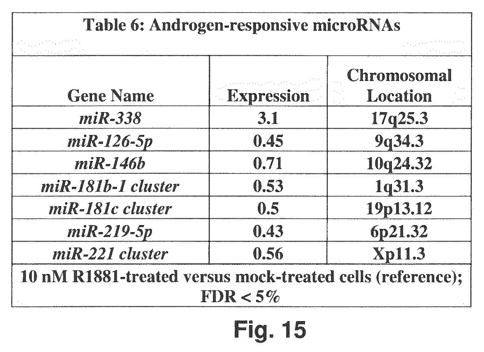

In certain embodiments, the at least one biomarker is an androgen-responsive biomarker, and is selected from one or more of the miRs, or functional variants thereof, listed in **Figure 15** **- Table 6:** miR-338, miR-126-5p, miR-181b-1 cluster, miR181c cluster, miR-219-5p, and miR221 cluster.

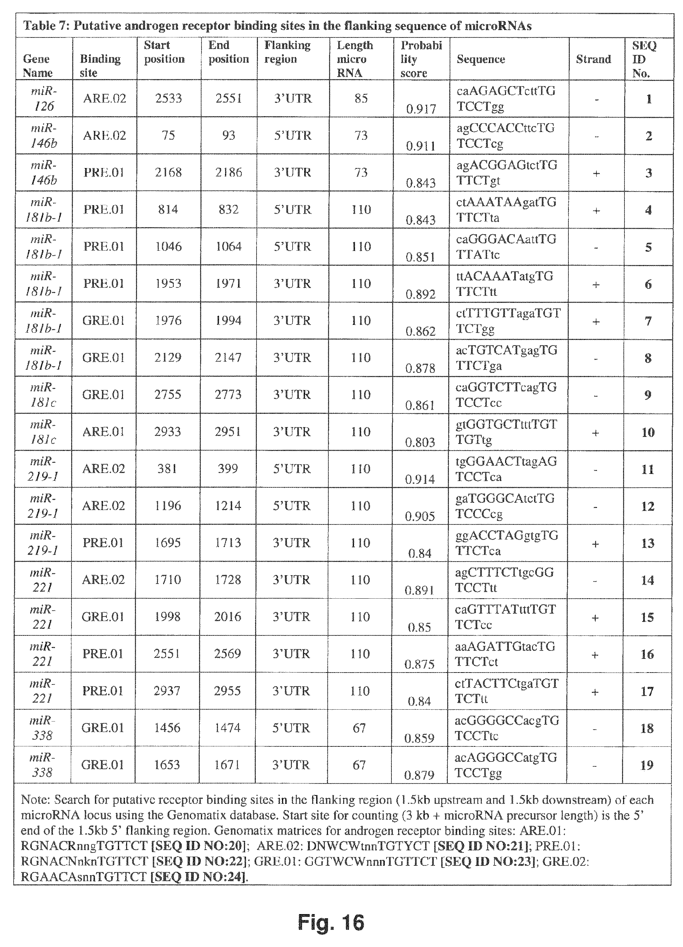

In certain embodiments, the at least one biomarker is selected from one or more of the miRs, or functional variants thereof, listed in **Figure 16** **- Table 7:** miR-126, miR-146b, miR-146b, miR-181b-1, miR-181b-1, miR-181b-1, miR-181b-1, miR-181b-1, miR-181c, miR-181c, miR-219-1, miR-219-1, miR-219-1, miR-221, miR-221, miR-221, miR-221, miR-338, miR-338.

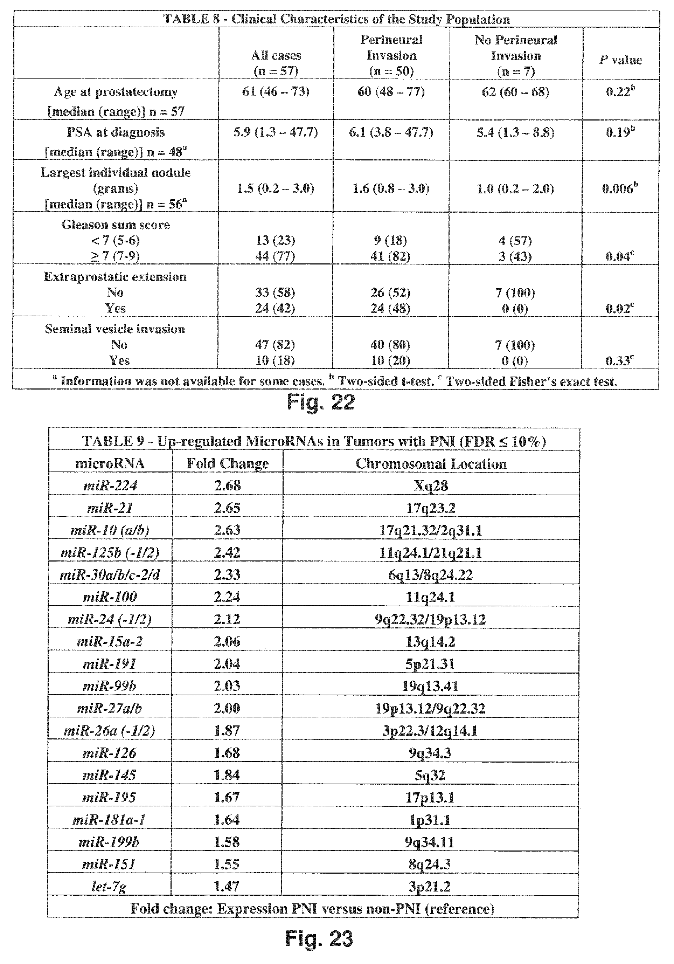

In certain embodiments, the at least one biomarker is selected from one or more of the miRs, or functional variants thereof, that are up-regulated in tumors with perineural invasion in prostate cancers, listed in **Figure 23** **- Table 9:** miR-224, miR-21, miR-10 (a/b), miR-125b (-1/2), miR-30a/b/c-2/d, miR-100, miR-24 (-1/2), miR-15a-2, miR-191, miR-99b, miR-27a/b, miR-26a (-1/2), miR-126, miR-145, miR-195, miR-181a-1, miR-199b, miR-151, let-7g.

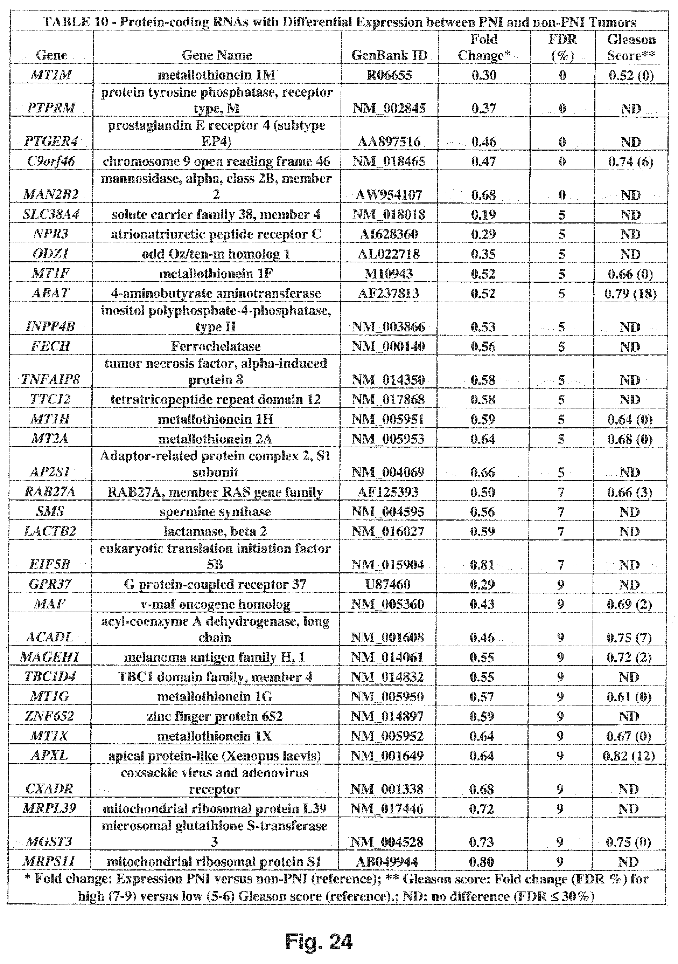

In certain embodiments, the at least one biomarker differentially expressed between PNT tumor tissue and non-PNT tumor tissue, and is one or more of the genes, or functional variants thereof, listed in **Figure 24** **- Table 12.**

The method disclosed herein comprises an increased expression of one or more of: Dicer and DGCR8 in prostate tumors, and/or Dicer and EIF2C2, which encodes argonuate-2, in tumors with a high Gleason score.

In certain embodiments, the sample comprises a blood sample.

In certain embodiments, the sample comprises one or more of serum or plasma blood samples.

Disclosed herein is a biomarker comprising at least one biomarker differentially expressed between tumor tissue and non-tumor tissue, and is one or more of the miRs, or functional variants thereof, listed in **Figure 11** **- Table 2.**

Disclosed herein is a biomarker comprising at least one biomarker is selected from one or more miRs or functional variants thereof, listed in **Figure 11** **- Table 2,** that are upregulated in prostate tumors: miR-32, miR-182, miR-31, miR-26a-1/2, miR-200c, miR-375, miR-196a-1/2, miR-370, miR-425, miR-194-1/2, miR-181a-1/2, miR-34b, let-7i, miR-188, miR-25, miR-106b, miR-449, miR-99b, miR-93, miR-92-1/2, miR-125a.

Disclosed herein is a biomarker comprising at least one biomarker is selected from one or more miRs, or functional variants thereof, listed in **Figure 11** **- Table 2,** that are down-regulated in prostate tumors: miR-520h, miR-494, miR-490, miR-133a-1, miR-1-2, miR-218-2,miR-220, miR-128a, miR-221, miR-499, miR-329, miR-340, miR-345, miR-410, miR-126, miR-205, miR-7-1/2, miR-145, miR-34a, miR-487, let-7b.

Disclosed herein is a biomarker comprising at least one biomarker is associated with extraprostatic disease, and is selected from one or more of the miRs, or functional variants thereof, listed in **Figure 12** **- Table 3:** miR-101-1/2, miR-200a, miR-200b, miR-196a-1/2, miR-30c-1/2, miR-484, miR-99b, miR-186, miR-195, let- 7f-2, miR-34c, miR-371, miR-373, miR-410 and miR-491.

Disclosed herein is a biomarker comprising one or more of the genes, or functional variants thereof, listed in **Figure 13A** **- Table 4A.**

Disclosed herein is a biomarker is selected from one or more of the miRs, or functional variants thereof, listed in **Figure 13B****- Table 4B:** miR-1, miR-31, miR-32, miR-128a, miR-133a, miR-181a, miR-182, miR-194, miR-196a, miR-200c, miR-218-2, miR-220, miR-329, miR-338, miR-369, miR-409-3p, miR-410, miR-448, miR-490, miR-494, miR-499, miR-520h, and let-7i.

Disclosed herein is a biomarker comprising a probe set showing a negative correlation with miR-181a in prostate tumors, wherein the probe set includes one or more of the genes, or functional variants thereof, listed in **Figure 14** **- Table 5.**

Disclosed herein is a biomarker comprising at least one biomarker is an androgen-responsive biomarker, and is selected from one or more of the miRs, or functional variants thereof, listed in **Figure 15** **- Table 6:** miR-338, miR-126-5p, miR-181b-1 cluster, miR181c cluster, miR-219-5p, and miR221 cluster.

Disclosed herein is a biomarker comprising at least one biomarker is selected from one or more of the miRs, or functional variants thereof, listed in **Figure 16** **- Table 7:** miR-126, miR-146b, miR-146b, miR-181b-1, miR-181b-1, miR-181b-1, miR-181b-1, miR-181b-1, miR-181c, miR-181c, miR-219-1, miR-219-1, miR-219-1, miR-221, miR-221, miR-221, miR-221, miR-338, miR-338.

Disclosed herein is a biomarker comprising at least one biomarker is selected from one or more of the miRs, or functional variants thereof, that are up-regulated in tumors with perineural invasion in prostate cancers, listed in **Figure 23** **- Table 9:** miR-224, miR-21, miR-10 (a/b), miR-125b (-1/2), miR-30a/b/c-2/d, miR-100, miR-24 (-1/2), miR-15a-2, miR-191, miR-99b, miR-27a/b, miR-26a (-1/2), miR-126, miR-145, miR-195, miR-181a-1, miR-199b, miR-151, let-7g.

Disclosed herein is a biomarker comprising at least one biomarker differentially expressed between PNT tumor tissue and non-PNT tumor tissue, and is one or more of the genes, or functional variants thereof, listed in **Figure 24** **- Table 12.**

Disclosed herein is a biomarker comprising an increased expression of one or more of: Dicer and DGCR8 in prostate tumors, and/or Dicer and EIF2C2, which encodes argonuate-2, in tumors with a high Gleason score.

Disclosed herein is a distinct microRNA expression signature in prostate tumors comprising alterations in the expression of one or more biomarkers that regulate tumor microRNA processing.

Disclosed herein in a method for influencing transcript abundance and/or protein expression of target mRNAs in the prostate, comprising deregulating one or more microRNAs in a subject in need thereof.

The method disclosed herein comprises inhibit the protein expression of cancer-related genes.

The method disclosed herein comprises altering expression of one or more of miR-32 and miR-106b to inhibit the protein expression of cancer-related genes.

In another aspect, there is described herein a use of a large-scale gene expression profiling of both microRNAs and protein-encoding RNAs to identify alterations in microRNA function that occur in human prostate tumors.

In another aspect, there is described herein a tumor gene signature for a prostate related disorder comprising: one or more of: up-regulated miR-32, followed by miR-182, miR-31, miR-26a, miR-200c, miR-196a; and the miR-106b-25 cluster; and/or one or more of significantly down-regulated miR-520h, miR-494, miR-490, and miR-1-133a cluster.

Disclosed herein is a tumor signature associated with extraprostatic disease extension at low margin of error, comprising miR-101.

In certain embodiments, the biomarker comprises host gene expression in prostate tumors that are increased in prostate tumors.

In certain embodiments, the biomarkers include one or more of: C9orf5 and MCM7 that are up-regulated, and whose expression is correlated with the expression of the intronic microRNAs, miR-32 and the miR-106b-25 cluster, respectively.

Disclosed herein is a use of miR-106b to target E2F1 and/or CDKN1A genes in prostate cancer cells and/or use in inhibiting protein expression of the E2F1 and/or CDKN1A genes.

Disclosed herein is regulation of one or more of XP06 and PTK9 by altering expression of miR-1 in prostate cancer cells.

Disclosed herein is a use of binding of microRNAs to 3'UTR sequences to lead to degradation and/or accumulation of the targeted mRNA in mammalian cells.

Disclosed herein is a use of an inverse and/or a positive correlation between a microRNA and a mRNA in a human tissue predictive of a microRNA target gene.

Disclosed herein is a method for identifying mRNAs that are regulated by microRNAs, comprising conducting a correlation analysis of microRNA and mRNA expression in human tissue.

Disclosed herein is a miR-expression antisense inhibitor comprising one or more of miR-32 and miR-106b.

Disclosed herein is an oncomiR biomarker of a prostate disorder or disease, comprising one or more of: miR-1, miR-32, and miR-106b-25 cluster.

In another aspect, there is described herein a method for regulating protein expression in prostate cancer cells, comprising modulating the expression of one or more of: miR-1, miR-32, and the miR-106b-25 cluster in the prostate cancer cells.

Disclosed herein is a composition for repressing expression of one or more of exportin-6 and PTK9 in prostate cancer cells, the composition comprising miR-1, or a functional variant thereof.

Disclosed herein is a method for regulating one or more of E2F1 and p21/WAF1 protein levels in a subject in need thereof, comprising using miR-106b, or a functional variant thereof.

Disclosed herein is a composition comprising antisense miR-106b useful to increase p21/WAF1 and/or E2F1 protein levels in a prostate cancer cell in a subject in need thereof.

In certain embodiments, the method comprises determining the prognosis of a subject with prostate cancer, comprising measuring the level of at least one biomarker in a test sample from the subject, wherein: i) the biomarker is associated with an adverse prognosis in prostate cancer; and ii) an alteration in the level of the at least one biomarker in the prostate test sample, relative to the level of a corresponding biomarker in a control sample, is indicative of an adverse prognosis.

The method disclosed herein comprises diagnosing whether a subject has, or is at risk for developing, prostate cancer, comprising: (1) reverse transcribing RNA from a test sample obtained from the subject to provide a set of target oligodeoxynucleotides; (2) hybridizing the target oligodeoxynucleotides to a microarray comprising miRNA-specific probe oligonucleotides to provide a hybridization profile for the test sample; and (3) comparing the test sample hybridization profile to a hybridization profile generated from a control sample, wherein an alteration in the signal of at least one miRNA is indicative of the subject either having, or being at risk for developing, prostate cancer.

In certain embodiments, the signal of at least one miRNA, relative to the signal generated from the control sample, is down-regulated, and/or wherein the signal of at least one miRNA, relative to the signal generated from the control sample, is up-regulated.

In certain embodiments, an alteration in the signal of at least one biomarker selected from the group listed in: **Table 2, Table 3, Table 4A, Table 4B, Table 5, Table 6, Table 7, Table 9** or **Table 10** are indicative of the subject either having, or being at risk for developing, a prostate cancer with an adverse prognosis.

Disclosed herein is a method of treating prostate cancer in a subject who has a prostate cancer in which at least one biomarker is down-regulated or up-regulated in the cancer cells of the subject relative to control cells, comprising: (1) when the at least one biomarker is down-regulated in the cancer cells, administering to the subject an effective amount of at least one isolated biomarker, or an isolated variant or biologically-active fragment thereof, such that proliferation of cancer cells in the subject is inhibited; or (2) when the at least one biomarker is up-regulated in the cancer cells, administering to the subject an effective amount of at least one compound for inhibiting expression of the at least one biomarker, such that proliferation of cancer cells in the subject is inhibited.

Disclosed herein is a method of treating prostate cancer in a subject, comprising: (1) determining the amount of at least one biomarker in prostate cancer cells, relative to control cells; and (2) altering the amount of biomarker expressed in the prostate cancer cells by: (i) administering to the subject an effective amount of at least one isolated biomarker, if the amount of the biomarker expressed in the cancer cells is less than the amount of the biomarker expressed in control cells; or (ii) administering to the subject an effective amount of at least one compound for inhibiting expression of the at least one biomarker, if the amount of the biomarker expressed in the cancer cells is greater than the amount of the biomarker expressed in control cells.

In another aspect, there is described herein a pharmaceutical composition for treating prostate cancer, comprising at least one isolated biomarker, and a pharmaceutically-acceptable carrier.

In certain embodiments, the pharmaceutical composition includes wherein the at least one isolated biomarker corresponds to a biomarker that is down-regulated in prostate cancer cells relative to control cells.

In certain embodiments, the pharmaceutical comprises at least one miR expression-inhibitor compound and a pharmaceutically-acceptable carrier.

In another aspect, there is described herein a method of identifying an anti-prostate cancer agent, comprising providing a test agent to a cell and measuring the level of at least one biomarker associated with decreased expression levels in prostate cancer cells, wherein an increase in the level of the biomarker in the cell, relative to a control cell, is indicative of the test agent being an anti-prostate prostate cancer agent.

In another aspect, there is described herein a method of identifying an anti-prostate cancer agent, comprising providing a test agent to a cell and measuring the level of at least one biomarker associated with increased expression levels in prostate cancer cells, wherein a decrease in the level of the biomarker in the cell, relative to a control cell, is indicative of the test agent being an anti-prostate cancer agent.

In another aspect, there is described herein a method of assessing the effectiveness of a therapy to prevent, diagnose and/or treat a prostate cancer associated disease, comprising: i) subjecting an animal to a therapy whose effectiveness is being assessed, and ii) determining the level of effectiveness of the treatment being tested in treating or preventing the disease, by evaluating at least one biomarker listed in one or more of **Table 2, Table 3, Table 4A, Table 4B, Table 5, Table 6, Table 7, Table 9** or **Table 10.**

In certain embodiments, the candidate therapeutic agent comprises one or more of: pharmaceutical compositions, nutraceutical compositions, and homeopathic compositions.

In certain embodiments, the therapy being assessed is for use in a human subject.

Disclosed herein is an article of manufacture comprising: at least one capture reagent that binds to a marker for a prostate cancer associated disease comprising at least one biomarker listed in one or more of **Table 2, Table 3, Table 4a, Table 4B, Table 5, Table 6, Table 7, Table 9 or Table 10.**

Disclosed herein is a kit for screening for a candidate compound for a therapeutic agent to treat a prostate cancer associated disease, wherein the kit comprises: one or more reagents of at least one biomarker listed in one or more of **Table 2, Table 3, Table 4A, Table 4B, Table 5, Table 6, Table 7, Table 9** or **Table 10,** and a cell expressing at least one biomarker.

In certain embodiments, the presence of the biomarker is detected using a reagent comprising an antibody or an antibody fragment which specifically binds with at least one biomarker.

Disclosed herein is a use of an agent that interferes with a prostate cancer associated disease response signaling pathway, for the manufacture of a medicament for treating, preventing, reversing or limiting the severity of the disease complication in an individual, wherein the agent comprises at least one biomarker listed in one or more of **Table 2, Table 3, Table 4A, Table 4B, Table 5, Table 6, Table7, Table 9** or **Table 10.**

Disclosed herein is a method of treating, preventing, reversing or limiting the severity of a prostate cancer associated disease complication in an individual in need thereof, comprising: administering to the individual an agent that interferes with at least a prostate cancer associated disease response cascade, wherein the agent comprises at least one biomarker listed in one or more of **Table 2, Table 3, Table 4A, Table 4B, Table 5, Table 6, Table 7, Table 9** or **Table 10.**

Disclosed herein is a use of an agent that interferes with at least a prostate cancer associated disease response cascade, for the manufacture of a medicament for treating, preventing, reversing or limiting the severity of a prostate cancer-related disease complication in an individual, wherein the agent comprises at least one biomarker listed in one or more of **Table 2, Table 3, Table 4A, Table 4B, Table 5, Table 6, Table 7, Table 9** or **Table 10.**

Disclosed herein is a composition comprising an antisense inhibitor of one or more of miR-1, miR-32 and miR-106b.

Disclosed herein is a method of treating a prostate disorder in a subject in need thereof, comprising administering to a subject a therapeutically effective amount of the composition.

In certain embodiments, the composition is administered prophylactically.

In certain embodiments, administration of the composition delays the onset of one or more symptoms of the disorder.

In certain embodiments, administration of the peptide inhibits development of prostate cancer.

In certain embodiments, administration of the peptide inhibits tumor growth.

In certain embodiments, administration of the peptide inhibits infection.

In another aspect, there is described herein a method for detecting the presence of prostate cancer in a biological sample, the method comprising: a) exposing the biological sample suspected of containing prostate cancer to a marker therefor; and b) detecting the presence or absence of the marker, if any, in the sample.

In certain embodiments, the marker includes a detectable label.

In certain embodiments, the method further comprises comparing the amount of the marker in the biological sample from the subject to an amount of the marker in a corresponding biological sample from a normal subject.

In certain embodiments, the method further comprises collecting a plurality of biological samples from a subject at different time points and comparing the amount of the marker in each biological sample to determine if the amount of the marker is increasing or decreasing in the subject over time.

Disclosed herein is a method for treating a prostate cancer in a subject, the method comprising: administering to the subject in need thereof a therapeutically effective amount of a prostate receptor agonist.

In certain embodiments, the receptor agonist is an antisense inhibitor of one or more of: miR1, miR-21 and miR-106b.

Disclosed herein is a use, to manufacture a drug for the treatment of prostate cancer, comprised of a nucleic acid molecule chosen from among the miR shown in **Table 2, Table 3, Table 4A, Table 4B, Table 5, Table 6, Table 7, Table 9** or **Table 10,** a sequence derived therefrom, a complementary sequence from such miR and a sequence derived from such a complementary sequence.

In certain embodiments, the use includes wherein the drug comprises a nucleic acid molecule presenting a sequence chosen from among: miRs listed in **Table 2,** a sequence derived from such miRs, the complementary sequence of such miRs, and a sequence derived from such a complementary sequence.

In another aspect, there is described herein an in vitro method to identify effective therapeutic agents or combinations of therapeutic agents to induce the differentiation of prostate cancer cells, the method comprising the stages of: i) culturing of cells derived from a prostate tumor, ii) adding at least one compound to the culture medium of the cell line, iii) analyzing the evolution of the level of expression of at least one miR between stages (i) and (ii) and iv) identifying compounds or combinations of compounds inducing a change in the level of expression of the miR between stages (i) and (ii).

In certain embodiments, the stage (iii) includes the analysis of the level of expression of at least one miR.

In certain embodiments, the stage (iv) includes the identification of the compounds or combinations of compounds modulating the level of expression of at least one miR.

In certain embodiments, the stage (iv) includes the identification of compounds or combinations of compounds reducing the level of expression of at least one miR.

In certain embodiments, the compound is a therapeutic agent for the treatment of cancer.

In another aspect, there is described herein a method for classifying a prostate tissue from a subject comprising: a) measuring the expression of one or more nucleic acid sequences selected from the group listed in **Table 2, Table 3, Table 4A, Table 4B, Table 5, Table 6, Table 7, Table 9** or **Table 10** in a test cell population, wherein at least one cell in said test cell population is capable of expressing one or more nucleic acid sequences selected from the group listed in **Table 2, Table 3, Table 4A, Table 4B, Table 5, Table 6, Table7, Table 9** or **Table 10;** b) comparing the expression of the nucleic acid sequence(s) to the expression of the nucleic acid sequence(s) in a reference cell population comprising at least one cell for which a prostate cancer classification is known; and c) identifying a difference, if present, in expression levels of one or more nucleic acid sequences selected from the group consisting, in the test cell population and reference cell population, thereby classifying the prostate cancer in the subject.

In certain embodiments, a difference in the expression of the nucleic acid(s) in the test cell population as compared to the reference cell population indicates that the test cell population has a different classification as the cells from the reference cell population.

In certain embodiments, a similar expression pattern of the nucleic acid(s) in the test cell population as compared to the reference cell population indicates that the test cell population has the same classification as the cells from the reference cell population.

In certain embodiments, the reference cell population is a plurality of cells or a database.

In certain embodiments, the reference cell population is selected from the group consisting of: a reference cell population classified as a cell population from normal prostate tissue, a reference cell population classified as a cell population from benign prostate tissue and a reference cell population classified as a cell population from malignant prostate tissue.

Various objects and advantages of this invention will become apparent to those skilled in the art from the following detailed description of the preferred embodiment, when read in light of the accompanying drawings.

### BRIEF DESCRIPTION OF THE DRAWINGS

The patent or application file may contain one or more drawings executed in color and/or one or more photographs. Copies of this patent or patent application publication with color drawing(s) and/or photograph(s) will be provided by the Patent Office upon request and payment of the necessary fee.

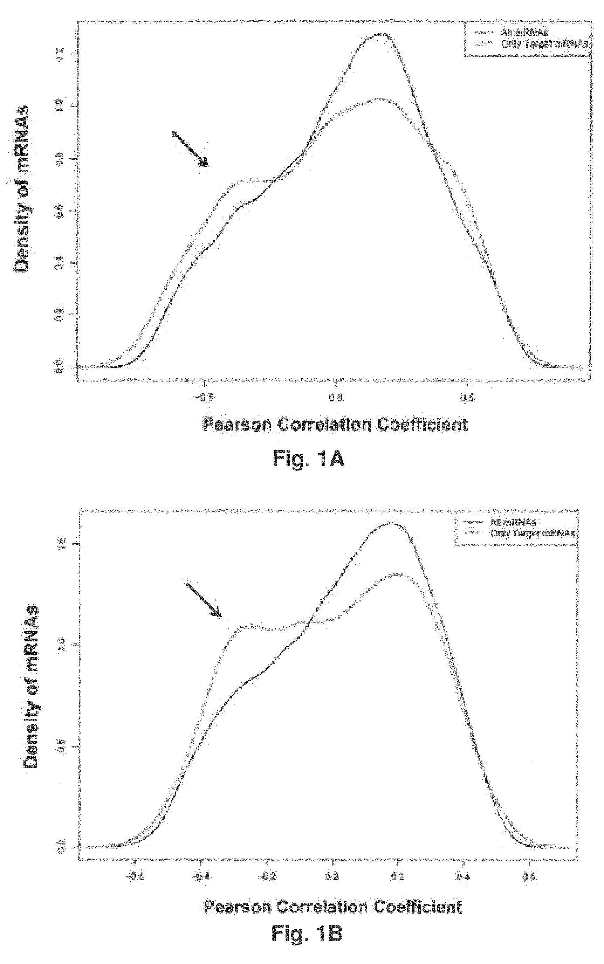

**Figures 1A-1B****:** Analysis of the relationship between transcript abundance of microRNAs and their respective target mRNAs in prostate tissue. Shown is the global distribution of the Pearson correlation coefficients between mRNAs and miR-106b **(****Fig. 1A****)** and between mRNAs and miR-181a **(****Fig. 1B****).** The black-lined curves show the distribution of the correlation coefficients for all mRNAs. The red-lined curves show the correlation coefficient distribution for only those mRNAs that are a predicted target of either miR-106b or miR-181a. The red-lined curves have an additional shoulder (arrow) indicating an enrichment of target mRNAs, whose transcript levels are negatively correlated with the transcript levels of the microRNA.

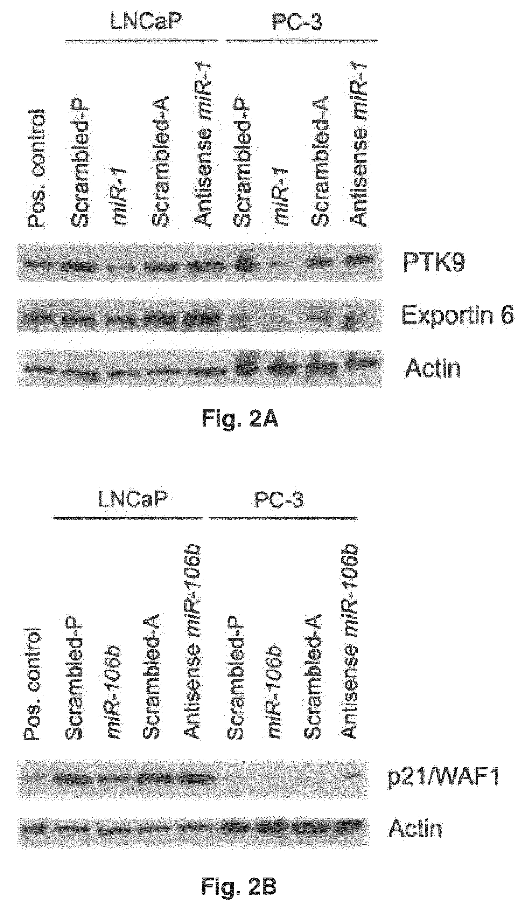

**Figures 2A-2B****:** Inhibition of protein expression by miR-1 and miR-106b. LNCaP and PC-3 human prostate cancer cells were transfected with either microRNA precursor (miR-1 and miR-106b) or antisense microRNA (antisense miR-1 and antisense miR-106b), or their respective vector controls, scrambled precursor microRNA (Scrambled-P) and scrambled antisense microRNA (Scrambled-A). Protein extracts were prepared 48 hours after transfection and protein expression was examined by Western blot analysis. Loading: 50 µg protein per lane.

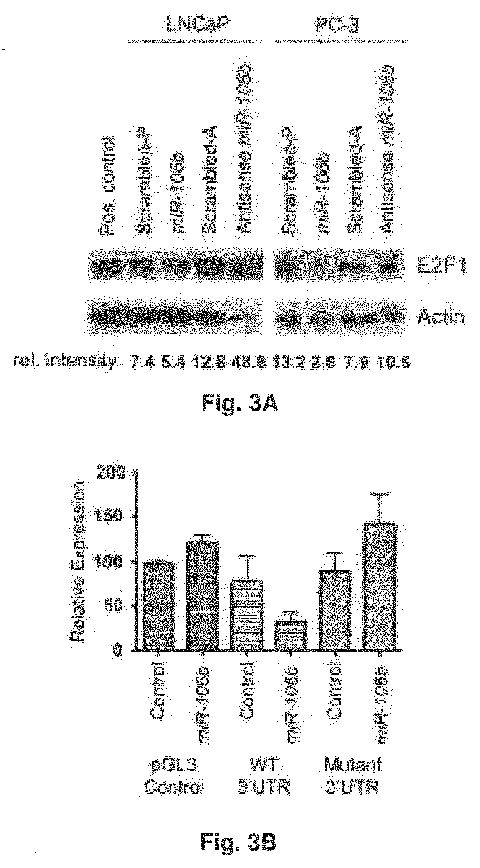

**Figures 3A-3B****:** miR-106b inhibits E2F1 protein expression by a 3'UTR-mediated mechanism.
**Figure 3A****:** LNCaP and PC-3 human prostate cancer cells were transfected with either microRNA precursor (miR-106b) or antisense microRNA (antisense miR-106b), or their respective vector controls, scrambled precursor microRNA (Scrambled-P) and scrambled antisense microRNA (Scrambled-A). Protein extracts were prepared 48 hours after transfection and protein expression was examined by Western blot analysis. To obtain the relative intensity values, E2F1 expression was normalized to β-actin.
**Figure 3B****:** pGL3 luciferase reporter constructs containing either the wild-type or mutant 3'UTR target sequence of miR-106b in the E2F1 gene were co-transfected into LNCaP cells with either precursor microRNA negative control or miR-106b precursor (each n = 3). For comparison, cells were also transfected with the pGL3 control vector that did not contain the 3'UTR. After 24 hours, luciferase activity was determined in the cell extracts. In the presence of the wild-type E2F1 3'UTR, transfection with precursor miR-106b lead to a significant inhibition of the luciferase reporter when compared with the vector control (P = 0.045, two-sided t-test).
**Figures 3C-3D****:** miR-32 inhibits Bim protein expression by a 3'UTR-mediated mechanism.
**Figure 3C****:** LNCaP and PC-3 human prostate cancer cells were transfected with either microRNA precursor (miR-32) or antisense microRNA (antisense miR-32), or their respective vector controls, scrambled precursor microRNA (Scrambled-P) and scrambled antisense microRNA (Scrambled-A). Protein extracts were prepared 48 hours after transfection and protein expression was examined by Western blot analysis. To obtain the relative intensity values, Bim expression was normalized to β-actin.

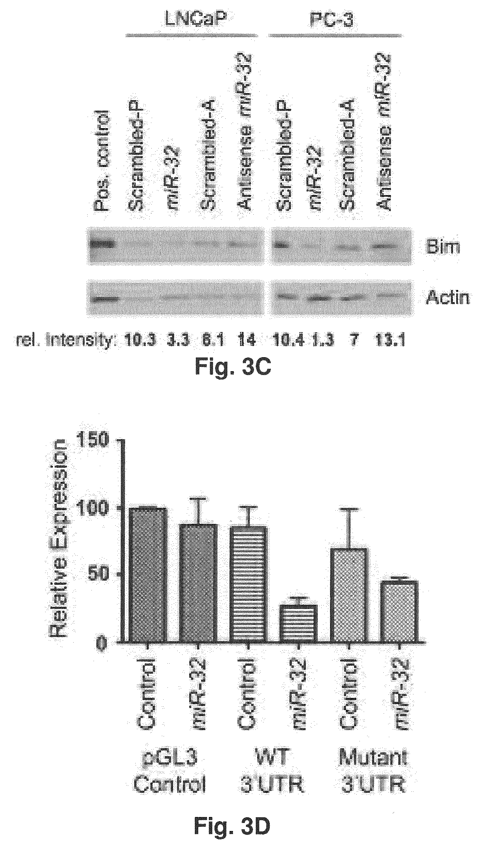

**Figure 3D****:** pGL3 luciferase reporter constructs containing either the wild-type or mutant 3 'UTR target sequence of miR-32 in the BCL2L11 (Bim) gene were co-transfected into LNCaP cells with either precursor microRNA negative control or miR-32 precursor (each n = 3). For comparison, cells were also transfected with the pGL3 control vector that did not contain the3'UTR. After 24 hours, luciferase activity was determined in the cell extracts. In the presence of the wild-type BCL2L11 3 'UTR, transfection with miR-32 lead to a significant inhibition of the luciferase reporter when compared with the vector control (P = 0.003, two-sided t-test). This inhibition was attenuated if the reporter construct contained a mutant 3 'UTR target sequence of miR.
**Figures 4A-4B****:** Quantitative RT-PCR expression analysis of DICER **(****Fig. 4A****)** and DGCR8 **(****Fig. 4B****).**

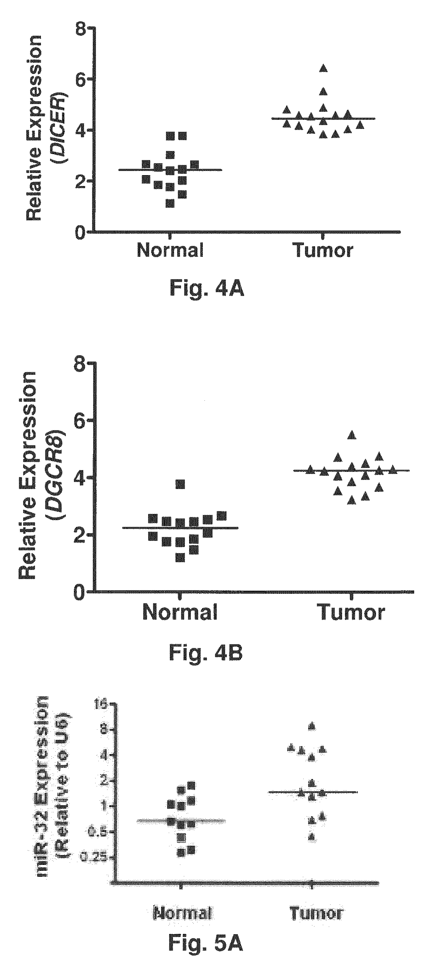

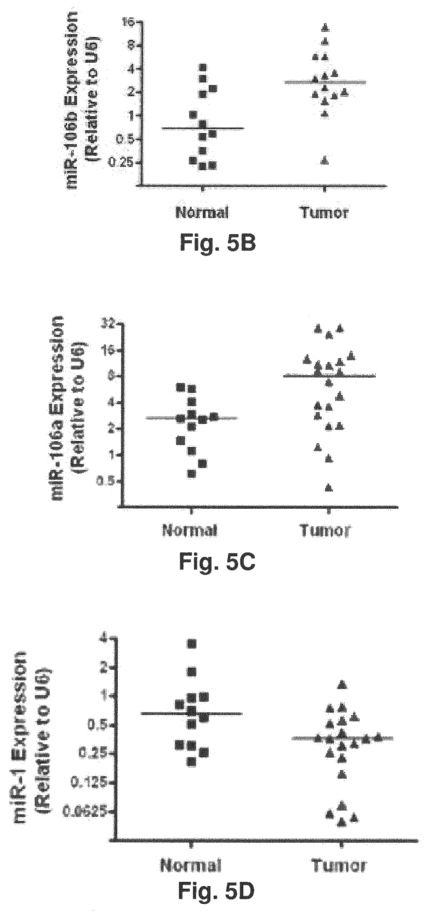

**Figures 5A-5D****:** Quantitative RT-PCR expression analysis of miR-32 **(****Fig. 5A****)**, miR-106b **(****Fig. 1B****),** miR-106a **(****Fig. 5C****)** and miR-1 **(****Fig. 5D****) in** nontumor prostate tissue (Normal) and tumors (Tumor) from prostate cancer patients. Plotted are the relative microRNA expression values for the individual samples and the median value for the sample set. MiR-32, miR-106b, and miR-106a are significantly higher expressed in tumor than in nontumor tissue: P = 0.037 (two-sided t-test) for miR-32; P = 0.009 (two-sided t-test) for miR-106b; P = 0.015 (two-sided t-test) for miR-106a.
**Figure 6****:** The relationship between XPO6 and miR-1 transcript levels in prostate tumors.
**Figure 7****:** miR-106b inhibits luciferase reporter activity by a *CDKN1A* (p21/WAF1) 3'UTR-mediated mechanism
**Figures 8A-8B****:** Significant inhibition of caspase-3/caspase-7 activation by miR cluster in anticancer drug-treated cells.
**Figures 9A 9B****:** qRT-PCR analysis of mature miR-338 and miR-221 showed that their expression level is androgen-regulated.
**Figure 10****:** Table 1: Clinical characteristics of the study population.
**Figure 11****:** Table 2: MicroRNAs differentially expressed between tumor and non-tumor tissue.
**Figure 12****:** Table 3: MicroRNAs associated with extraprostatic disease.
**Figure 13A****:** Table 4A: Inverse correlation between miR-1 and target gene transcript levels in prostate tumors.
**Figure 13B****:** Table 4B: 37-probeset PAM predictor for prostate tumors.
**Figure 14****:** Table 5: Target genes of miR-181a that are negatively correlated with miR-181a in prostate tumors.
**Figure 15****:** Table 6: Androgen-responsive microRNAs.
**Figure 16****:** Table 7: Putative androgen receptor binding sites in the flanking sequence of microRNAs.
**Figures 17-17B****:** Unsupervised hierarchical cluster analysis of 57 prostate tumors based on the expression of 235 microRNAs.
**Figure 17A****:** The microRNA expression yielded two prominent clusters with distinct microRNA profiles. Cluster #1 contained all non-PNI tumors.
**Figure 17B****:** Non-random distribution of tumors by PNI status among the two clusters (P = 0.002; two-sided Fisher's exact test).

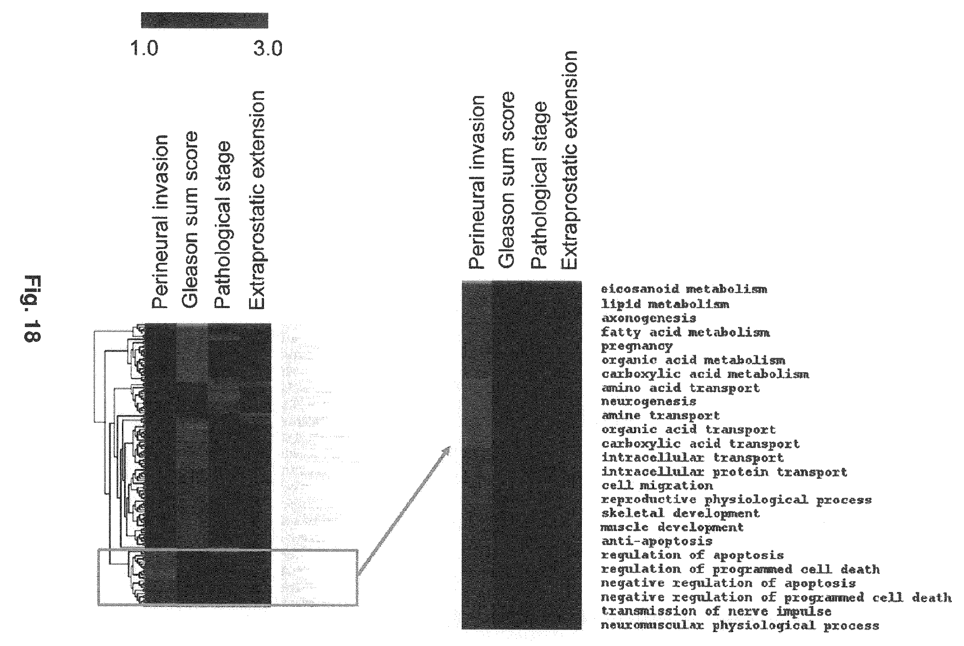

**Figure 18****:** Cluster analysis of Gene Ontology Biological Processes that are enriched for differently expressed genes comparing PNI tumors with non-PNI tumors. The results of a cluster analysis are displayed in a heatmap with the red color indicating an enrichment of differentially expressed genes in a biological process, e.g., eicosanoid metabolism, for a particular comparison, e.g., PNI tumor versus non-PNI tumor ("Perineural invasion"). The heatmap also shows the cluster analysis for the high (7-9) versus low (5-6) Gleason score comparison ("Gleason sum score"), the pT3 versus pT2 comparison ("Pathological stage"), and the positive versus negative extraprostatic extension comparison ("Extraprostatic extension"). Analysis revealed that gene expression differences are non-random and create unique patterns of frequently affected biological processes for the four comparisons. The enlarged cluster shows the biological processes that are uniquely enriched for differentially expressed genes comparing PNI tumors with non-PNI tumors. Eicosanoid metabolism, lipid metabolism, and axonogenesis are also enriched for differentially expressed genes comparing pT3 versus pT2.
**Figures 19A-19D****:** Expression of metallothionein in prostate tumors by immunohistochemistry. The panels show examples of metallothionein expression in the tumor epithelium. Marked cytoplasmic expression of metallothionein in cancer cells distant to neurons **(****Figure 19A****)** and absence of this expression in perineural cancer cells **(****Figure 19B****) in** the same tumor. The expression of metallothionein is decreased as tumor cells approach the nerve **(Figures 19C, 19D).** Arrow and "N" indicate the location of the brown stained nerve trunks. Counterstain: Methyl green.
**Figures 20A-20D****:** Expression of the coxsackie adenovirus receptor in prostate tumors by immunohistochemistry. The panels show examples of receptor expression in the tumor epithelium. Membranous and cytoplasmic staining for the receptor in cancer cells distant to neurons **(****Figure 20A****)** and in perineural cancer cells **(****Figure 20B****)** in the same tumor. The expression of the coxsackie adenovirus receptor is decreased in perineural cancer cells **(Figures 20C, 20D).** N: nerve trunk. Counterstain: Methyl green.
**Figures 21A-21D****:** *miR*-*224* in prostate tumors by in-situ hybridization. Shown are representative examples of cytoplasmic expression of *miR*-*224* in the tumor epithelium. The granular brown staining shows the presence of *miR*-*224*. Most tumors showed weak labeling for *miR*-*224* **(****Figure 21A****).** In a subset of tumors, moderate to strong *miR*-*224* labeling was observed in perineural cancer cells **(****Figures 21B, 21C, 21D****).** N = nerve trunk. Counterstain: Hematoxylin.
**Figure 22****: Table 8:** Clinical characteristics of the perineural invasion (PNI) study population.
**Figure 23** **- Table 9:** Up-regulated microRNAs in tumors with PNI (FDR ≤ 10%).
**Figure 24** **- Table 10:** Protein-coding RNAs with differential expression between PNI and non-PNI tumors.
**Figure 25** **- Table 11:** Validation of microarray results by qRT-PCR for selected genes.
**Figure 26** **- Table 12:** Biological processes most significantly enriched for differently expressed genes comparing PNI tumors with non-PNI tumors.

### DETAILED DESCRIPTION OF THE INVENTION

Before describing the present invention in detail, it is to be understood that this invention is not limited to particular formulations or process parameters as such may, of course, vary. It is also to be understood that the terminology used herein is for the purpose of describing particular embodiments of the invention only, and is not intended to be limiting.

Although a number of methods and materials similar or equivalent to those described herein can be used in the practice of the present invention, the preferred materials and methods are described herein.

MicroRNAs are small non-coding RNAs that regulate the expression of protein-coding genes. To evaluate the involvement of microRNAs in prostate cancer, we determined genome-wide expression of microRNAs and mRNAs in 60 primary prostate tumors and 16 non-tumor prostate tissues.

MicroRNA expression becomes altered with the development and progression of prostate cancer. Some of these microRNAs regulate the expression of cancer-related genes in prostate cancer cells.

As used herein interchangeably, a "miR gene product," "microRNA," "miR," or "miRNA" refers to the unprocessed or processed RNA transcript from a miR gene.

As used herein, "biomarker" can include one or more of a "miR gene product," "microRNA," "miR," or "miRNA," or a protein-encoding RNA.

The active 19-25 nucleotide RNA molecule can be obtained from the miR precursor through natural processing routes (e.g., using intact cells or cell lysates) or by synthetic processing routes (e.g., using isolated processing enzymes, such as isolated Dicer, Argonaut, or RNAse III). It is understood that the active 19-25 nucleotide RNA molecule can also be produced directly by biological or chemical synthesis, without having to be processed from the miR precursor. When a microRNA is referred to herein by name, the name corresponds to both the precursor and mature forms, unless otherwise indicated.

The present invention discloses methods of diagnosing whether a subject has, or is at risk for developing, a prostate related disorder. As used herein, a "subject" can be any mammal that has, or is suspected of having, prostate cancer.

The mRNA analysis revealed that key components of microRNA processing and several microRNA host genes, e.g., MCM7 and C9orf5, were significantly up-regulated in prostate tumors. Consistent with these findings, tumors expressed the miR-106b-25 cluster, which maps to intron 13 of MCM7, and miR-32, which maps to intron 14 of C9orf5, at significantly higher levels than non-tumor prostate.

The expression levels of other microRNAs, including miR-106b-25 cluster homologues and the miR-1-133a cluster, were also altered in prostate tumors.

Additional differences in microRNA abundance were found between organ-confined tumors and those with extraprostatic disease extension.

Also, we found evidence that the deregulation of microRNAs influences transcript abundance of protein-coding target genes in the prostate.

In cell culture, E2F 1 and p21/WAF 1 were identified as targets of miR-106b, Bim of miR-32, and exportin-6 and PTK9 of miR-1.

Gene-based classifiers can be powerful diagnostic and prognostic tools in improving disease diagnosis and for the prediction of clinical behavior. We used the PAM application to identify microRNA signatures that discriminate between tumor and nontumor tissue. PAM identified two microRNA signatures consisting of 7-probesets and 37-probests that best distinguished between tumor and non-tumor tissue **(****Figure 13B** **- Table 4B,** where the 7-probeset signature is indicated by *).

The 7-probeset signature achieved a correct classification of 14 (88%) out of 16 non-tumor tissues and 49 (82%) out of 60 tumors. This signature was based on the expression pattern of only four microRNAs, miR-32, miR-218-2, miR-490, and miR-520h.

Further improvement of the overall prediction accuracy was obtained with a 37-probeset signature that represented 23 microRNAs (Figure 11 [Table 5]). This signature completely overlapped with the 7-probeset signature. With the 37-probeset signature, PAM achieved a correct classification of 16 (100%) out of 16 non-tumor tissues and 48 (80%) out of 60 tumors.

The present invention is further explained in the following Examples, in which all parts and percentages are by weight and degrees are Celsius, unless otherwise stated. It should be understood that these Examples, while indicating preferred embodiments of the invention, are given by way of illustration only. From the above discussion and these Examples, one skilled in the art can ascertain the essential characteristics of this invention, and without departing from the spirit and scope thereof, can make various changes and modifications of the invention to adapt it to various usages and conditions.

### EXAMPLE I

### Clinical samples

Sixty fresh-frozen prostate tumors were received from the NCI Cooperative Prostate Cancer Tissue Resource (CPC'I'R) and the Department of Pathology at the University of Maryland (UMD). Written informed consent was obtained from all donors. The tumors were resected adenocarcinomas that had not received any therapy prior to prostatectomy. The macro-dissected tumor specimens were reviewed by a pathologist, who confirmed the presence of tumor in the frozen specimens. Surrounding non-tumor prostate tissue was collected from 16 patients with prostate cancer. All tissues were collected between 2002 and 2004. Information on race/ethnicity was either extracted from medical records (CPCTR) or obtained through an epidemiological questionnaire (UMD). Clinicopathological characteristics of the patients, including age at prostatectomy, histology, Gleason score, pathological stage, PSA at diagnosis, tumor size, extraprostatic extension, margin involvement, and seminal vesicle invasion were obtained from CPCTR. For UMD cases, this information was extracted from the medical and pathology records, if available. The study was approved by the institutional review boards of the participating institutions.

### RNA extraction

Total RNA was isolated using the TRIZOL reagent according to the manufacturer's instructions (Invitrogen, Carlsbad, CA). RNA integrity for each sample was confirmed with the Agilent 2100 Bioanalyzer (Agilent Technologies, Palo Alto, CA). Each RNA sample was then split into two aliquots that were either processed for the microRNA microarray or the mRNA microarray.

### Gene microarrays

Custom microRNA oligonucleotide chip. MicroRNA labeling and hybridization were performed as described previously. The microRNA microarray (Ohio State University Comprehensive Cancer Center, Version 3.0) contains probes spotted in quadruplicate for 329 human and 249 mouse microRNAs. More information about the array platform can be found in the ArrayExpress and GEO databases under the accession numbers A-MEXP-620 and GSE8126, respectively.

### Affymetrix GeneChip™

RNA labeling and the hybridization were performed according to Affymetrix standard protocols (Santa Clara, CA). Briefly, 5µg of total RNA was reverse transcribed with an oligo (dT) primer that has a T7 RNA polymerase promoter at the 5' end. Second-strand synthesis was followed by cRNA production with incorporation of biotinylated ribonucleotides using the BioArray High Yield RNA Transcript Labeling Kit T3 from Enzo Life Sciences (Farmingdale, NY). The labeled cRNA was fragmented and hybridized to Affymetrix GeneChip HG-U133A 2.0 arrays. This array contains 22,283 probe sets that represent approximately 13,000 human protein-coding genes. Hybridization signals were visualized with phycoerythrin-conjugated streptavidin (Invitrogen) and scanned using a GeneChip Scanner 3000 7G (Affymetrix). In accordance with Minimum Information About a Microarray Experiment (MIAME) guidelines, we deposited the CEL files for the microarray data and additional patient information into the GEO repository (ncbi.nlm.nih.zov/aeo/). The GEO submission accession numbers for the microRNA and mRNA profiling data are GSE8126 and GSE6956, respectively.

### Data normalization and statistical analysis of microarrays

Affymetrix chips were normalized using the robust multichip analysis (RMA) procedure. Median-centric normalization was used for the custom microRNA oligonucleotide chips. To generate lists of significantly differently expressed genes, the resulting data set was subjected to the significance analysis of microarray (SAM) procedure. We generated gene lists based on both P values from two-sided t-tests and intended false discovery rates (FDRs). The FDR calculation followed the method described by Storey and Tibshirani. Prediction analysis for microarrays (PAM) (25) was used to classify tissues into intended categories, e.g., tumor or non-tumor tissue. In this analysis, the threshold delta was chosen based on the best compensation for both training error rates and coefficient of variation (CV) error rates. Cross validation was performed by leaving out 10% of samples to determine the appropriate threshold parameter in PAM.

### MicroRNA target prediction

We used TargetScanS (http://genes.mit.edu/tscan/taraetscanS.html) for microRNA target prediction. Only those predicted binding sites for microRNA, which are located within the 3' UTR and are conserved across species, were considered in our analysis. For analysis and data output, the data were formatted into the WholePathwayScope database. To identify the microRNAs that regulate transcript abundance of their target mRNA in human prostate tissue, a correlation analysis was performed. For that, the Pearson correlation coefficient was computed. The statistical significance of the Pearson correlation coefficient was determined by a two-sided t-test.

### Quantitative Real-time PCR

Abundance of mature microRNAs was measured using the stem-loop TaqMan® MicroRNA Assays kit (Applied Biosystems, Foster City, CA) according to a published protocol. Briefly, cDNA was reversed transcribed from 10 ng of total RNA with specific microRNA primers from the TaqMan® MicroRNA Assays kit and reagents from TaqMan® MicroRNA Reverse Transcription kit (Applied Biosystems) following the manufacturer's directions. Real-time PCR was performed on the cDNA with Applied Biosystems Tagman 2X Universal PCR Master Mix and the appropriate 5X Taqman® MicroRNA Assay Mix for each microRNA of interest. Triplicate reactions were incubated in an Applied Biosystems 7500 Real-Time PCR system in a 96 well plate for 10 min at 95°C, followed by 40 cycles for 15 s at 95°C and 1 min at 60°C. For each sample, the threshold cycle (CO was calculated by the ABI 7500 Sequence Detection System software. Standard curves were used to determine microRNA concentrations in the samples, which were then normalized to U6 RNA.

### Regulation of protein expression by microRNAs

LNCaP and PC3 human prostate cancer cells (ATCC, Manassas, VA) were grown to 50% confluency and transfected with either microRNA precursor or antisense microRNA inhibitor (both Ambion, Austin, TX) at 100 nM final concentration using Lipofectamine 2000 reagent (Invitrogen). After 48 hours, cells were harvested by scraping and protein was extracted with RIPA buffer (Pierce Biotechnology, Rockford, IL). The Bradford assay (BioRad Laboratories, Hercules, CA: #500-0006) was performed to determine the protein concentration, and 50 ug of protein was loaded on the gel for Western blot analysis. The following microRNA precursors were used: pre-microRNA negative control (AM17110); hsa-miR-1 (cat# AM17100 Product ID: PM10617); hsa-miR-32 (cat#AM17100 product ID: PM12584); and hsa-miR-106b (cat#AM17100 product ID: PM10067). The following microRNA inhibitors (antisense) were used: anti-microRNA negative control (AM17010); hsa-miR-1 (cat#AM17000 Product ID: AM10617); hsa-miR-32 (cat#AMI7000 product ID: AM12584); and hsa-miR-106b (cat#AM17000 product ID: AM10067). The following primary antibodies were used to visualize protein expression by Western blot analysis: polyclonal rabbit anti-exportin-6 antibody, 1:200 (ProteinTech Group, Chicago, IL: 11408-1-AP); monoclonal mouse anti-PTK9 antibody, 1:500 (Abnova Corp., Taipei, Taiwan: clone 1E2); monoclonal mouse antiE2F1 antibody, 1:200 (Santa Cruz Biotechnology, Santa Cruz, CA: sc-251); monoclonal mouse anti-p21/WAF 1 antibody, 1:200 (Santa Cruz Biotechnology: sc-6246); and polyclonal rabbit anti-BIM antibody, 1:1000 (Cell Signaling/Santa Cruz Biotechnology: #2819). A quantification of protein expression was obtained with the AIDA Biopackage, 2D-Densitometry (raytest Isotopenmessgeraete GmbH, Straubenhardt, Germany).

### Luciferase assays of a reporter construct containing the 3'UTR of E2FI and BCL2L11

The E2FI and BCL2L11 (encodes Bim) 3'UTRs containing the predicted miR-106b and miR-32 target sequence, respectively, were amplified from genomic DNA (293T cells) and cloned into the pGL3 firefly luciferase control vector (Promega, Madison, WI) at the Xbal restriction site immediately downstream of the Luciferase reporter gene. To generate 3 'UTRs with a mutant target sequence, a deletion of the first 3 nucleotides was inserted into the miR-106b and miR-32 seed region complementary sites using the QuikChange-site-directed mutagenesis kit (Stratagene, La Jolla, CA). Translational inhibition of the luciferase reporter gene by either miR-106b or miR-32 was assayed in LNCaP cells. Briefly, 1.2x105 LNCaP cells per well were seeded in 24 well plates. The next day, cells were transfected with 500 ng of reporter plasmid, 2 ng Renilla reporter, and either microRNA negative control or precursor microRNA at a 100 nM final concentration using the lipofectamine 2000 reagent according the manufacturer's instructions (Invitrogen). Transfections were performed in triplicates. Cells were transfected with either the pre-microRNA negative control (AM17110), hsa-miR-106b (cat#AM17100 product ID: PM10067), or the hsa-miR-32 precursor (cat#AM17100 product ID: PM12584). After 24 hours, cells were lysed according to a Promega standard protocol, and the relative luciferase activity was determined using a DYNEX Technologies MLX luminometer. Reporter activity was normalized to the protein concentration in the cell extracts.

### Treatment of prostate cancer cells with an androgen receptor agonist

DU-145 (1x10⁶) and LNCaP (2x10⁶) human prostate cancer cells (ATCC) were plated in 75 cm2 flasks and cultured with RPMI 1640 supplemented with 10% PBS, 100 µg/ml streptomycin, 100 units/ml penicillin, and 0.25 µg/ml amphotericin B for 24 hours. Subsequently, cells were placed into phenol red-free RPMI 1640 with 5% Dextran coated charcoal-treated PBS (Invitrogen) for 48 hours for hormone depletion. Then, cells were treated with either 10 nM R1881 (methyltrienolone, PerkinElmer Life Sciences, Waltham, MA) or solvent (ethanol). After 24 hours, cells were harvested and total RNA was isolated using the mirVana PARIS Kit (Ambion,Inc.). This experiment was repeated five times. MicroRNA labeling and hybridization were performed as described previously (21), and the global expression of microRNAs was determined on the Ohio State University Comprehensive Cancer Center microarray (Version 4.0).

### RESULTS for EXAMPLE I

### Up-regulation of Dicer in prostate tumors

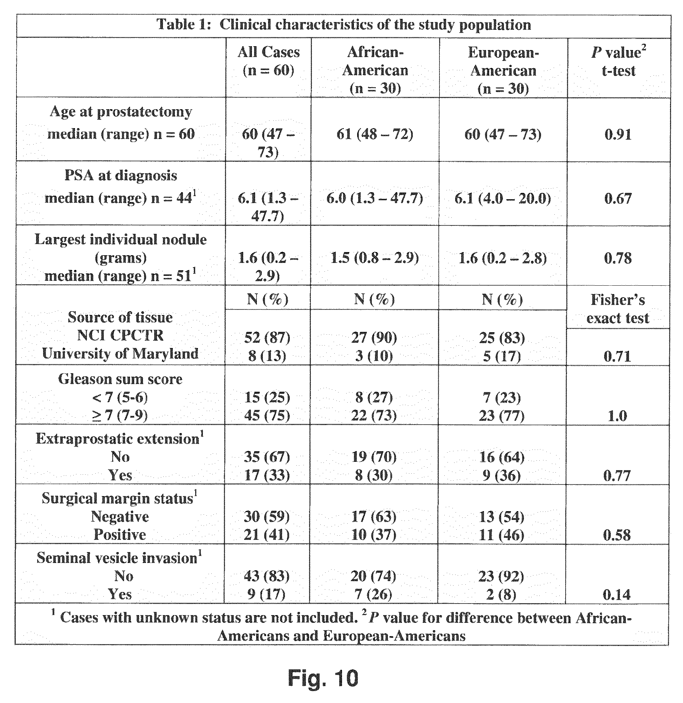

Prostate tumors were collected from African-American and European-American patients with localized disease **(****Figure 10** **- Table 1).**

After isolation of total RNA from these tumors and from 16 non-tumor tissues, the expression of about 13,000 protein-coding genes and 329 unique human microRNAs was determined with microarrays.

Initially, the gene expression profiles of these samples were searched for cancer-related alterations in the expression of those mRNAs that have been shown to regulate the processing of microRNAs, e.g. mRNAs that encode Drosha or Dicer, among others. Our analysis revealed that Dicer is significantly higher expressed in prostate tumors (1.6-fold; FDR < 1%) when compared with non-tumor tissue. DGCR8, which encodes an essential cofactor for Drosha, was also up-regulated in tumors but to a lesser extent (1.2-fold; FDR < 1%) than Dicer when compared with nontumor tissue. DGCR8, which encoded an essential cofactor for Drosha, was also up-regulated in tumors (1.2 fold, FDR< 1%). The increased expression of *Dicer* and *DGCR8* in tumors was confirmed by qRT-PCR, which revealed a larger fold difference than indicated by the microarray **(****Figures 4A-4B****).**

Further analysis showed that Dicer and EIF2C2, both components of the RISC complex, were more highly expressed in tumors with a high Gleason sum score (score 7-9) than in tumors with a low Gleason sum score (score 5-6). However, these expression differences were rather modest (Dicer: 1.2-fold; EIF2C2: 1.3-fold). Because a frequent co-expression of host genes and intronic microRNAs has been found in human cells, we also investigated the expression of microRNA host genes in prostate tumors.

Among the host genes for microRNAs, the expression of five was found to be altered in prostate cancer (all FDR < 1%). Of those, C9orf5 (2.1-fold up-regulation), which is the host for miR-32, and MCM7 (1.7-fold up-regulation), which is the host for the miR-25 cluster (miR-25/miR-93/miR-106b), were most highly over-expressed in tumors. NFYC (host of miR-30c-1), SMC4L1 (host of miR-15b and miR-16-2), and PTPRN2 (host of miR-153-2) showed a more moderate 30% to 40% increased expression in tumors when compared to non-tumor tissue.

### MicroRNA gene signature of prostate cancer

We first searched for those microRNAs that showed differential expression between tumor and non-tumor tissue. As shown in **Figure 11** **- Table 2,** the expression of multiple microRNAs was altered in prostate tumors.

Among the microRNAs with lower transcript levels in tumors than non-tumor tissues, miR-520h, miR-494, and miR-490 were most highly decreased. Two other notable microRNAs in this list were miR-1(-2) and miR-133a(-1). These two microRNAs are encoded by the same pri-mRNA. miR-32 was the most significantly up-regulated tumor microRNA, followed by miR-182, miR-31, and miR-26a. The list of more highly expressed tumor microRNAs also contained all members of the miR-106b-25 cluster (miR-106b/miR-93/miR-25) and two members of the miR-99b cluster, miR-99b and miR-125a. The up-regulation of both miR-32 and the miR-106b-25 cluster is consistent with the increased expression of their respective host genes, C9orf5 and MCM7, in prostate tumors.

A statistical analysis of the microarray data confirmed that tissue transcript levels of C9orf5 and miR-32 are statistically significantly correlated (P = 0.0003). The Pearson coefficient indicated that this correlation was moderately strong across all samples (0.39; 95% confidence interval: 0.18 to 0.57; n = 76). Similar data were obtained for the correlation between *MCM7* and *miR-106b-25* cluster transcript levels (*miR-106b*: 0.37 (Pearson coefficient), P = 0.001; *miR-93*: *0.35, P* = 0.002; *miR-25*: 0.23, *P* = 0.04).

We corroborated the microarray data by qRT-PCR analysis of selected microRNAs in a random subset of the tumor and nontumor tissues. Consistent with the microarrays, we found that mature miR-32 (3.2-fold) and miR-106b (3.0-fold) are higher expressed in tumors than nontumor tissues **(****Figures 5A-5D****).** We also found that mature *miR-1* was down-regulated (average: 0.44-fold) and *miR-106a,* a *miR-106b* homologue, was over-expressed (average: 3.7-fold) in the tumors when compared with nontumor tissues.

We also performed a paired analysis of the microarray data for those 10 tumors in our study whose surrounding nontumor tissue was available. The paired analysis corroborated our previous findings. At a FDR <10%, miR-26a, miR-30c-1, miR-32, miR-146b, miR-181a, miR-182, miR-196a, miR-200c, miR-375, and all microRNAs of the miR-106b-25 cluster ere found to be up-regulated in tumors (1.5-fold to 2.5-fold). The most significantly down-regulated tumor microRNA was miR-494 (0.4-fold) and miR-126 (0.6 fold). However, the miR-133a cluster was not found to be significantly differently expressed in this tumor subset.

### Association of microRNAs with extraprostatic extension and Gleason score

We next analyzed our dataset for differences in microRNA expression associated with extraprostatic extension of the tumors. Extraprostatic extension is an unfavorable prognostic factor in patients with prostate carcinoma. At a FDR < 20%, we found 15 microRNAs with a difference in expression between tumors that showed an extraprostatic extension of the disease (n = 17) and those that did not (n = 35) **(****Figure 12** **- Table 3).**

miR-101 was the most consistently over-expressed microRNA in localized prostate tumors that spread out of the prostate gland (FDR < 1%). Extraprostatic extension shared a portion of its microRNA signature with the tumor signature **(****Figure 11** **- Table 2).**

Two microRNAs, miR-99b and miR-196a, are common to both signatures. Two other microRNAs of the extraprostatic extension signature, miR-200a and miR-200b, have an extensive homology with miR-200c in the tumor signature. We also studied the association of microRNAs with seminal vesicle invasion although few tumors in the study were diagnosed with this characteristic. Only one microRNA was differently expressed between tumors with seminal vesicle invasion (n = 9) and those without invasion (n = 43) at a FDR < 20%. This microRNA was miR-199a-1, and it was 2.3-fold increased in tumors with seminal vesicle invasion when compared to the other tumors.

MicroRNA expression profiling did not reveal a robust signature for Gleason score. Only very few microRNAs were found to be differently expressed at a FDR <20%. Significantly up-regulated microRNAs (P < 0.01) in tumors with a high Gleason sum score (score 7-9, n = 45) were miR-92-2 (1.3-fold), a miR-25 and miR-32 homologue, and miR-335 (1.2-fold), and significantly down-regulated microRNAs (P < 0.01) were the miR-1-133a cluster (0.7-fold) and miR-130 (0.8-fold), when compared with tumors that have a low Gleason sum score (score 5-6, n = 15).

Because the tumors in our study were collected from African-American and European-American patients that were well matched on clinicopathological parameters **(****Figure 10** **- Table 1),** we compared the tumor microRNA signatures between African-Americans (n = 30) and European-Americans (n = 30). Few microRNAs were differently expressed (P < 0.01). At a FDR < 20%, miR-129, miR-196b, and miR-342 were found to be less abundant (20% to 30% lower) in tumors of African-Americans than in tumors of European-Americans. From this analysis, it does not appear that tumor microRNAs are very differently expressed by race/ethnicity.

### Classification of tumor and nontumor tissue with microRNAs.

Gene-based classifiers are powerful diagnostic and prognostic tools in improving disease diagnosis and for the prediction of clinical behavior. We used the PAM application to identify microRNA signatures that discriminate between tumor and non-tumor tissue, between organ-confined tumors and tumors with extraprostatic extension, and between tumors with high and low Gleason sum score. PAM identified two microRNA signatures consisting of 7-probesets and 37-probests that best distinguished between tumor and non-tumor tissue. The 7-probeset signature achieved a correct classification of 14 (88%) out of 16 non-tumor tissues and 49 (82%) out of 60 tumors. This signature was based on the expression pattern of only four microRNAs, miR-32, miR-218-2, miR-490, and miR-520h **(****Figure 13B** **- Table 4B),** all of which were among the most significantly up- or down-regulated tumor microRNAs **(****Figure 11** **- Table 2).** Further improvement of the overall prediction accuracy was obtained with a 37-probeset signature that represented 23 microRNAs **(****Figure 14** **- Table 5)).**

This signature completely overlapped with the 7-probeset signature. With the 37-probeset signature, PAM achieved a correct classification of 16 (100%) out of 16 non-tumor tissues and 48 (80%) out of 60 tumors. PAM could not identify good classifiers for extraprostatic extension and Gleason score. A weak to modest classifier for Gleason score required 149probesets (data not shown).

### Relationship between transcript Abundance of microRNAs and their target mRNAs in prostate tissue.

MicroRNAs regulate the expression of protein-coding genes by target-specific translational inhibition. However, it has recently been shown that some microRNAs, e.g., miR-1, can down-regulate the transcript levels of a large number of target genes in mammalian cells. Because miR-1 was among the most significantly down-regulated microRNAs in prostate tumors, we performed a correlation analysis between miR-1 expression levels and the expression levels of predicted miR-1 target genes in these tumors. This test was performed to identify candidate miR-1 target genes that become over-expressed in prostate tumors because of diminished miR-1 expression. The analysis yielded putative target mRNAs that were found to be up-regulated in prostate tumors (FDR < 1%) and inversely correlated with miR-1 expression **(****Figure 13** **- Table 4)).**

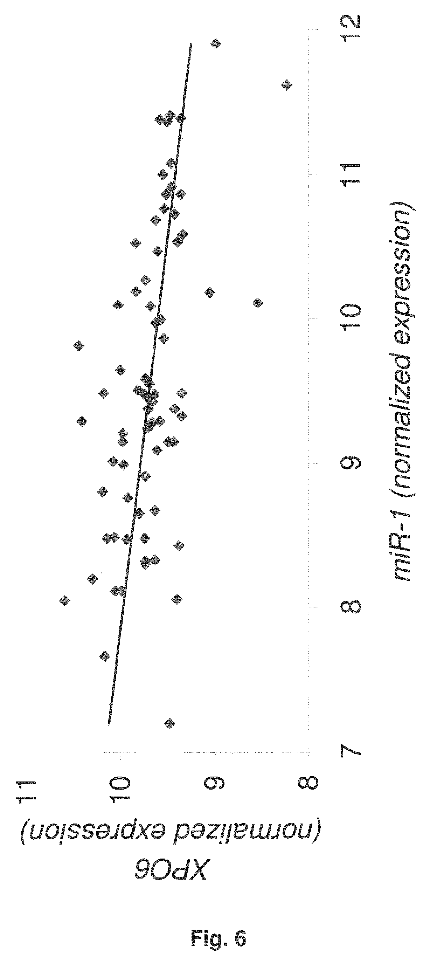

Among those, transcripts for WDR6, XP06, and SMARCA4 showed the most significant inverse correlation with tumor miR-1 expression (each P < 1x 10⁻¹⁰). The relationship between XPO6 and miR-1 transcript levels in prostate tumors is pictured in **Figure 6****.**

We also found that XPO6 protein levels in the tumors are inversely correlated with miR-1 (-0.29, Spearman correlation coefficient; n = 8). However, not all predicted targets of miR-1 showed an inverse relationship with miR-1 transcript levels in the tumors. For example, TWF1 (also termed PTK9) was positively correlated with miR-1, suggesting that binding of microRNAs to its target sequence may sometimes lead to mRNA sequestration and cellular accumulation of the inhibited mRNA (30).

Our analyses were extended to other microRNAs that were either significantly up-or down-regulated in prostate tumors. Here, we initially determined the global distribution of the Pearson correlation coefficients between the microRNA of interest and either all mRNAs that are probed by the HG-U133A 2.0 array or only those mRNAs that are predicted targets of the microRNA. For two microRNAs, miR-106b and miR-181a, the distribution of the correlation coefficients was notably different between all mRNA and those mRNA that are the predicted targets of miR-106b and miR-181a **(****Figures 1A-1B****).**

The distribution curves for predicted target mRNAs of miR-106b and miR-181a showed a distinct shoulder that extended toward negative Pearson correlation coefficients. This pattern is a departure from a normal distribution and indicates that tissue transcript levels of a subset of mRNAs, which have a predicted microRNA target sequence in the 3'UTR, are reduced by miR-106b and miR-181a. A list of target genes that were significantly down-regulated in tumors (FDR < 1%) and whose transcript level inversely correlated with miR-181a expression is shown in **Figure 14****- Table 5.**

A comparison of these target genes with a list of genes that correlated with miR-181a transcript levels in leukemia samples (31) showed that several, e.g., SLC9A6, RIN2, KLHL2, and GHITM, were negatively correlated with miR-181a in both lists.

### Inhibition of protein expression by candidate oncomiRs in prostate cancer cells

Our results from the tumor studies suggest that miR-1, miR-32, and the miR-106b-25 cluster are oncomiRs in prostate cancer, miR-32 and miR-25 share a high degree of homology, and their predicted target genes are the same (targetscan.org). Moreover, the miR-106b-25 cluster is highly homologous to a known oncomiR, the miR-17-92 cluster, and the predicted targets of miR-17-5p and miR-106b are identical. A target of the miR-17-92 cluster is E2F1.

We transfected two human prostate cancer cell lines with precursor and antisense microRNAs to examine whether miR-1, miR-32, and miR-106b regulate the protein expression of cancer-related genes in these cells.

The endogenous expression of these microRNAs in the cell lines was miR-106 > miR-32 > miR-1 by qRT-PCR. miR-1 was at the detection limit. For miR-1, we tested whether the relationship between transcript abundance of microRNAs and their target mRNAs in tumor tissue is useful to identify microRNA targets and examined whether the protein expression of exportin-6 (XPO6) and protein tyrosine kinase 9 (TWF1) is regulated by miR-1. Transfection of the prostate cancer cells with miR-1 confirmed that it represses both exportin-6 and PTK9 on the protein level in both prostate cancer cell lines **(****Figure 2A****).** Neither miR-32 nor miR-106b altered the expression of these proteins (data not shown).

We next investigated the regulation of E2F1 and p21/WAF1 protein levels by miR-106b. Both proteins are encoded by mRNAs that have a predicted target sequence of miR-106b in their 3 'UTRs. Whereas E2F1 did not correlate with miR-106b on the transcript level in prostate tumors, a significant inverse correlation exists between the expression of CDKN1A (encodes p21/WAF1) and miR-106b in these tumors (-0.34; 95% CI;; -0.9 to -0.55; P = 0.003).

As shown in **Figure 2B****,** transfected precursor miR-106b decreased p21/WAF1 protein levels and antisense miR-106b increased p21/WAF1 protein levels in the two cell lines. We obtained the same results for E2F1 after transfection of the prostate cancer cells with precursor and antisense miR-106b **(****Figure 3A****).**

We also studied the effect of miR-32 on Bim protein expression. Bim is encoded by BCL2L11 and is a predicted target of miR-32. Transfection of prostate cancer cells with precursor miR-32 decreased Bim protein levels while antisense miR-32 increased Bim protein levels **(****Figure 3C****).** Bim is encoded by BCL2L11 and is a predicted target of miR-32. BCL2L11, and miR-32 transcript levels did not correlate in the tissue samples suggesting that miR-32 may regulate this target mostly by translation inhibition.

The protein expression of E2F1 and p21/WAF1 was not influenced by miR-32, nor was the protein expression of Bim influenced by miR-106b in these cell lines (data not shown).

### E2FI and Bim are direct targets of miR-106b and miR-32

To further corroborate our findings and to provide evidence that these proteins are direct targets of miR-106b and miR-32, LNCaP cells were co-transfected with precursor microRNA and pGL3 luciferase reporter constructs containing either wild-type or mutant 3'UTR of two genes, E2F1 and BCL2L11 (encodes Bim), respectively. Mutant 3'UTRs contained a deletion of the first 3 nucleotides in the miR-106b and miR-32 seed region complementary sites. The 3'UTRs were placed at a position that would lead to a translational inhibition of the luciferase reporter when the microRNA binds to the target sequence.

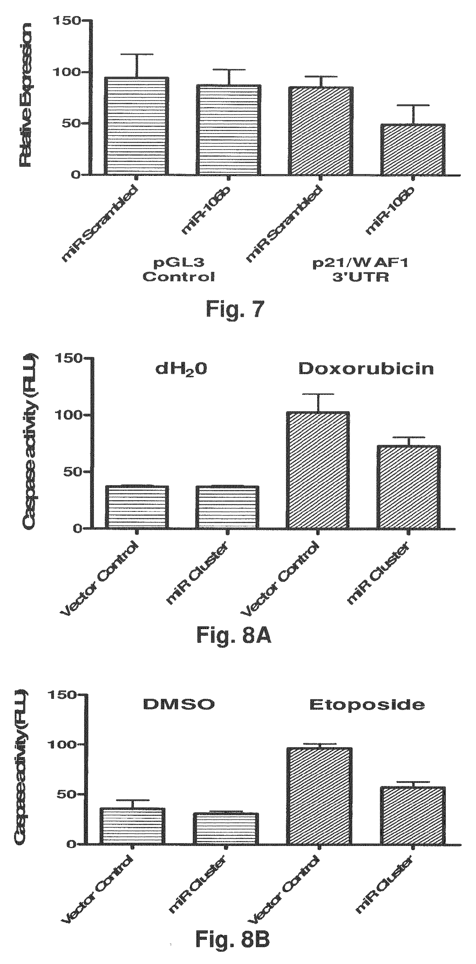

As shown in **Figure 3B** and **Figure 3D****,** co-transfection of either miR-106b with the reporter construct containing the wild-type 3'UTRs of E2F1 or miR-32 with the reporter construct containing the wild-type 3'UTRs of BCL2L11 resulted in a significant inhibition of the luciferase reporters when compared with the precursor microRNA negative control. There was no inhibition of the reporter by the microRNAs in the absence of the 3'UTR. The presence of a mutant 3'UTR either abolished or attenuated the effect of the microRNAs. The results are consistent with a direct effect of the microRNAs on protein translation by binding to their 3'UTR target sequence. Such a mechanism has also been established for the regulation of p21/WAF1 by miR-106b in human colon and gastric cancer cells. Accordingly, we observed that miR-106b inhibits a luciferase reporter by a CDKN1A 3S UTR-mediated mechanism in LNCaP cells **(****Figure 7****).**

### Inhibition of caspase activation by the miR-106b-25 cluster in 22Rv1human prostate cancer cells.

Our previous data indicated that miR-32, miR-106b, and their homologues (e.g., miR-25) may act as oncogenes, because they target the proapoptotic function of Bim and E2F1. To evaluate the effect of the miR-106b-25 cluster on apoptosis induced by doxorubicin and etoposide, we infected 22Rv1 cells, a nonmetastatic human prostate cancer cell line, with a lentiviral expression construct encoding the miR-106b-25 cluster. Using the Caspase-Glo apoptosis assay, we observed a significant inhibition of caspase-3/caspase-7 activation by this cluster in anticancer drug-treated cells **(****Figures 8A-8B****).** The data are consistent with an antiapoptotic function of the miR-106b-25 cluster in prostate cancer cells.

### Identification of androgen-regulated microRNAs

Androgens play a key role in physiology and tumor biology of the prostate. We examined the regulation of microRNAs by androgens in DU145 and LNCaP cells. Treatment of the DU145 cells with R1881 did not yield any significant changes in microRNA expression. In contrast, expression of numerous microRNAs was significantly changed (FDR < 5%) in LNCaP cells following the R1881 Treatment **(****Figure 15** **- Table 6).**

One microRNA, miR-338, was significantly up-regulated. The other microRNAs were down-regulated, including miR-126-5, miR-146b, miR-219-5p and all members of the miR181b-1, miR-181c, and miR-221 clusters. An analysis of the baseline microRNA expression in cultured DU145 and LNCaP cells revealed that all members of those three microRNA clusters had a significantly higher expression in the androgen-insensitive DU145 cells than in the androgen-responsive LNCaP cells (FDR < 5%).

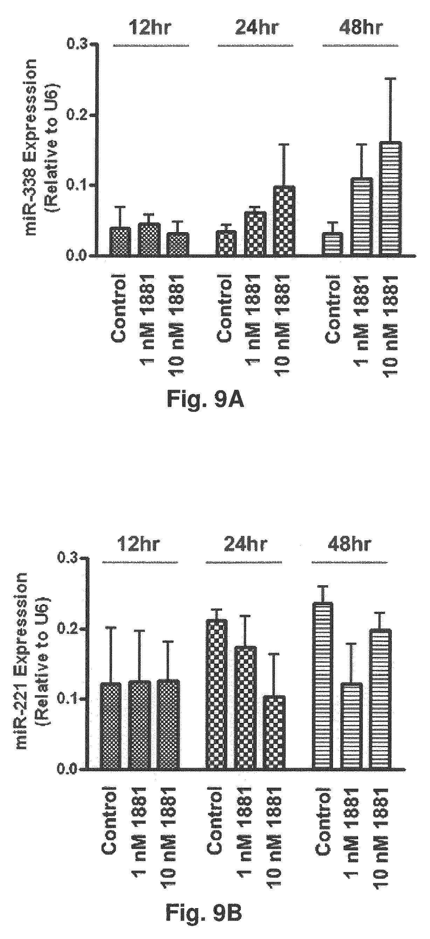

Using a motif search in the Genomatix transcription factor binding site database, we found that the aforementioned microRNAs have putative androgen receptor binding sites in their flanking regions **(****Figure 16** **- Table 7).** We further corroborated the microarray results in experiments with LNCaP cells that were treated with either 1 or 10 nmol/L R1881 for 12, 24, and 48 hours. qRT-PCR analysis of mature miR-338 and miR-221 showed that their expression level is androgen-regulated **(****Figures 9A-9B****).**

### DISCUSSION of EXAMPLE I

We have now discovered a distinct microRNA expression signature in prostate tumors and alterations in the expression of genes that regulate tumor microRNA processing. Furthermore, we found evidence that the deregulation of microRNAs influences transcript abundance and protein expression of target mRNAs in the prostate. In cell culture, we showed that candidate oncomiRs in prostate cancer, e.g., miR-32 and miR-106b, inhibit the protein expression of cancer-related genes. The results are consistent with a pathogenic role of altered microRNA expression in human prostate carcinogenesis.

This is the first study to use large-scale gene expression profiling of both microRNAs and protein-encoding RNAs to identify alterations in microRNA function that occur in human prostate tumors. Other strengths of the study are its large sample size and the inclusion of both African-American and European-American patients. Thus, the findings are representative for the two race/ethnic groups that have the highest prostate cancer burden in the United States.

We found an increased expression of Dicer and DGCR8 in prostate tumors, and of Dicer and EIF2C2, which encodes argonuate-2, in tumors with a high Gleason score. The observation that Dicer is up-regulated in prostate cancer is consistent with a recent report and indicates that prostate tumors could be more efficient than normal prostate tissue in processing microRNA precursors into mature microRNA. Impaired microRNA processing has recently been shown to enhance tumorigenesis in mice, and others have hypothesized that microRNA processing is generally down-regulated in cancer.

In prostate tumors, however, the opposite effect of enhanced microRNA processing may take place, as portrayed by our data and the aforementioned study that showed increased protein expression of Dicer in prostate cancer cells and over-expression of Dicer and EIF2C2 in the metastatic disease.

MicroRNA expression profiles classify human cancers. Distinct signatures for several epithelial cancers, including breast, colon, lung and pancreatic cancer, have been reported. Two other studies explored microRNA expression in prostate cancer. Consistent with the Baylor prostate data, we also observed that miR-145 is significantly down-regulated in prostate tumors. However, these two published studies were rather small and examined only few tumors when compared to our study.

We identified a tumor gene signature that contained up- and down-regulated microRNAs.

The most highly up-regulated microRNA was miR-32, followed by miR-182, miR-31, miR-26a, miR-200c, and miR-196a. The list of over-expressed tumor microRNAs also contained the miR-106b-25 cluster, which is consistent with the observed gain in copy number for miR-25, miR-93, and miR-106b in several human malignancies.

The most significantly down-regulated microRNAs included miR-520h, miR-494, miR-490, and the miR-1-133a cluster.

Altered expression of microRNAs in human cancer has been observed in numerous studies. Up-regulation of microRNAs in tumors is common, and it is consistent with the known oncogenic activity of many microRNAs. Mechanisms of up-regulation include transcriptional activation and the increase in gene copy numbers. A decrease in the abundance of mature microRNA may result from altered processing, as shown recently), which would lead to an indiscriminate lower expression of mature microRNAs. We did not observe that in the present study. Alternatively, microRNA expression could be lost because of mutations or genomic alterations (21) or epigenetic silencing of microRNA loci. Epigenetic silencing is an important mechanism in prostate cancer, and future studies will have to address whether this mechanism impedes microRNA expression in prostate tumors.

Little is known about the function of most of these microRNAs. miR-32 is a homologue of miR-25, miR-92, miR-363, and miR-367. Several of these microRNAs were also up-regulated in the prostate tumors. miR-32 is increased in colon and pancreatic cancer, and is a mediator of the antiviral defense of human cells. This function of miR-32 could be a link between its altered expression and prostate cancer development because several of the known prostate cancer susceptibility genes are also involved in host defense. As shown for other microRNAs, miR-32 should regulate protein expression of target genes.

We made the novel observation that miR-32 inhibits the expression of Bim, a pro-apoptotic member of the BCL-2 family. Bim is haploinsufficient and inactivation of one allele is sufficient to accelerate Myc-induced tumorigenesis. Bim has key roles in the apoptosis of epithelial tumors and mediates antitumor effects of chemotherapy. Thus, down-regulation of Bim by miR-32 may contribute to the resistance of tumor cells to apoptotic stimuli in the tumor environment.

Other notable microRNAs with a known function include miR-1, miR-133a, and miR-196a. The miR-1-133a cluster is preferentially expressed in muscle cells and has been shown to regulate cell differentiation. miR-1 is a homologue of miR-206, which is a suppressor of metastasis in breast cancer. The inventors' discovery that miR-1 is down-regulated in prostate tumors is consistent with the tumor suppressor function of its homologue.

We examined miR-1 and observed that expression of this microRNA is inversely correlated with the expression of exportin-6 and protein tyrosine linase 9 (also termed A6/twinfilin) in prostate tumors and cultured prostate cancer cells. Not much is known about the function of these two genes, but recent data suggest that both regulate cellular actin dynamics. MiR-196a was identified as a repressor of HOXB8, and elevated expression of miR-196a predicts poor survival in pancreatic cancer. This microRNA was common to both the tumor signature and the extraprostatic extension signature in our study, indicating that up-regulation of miR-196a in prostate cancer could be a factor in disease progression.

MicroRNA signatures have been shown to have both diagnostic and prognostic value in human cancer.

We determined the diagnostic and prognostic significance of microRNA expression in prostate cancer by investigating the association of microRNAs with tumor diagnosis, extraprostatic extension, Gleason score, and race/ethnicity of the patients. The tumor microRNA signature of African-American patients and European-American patients was compared because recent evidence emerged that differences in tumor biology may exist between these two patient groups. Although great differences in microRNA expression were found between tumor and non-tumor tissue in the prostate, relatively few microRNAs were differently expressed between tumors that spread out of the prostate gland and those that did not, between tumors that have a Gleason score > 7 and those that have a score < 7, and between tumors from African-American patients and European-American patients.

Moreover, a seven (7) probeset signature consisting of four (4) microRNAs was identified by PAM that could distinguish between tumor and non-tumor prostate tissue. However, PAM analysis could not generate a good classifier for extraprostatic extension, Gleason score, or race/ethnicity.

We identified one microRNA, miR-101, that was associated with extraprostatic disease extension at a low margin of error. The function of this microRNA is unknown. We believe that the intrinsic heterogeneity of the prostate tumors precluded us from finding more microRNAs that are associated with unfavorable prognostic factors in prostate cancer.

The analysis of the genomic location of microRNAs can provide clues about their putative function and the mechanisms that cause altered microRNA expression in tumors. Recent studies have shown that microRNAs are frequently located within introns of protein-coding genes and are co-expressed with these host genes . We investigated host gene expression in prostate tumors and found that several of them were increased in prostate tumors. C9orf5 and MCM7 were the two most highly up-regulated host genes, and their expression correlated with the expression of the intronic microRNAs, miR-32 and the miR-1 06b-25 cluster, respectively. The data suggest a common mechanism that leads to the up-regulation of host gene and co-transcripted microRNA in prostate tumors.

While the role of C9orf5 in cancer is unknown, MCM7 amplifications have previously been associated with prostate cancer. The MCM7 locus was found to be amplified in 88% of cases with cancer relapse. MCM7 over-expression is not restricted to prostate cancer and has been observed in other malignancies, including cervical cancer.

We examined whether miR-106b targets E2F1 and CDKN1A (encodes p21/WAF1) in prostate cancer cells and found that protein expression of these genes is inhibited by miR-106b. The miR-106b-25 cluster has extensive homologue with two other microRNA clusters that are candidate human oncogenes, the miR-17-92 cluster and the miR-106a-363 cluster. E2F1 is also a target of miR-17-5p and miR-20a in the miR-17-92 cluster, and it has both oncogene and tumor suppressor functions. Like Bim, translated E2F1 is pro-apoptotic and cooperates with the tumor suppressor p53 to mediate apoptosis. Its overexpression induces apoptosis in LNCaP cells, which indicates that inhibition of E2F1 translation by miR-106b can protect prostate cancer cells from apoptosis in the tumor environment.

p21/WAF1 is a mediator of p53 tumor suppression. The growth inhibitory effect of p21/WAF1 in prostate cancer has been shown, and it mediates cell cycle arrest in prostate carcinoma cells in response to anticancer agents. We tested whether the miR-106b-25 cluster has antiapoptotic activity and found that it inhibits caspase activation by doxorubicin and etoposide in 22Rv1 cells.

These data are consistent with an oncogenic function of miR-106b in prostate cancer, in part, because of its ability to suppress E2F1 and p21/WAF1 protein expression.

Neither miR-1, miR-32, nor the miR-106b-25 cluster was regulated by androgen stimulation of LNCaP cells. However, we identified several other microRNAs that were up- or down-regulated by androgen treatment. Those included miR-338 and miR-126, and the miR-181b-1, miR-181c, miR-221 clusters, among others. A motif search showed that these microRNAs have putative androgen receptor binding sites in their flanking regions.

MiR-338 was the only significantly up-regulated microRNA. There are no reports on the function of this microRNA, but it is located in a region with frequent copy number gains in three epithelial cancers.

MiR-181 family members influence hematopoietic lineage differentiation, and their expression is altered in leukemia and several solid tumors. The miR-221 clusters has been found to regulate the p27^{Kip1} tumor suppressor and may have oncogenic properties in prostate cancer. However, this cluster also inhibits the oncogene c-Kit and angiogenesis.

The identification of protein-coding genes that are regulated by a specific microRNA has been proven difficult despite the development of computational approaches to predict microRNA targets. The ability to find target mRNAs is further complicated by the fact that target selectivity of microRNAs may depend on the cellular microenvironment.

We used an exploratory approach and conducted a correlation analysis between microRNA expression and mRNA expression in prostate tissue. While not wishing to be bound by theory, the inventors herein now believe that this approach will be successful if the microRNA of interest affects transcript abundance of target mRNAs, but it will fail if the target genes are regulated only by translational inhibition.

We found that the expression of miR-1 is inversely correlated with a number of computationally predicted target genes in prostate tumors, e.g., XPO6. However, we also found that tumor miR-1 expression correlated positively with the transcript level of predicted targets, e.g., PTK9. Subsequent validation of these observations in cell culture confirmed that XP06 and PTK9 are both regulated by miR-1 in prostate cancer cells.

The data provide new evidence that binding of microRNAs to 3'UTR sequences can lead to both degradation and accumulation of the targeted mRNA in mammalian cells, and that both an inverse and a positive correlation between a microRNA and a mRNA in a human tissue can be predictive of a microRNA target gene. Thus, correlation analysis of microRNA and mRNA expression in human tissue may prove useful in identifying mRNAs that are regulated by microRNAs.

We have now identified alterations in microRNA expression that occur in human prostate tumors and correlate with expression variations of protein-coding genes in these tissues. Experiments in cell culture showed that tumor microRNAs can regulate the expression of cancer-related genes in human prostate cancer cells. These results show a pathogenic role of microRNAs in prostate cancer biology.

### EXAMPLE II

### Introduction

Prostate cancer is the most frequently diagnosed malignancy and the second most common cause of cancer mortality in American men. The mortality can be attributed to the spread of cancer cells beyond the prostate. Perineural invasion (PNI) is the dominant pathway for local invasion in prostate cancer and is also a mechanism for extraprostatic spread of the disease. Yet, the prognostic significance of PNI remains controversial. Several studies have observed an association of PNI with markers of poor outcome, but others did not find it to be a prognostic factor in prostate cancer.

The occurrence of PNI is a relatively early event in the development of the clinical disease, and most tumor specimens from radical prostatectomy are PNI-positive. It is this high occurrence rate of PNI in clinical samples (85% to 100%) and the inadequate knowledge of its biology that limit our understanding of PNI's role in prostate cancer progression and disease outcome.

PNI is the process where cancer cells adhere to and wrap around nerves. It occurs in many other types of cancer, including pancreatic and head and neck cancers. Prostate cancer cells that have a perineural location acquire a survival and growth advantage and exhibit reduced apoptosis and increased proliferation when compared with cells located away from nerves. Altered expression of adhesion molecules in both prostate cancer cells and the adjacent nerves has been observed in PNI, and it has been hypothesized that the changed expression of these molecules allows cancer cells to thrive in the vicinity of nerves.

Nevertheless, the molecular mechanisms that lead to PNI remain poorly understood.

In EXAMPLE II, the inventors applied gene expression profiling of both microRNAs and protein-coding genes to identify the gene expression changes associated with PNI in human prostate cancer. The inventors investigated whether the gene expression signature that differentiates PNI from non-PNI tumors will reveal molecular alterations that take place at the transition from a non-invasive tumor to a tumor with PNI. We assayed microRNAs because a crucial role for them in cancer has been demonstrated. Their expression profiles have been shown to classify tumors by developmental lineage and differentiation state.

### MATERIALS AND METHODS for EXAMPLE II

### Tissue samples.

Frozen tumor specimens were obtained from the NCI Cooperative Prostate Cancer Tissue Resource (CPCTR). The tumors were resected adenocarcinomas that had not received any therapy prior to prostatectomy. The macro-dissected tumor specimens were reviewed by a pathologist, who confirmed the presence of tumor in the frozen specimens. All tissues were collected between 2002 and 2004. Tissue collection was approved by the institutional review boards of the participating institutions.

### RNA extraction.

Total RNA was isolated using TRIZOL reagent according to the manufacturer's instructions (Invitrogen, Carlsbad, CA). RNA integrity for each sample was confirmed with the Agilent 2100 Bioanalyzer (Agilent Technologies, Palo Alto, CA). Each RNA sample was then split into two aliquots that were either processed for the microRNA microarray or the mRNA microarray.

### Gene microarrays.

MicroRNA labeling and hybridization were performed as described previously. The microRNA microarray (Ohio State University Comprehensive Cancer Center, Version 2.0) contains probes spotted in quadruplicate for 235 human and 222 mouse microRNAs [24]. The labeling and the hybridization of mRNAs were performed according to Affymetrix standard protocols (Santa Clara, CA). Briefly, 5 µg of total RNA was reverse transcribed with an oligo (dT) primer that has a T7 RNA polymerase promoter at the 5' end. Second-strand synthesis was followed by cRNA production with incorporation of biotinylated ribonucleotides using the BioArray High Yield RNA Transcript Labeling Kit T3 from Enzo Life Sciences (Farmingdale, NY). The labeled cRNA was fragmented and hybridized to Affymetrix GeneChip HG-U133A 2.0 arrays. This array contains 22,283 probe sets that represent approximately 13,000 human protein-coding genes. Hybridization signals were visualized with phycoerythrin-conjugated streptavidin (Invitrogen) and scanned using a GeneChip Scanner 3000 7G (Affymetrix). In accordance with Minimum Information About a Microarray Experiment (MIAME) guidelines, we deposited the CEL files for the microarray data and additional patient information into the GEO repository (.ncbi.nlm.nih.gov/geo/). The GEO submission accession number for both the microRNA and mRNA profiling data is GSE7055. Additional information about the custom microRNA microarray, Version 2.0, can be found under the ArrayExpress accession number: A-MEXP-258.

### Data normalization and statistical analysis.

Median-centric normalization was used for the custom microRNA oligonucleotide chips. Affymetrix chips were normalized using the robust multichip analysis (RMA) procedure. To generate lists of significantly differently expressed genes, the resulting data set was subjected to the significance analysis of microarray (SAM) procedure. We generated gene lists based on both P values from two-sided t-tests and intended false discovery rates (FDRs). The FDR calculation followed the method described by Storey and Tibshirani. Unsupervised hierarchical clustering was performed according to principles described by Eisen et *al..*

### Quantitative renl-time PCR analysis of microRNA and mRNA.

Abundance of mature microRNAs was measured using the stem-loop TaqMan® MicroRNA Assays kit (Applied Biosystems, Foster City, CA) according to a published protocol [29]. Using 10 ng of total RNA, mature microRNA was reverse transcribed into a 5'-extended cDNA with mature microRNA-specific looped RT primers from the TaqMan® MicroRNA Assays kit and reagents from TaqMan® MicroRNA Reverse Transcription kit (Applied Biosystems) following the manufacturer's directions. Real-time PCR was performed on the cDNA with Applied Biosystems Taqman® 2X Universal PCR Master Mix and the appropriate 5X Taqman® MicroRNA Assay Mix for each microRNA of interest. Triplicate reactions were incubated in an Applied Biosystems 7500 Real-Time PCR system in a 96 well plate for 10 min at 95°C, followed by 40 cycles for 15 s at 95°C and 1 min at 60°C. For each sample, the threshold cycle (*Ct*) was calculated by the ABI 7500 Sequence Detection System software. Standard curves were used to determine microRNA concentrations in the samples, which were then normalized to U6 RNA. Abundance of mRNA was determined according to a previously described quantitative real-time (qRT) PCR method [30]. Accordingly, 100 ng of total RNA was reverse transcribed using the High-Capacity cDNA Archive Kit (Applied Biosystems, Foster City, CA). qRT-PCR was subsequently performed in triplicate using TaqMan Gene Expression Assays (Applied Biosystems), which include pre-optimized probe and primer sets specific for the genes being validated. The assay ID numbers of the validated genes are as follows: Hs00744661_sH for metallothionein 1F and Hs00828387_g1 for metallothionein 1M. Data were collected using the ABI PRISM^{®} 7500 Sequence Detection System. The 18s RNA was used as the internal standard reference. Normalized expression was calculated using the comparative C_{T} method as described and fold changes were derived from the 2^{-ΔΔCt} values for each gene.

### Immunohistochemistry.

Protein expression in perineural and nonperineural cancer cells was assessed immunohistochemically on formalin-fixed, paraffin-embedded tumor sections. The tumors (n = 30) were from prostate patients treated by radical prostatectomy at the Baltimore VA Hospital and the University of Maryland Medical Center. Five micron sections were immunohistochemically stained for S100, a marker for nerve trunks, to visualize areas with PNI. Sections from fourteen tumors were found to contain representative areas with perineural and nonperineural cancer cells. For antigen retrieval, deparaffinized sections were microwaved in 1x Citra buffer (Biogenex, San Ramon, CA). Immunohistochemical staining was performed with the Dako Envision system (DakoCytomation, Carpinteria, CA). The following primary antibodies were used: 1:500 diluted rabbit polyclonal antibody for S100 (Ventana, Tucson, AR); 1:1000 diluted mouse monoclonal antibody for coxsackie adenovirus receptor (CXADR) (Atlas Antibodies, Stockholm, Sweden); and 1:500 diluted mouse monoclonal antibody for metallothionein (DakoCytomation). This antibody (E9) recognizes metallothionein-1 and -2 family members (# M0639). Positive controls: intestine (CXADR) and liver (metallothionein). Omission of the primary antibody was the negative control. A pathologist, who was blinded to the microarray results, evaluated the intensity of the immunostains in perineural and nonperineural cancer cells and categorized immunostaining as less intensive, same, or more intensive in the perineural cancer cells when compared with nonperineural cancer cells. Images of representative areas were taken to document the expression differences.

### In-situ Hybridization.

In-situ hybridization (ISH) was performed using the GenPoint™ Catalyzed Signal Amplification System (DakoCytomation) following the manufacturer's protocol. Briefly, slides were incubated at 60°C for 30 minutes and deparaffinized as described. Sections were treated with Proteinase K (DakoCytomation) for 30 minutes at room temperature, rinsed several times with dH₂O, and immersed in 95% ethanol for 10 seconds before air-drying. Slides were pre-hybridized at 54°C for 1 hour with *in situ* hybridization buffer (Enzo Life Sciences, Inc. Farmingdale, NY) before an overnight 54°C incubation in buffer containing either 5'-biotin labeled *miR*-*224* miRCURY™ LNA detection probe (Exiqon, Woburn, MA) or scrambled negative control probe (Exiqon) at 50 nM final concentration. Slides were washed in both TBST and GenPoint™ stringent wash solution (54°C for 30 minutes). Slides were then exposed to N₂O₂ blocking solution (DakoCytomation) for 20 minutes and further blocked in a blocking buffer (DakoCytomation, X0909) for 30 minutes before being exposed to primary Streptavidin-HRP antibody, biotinyl tyramide, secondary Streptavidin-HRP antibody, and DAB chromogen solutions following the manufacturer's protocol. Slides were then briefly counterstained in hematoxylin and rinsed with both TBST and water before mounting. A pathologist evaluated the ISH intensity of *miR*-*224* in perineural and nonperineural cancer cells using the same criteria that were used for immunohistochemistry.

### Pathway analysis.

This analysis was performed with the in-house WPS software. Pathways were annotated according to Gene Ontology Biological Processes (GOBP) (Gene Ontology Consortium: geneontology.org). Our database had 16,762 human genes annotated for GOBP. Genes were included into the pathway analysis based on the FDR (≤ 30%) of their corresponding probesets on the microarray. If several probesets encoded the same gene, the software recognized this and assured that the gene was counted only once for significance testing at the pathway level. A one-sided Fisher's exact test was used to determine which biological processes had a statistically significant enrichment of differently expressed genes (P < 0.05). We compiled the Fisher's exact test results for cluster analyses and displayed the results in color-coded heat maps to reveal the patterns of significantly altered biological processes. The color coding of the heat maps is related to the enrichment of genes in a biological process (-Log(*P* value)-based) with red indicating a higher enrichment.

### RESULTS for EXAMPLE II

### Clinical samples and gene expression analysis.

We collected macro-dissected tumor specimens from radical prostatectomies of 57 prostate cancer patients **(****Figure 22** **- Table 8).** Seven (12%) of the tumors were negative for PNI. Consistent with the literature, those tumors had a smaller size and a lower Gleason score than PNI-positive tumors. In addition, all PNI-negative tumors were confined to the prostate. We investigated the gene expression differences between tumors with PNI and those that were negative for PNI. Gene expression profiles from these tumors were generated using both a custom microRNA microarray that represents 235 human microRNAs and the Affymetrix GeneChip HG-U133A 2.0 array that represents approximately 13,000 human protein-coding genes.

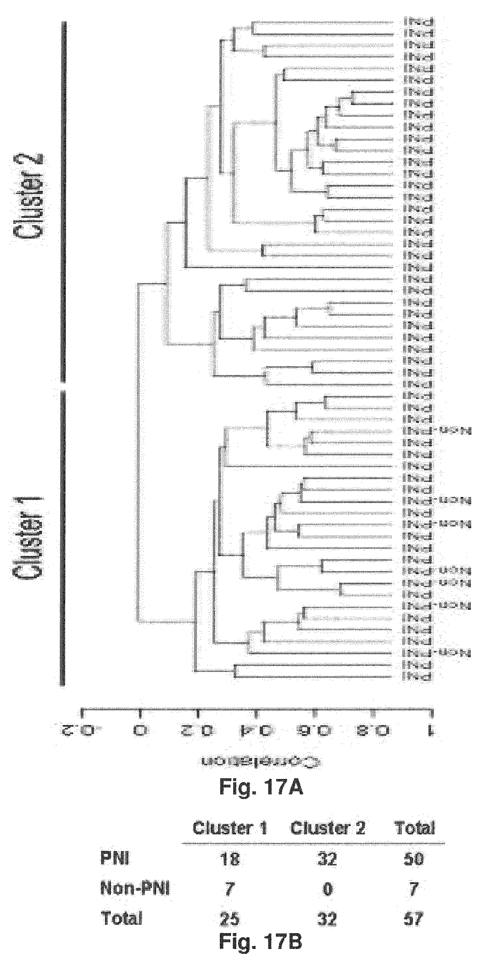

In an initial analysis of our dataset, we applied unsupervised hierarchical clustering to examine whether expression of microRNAs and mRNAs can distinguish between tumors with PNI and those without PNI. Hierarchical clustering based on the global expression of mRNA did not separate PNI cases from non-PNI cases (data not shown). However, the expression patterns of the microRNAs in these samples yielded two prominent clusters with distinct microRNA profiles **(****Figures 17A-17B****).** Cluster #1 contained all non-PNI tumors and a subgroup of tumors with PNI. Cluster #2 contained PNI tumors that were significantly more likely to have a high Gleason score (≥ 7) and an extraprostatic disease extension than tumors in cluster #1 *(P* < 0.05, respectively; two-sided Fisher's exact test).

Significance analysis of microarray data revealed that 19 microRNAs and 34 protein-coding genes were significantly differently expressed between PNI and non-PNI tumors at a FDR ≤ 10%. At this threshold, all microRNAs were up-regulated in tumors with PNI **(****Figure 23** **- Table 9),** while all mRNAs had a lower expression in PNI tumors than in non-PNI tumors **(****Figure 24** **- Table 10).**

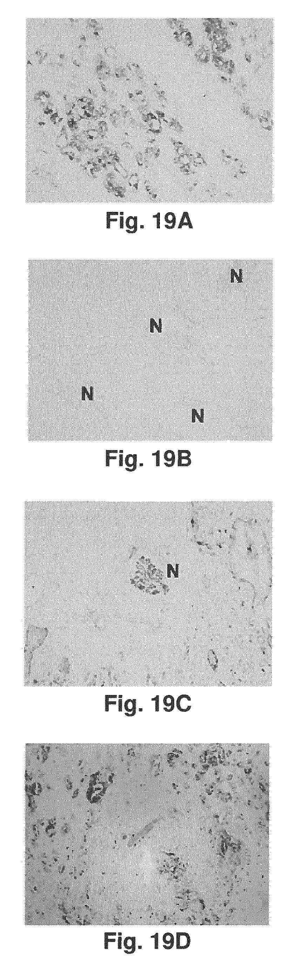

This list of differently expressed microRNAs was unique to the comparison between PNI and non-PNI tumors in our dataset. None of these microRNAs were significantly differently expressed by either tumor grade or stage (FDR < 30%). In contrast to the PNI to non-PNI comparison, only very few microRNAs were significantly differently expressed between high (sum score 7-9) and low (sum score 5-6) Gleason score, e.g., miR-1 was down-regulated in tumors with high Gleason score, and between organ-confined and those with extraprostatic extension. Among the protein-coding genes that were differently expressed between PNI and non-PNI tumors, many encoded either metallothioneins (metallothionein 1F, 1G, 1H, 1M, 1X, 2A) or proteins with mitochondrial localization (4-aminobutyrate aminotransferase, ferrochelatase, long chain acyl-coenzyme A dehydrogenase, mitochondrial ribosomal proteins L39/S1). A subset of these genes was also down-regulated in tumors with a high Gleason score when compared with low Gleason score tumors **(****Figures 19A-19D****).** There was no overlap with genes differently expressed by tumor stage.

### Validation of microarray data by qRT-PCR.

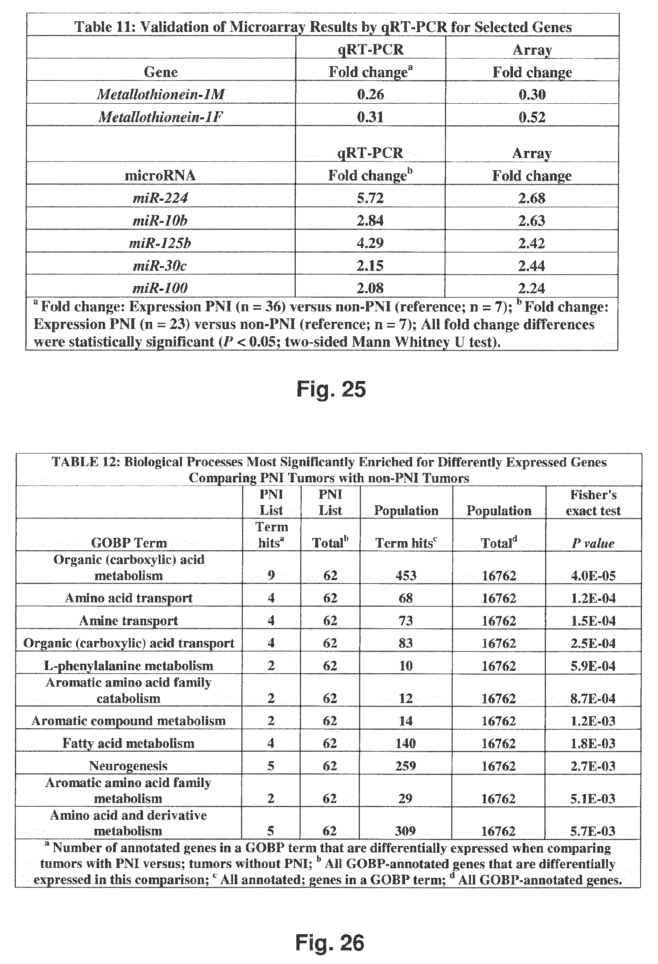

Five microRNAs and two mRNAs were chosen for validation by qRT-PCR **(****Figure 25** **- Table 11).** Consistent with the microarray data, we found a significantly higher expression of mature miR-224, miR-10, miR-125b, miR-30c, and miR-100 in PNI tumors when compared with non-PNI tumors. The transcript levels of the metallothioneins 1M and 1F were significantly lower in PNI tumors when compared with non-PNI tumors, which is also consistent with our microarray data.

### Pathway association of protein-coding genes that are differently expressed by PNI status.

We performed a pathway analysis based on those GOBP-annotated genes (n = 62) whose mRNA was differently expressed between PNI and non-PNI tumors at a FDR ≤ 30%. The analysis revealed a number of biological processes that were enriched for differently expressed genes comparing PNI tumors with non-PNI tumors. The most significantly altered biological processes included transport and metabolism of organic (carboxylic) acids/fatty acids, amino acids, and (poly)amines **(****Figure 26** **- Table 12).** They also included the biological process of "neurogenesis", which is consistent with the known interaction between tumor cells and nerves in PNI.

A cluster analysis was performed to identify biological processes that are enriched for differently expressed genes by tumor PNI status (PNI-positive versus PNI-negative), but not by Gleason score (high versus low Gleason score), pathological stage (pT3 versus pT2), or by the presence of extraprostatic extension (yes versus no). As shown by a heatmap, the analysis identified a number of biological processes that were uniquely enriched for differently expressed genes comparing PNI-positive with PNI-negative tumors **(****Figure 18****).** These biological processes included metabolism and transport of organic (carboxylic) acids/fatty acids, amino acids, and (poly)amines, as described before, but also processes related to the negative regulation of programmed cell death.

### Expression of metallothionein, coxsackie adenovirus receptor, and miR-224 is perineural cancer cells.

Although our microarray-based analysis indicated that PNI and non-PNI tumors differ in their gene expression pattern, this approach is not informative with respect to the expression of these genes in perineural and nonperineural cancer cells. We used immunohistochemistry and in situ hybridization to investigate the relative expression of two protein-coding genes, metallothionein (metallothionein-1 and -2) and coxsackie adenovirus receptor (CXADR), and of *miR-224* in perineural and nonperineural cancer cells. Immunohistochemistry was performed on sections from 14 tumors that contained representative areas for perineural and nonperineural cancer cells. *In situ* hybridization was performed on sections from 11 tumors.

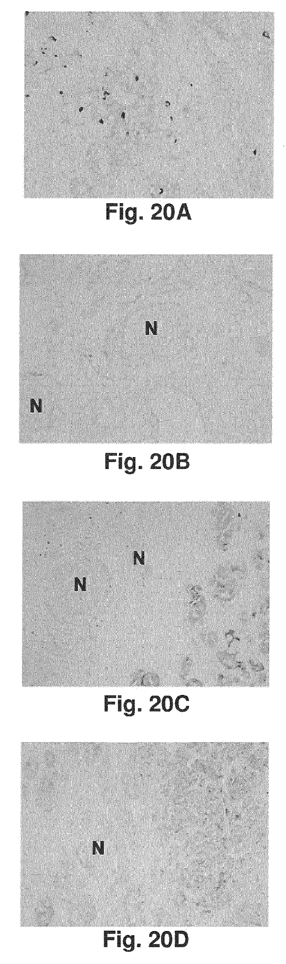

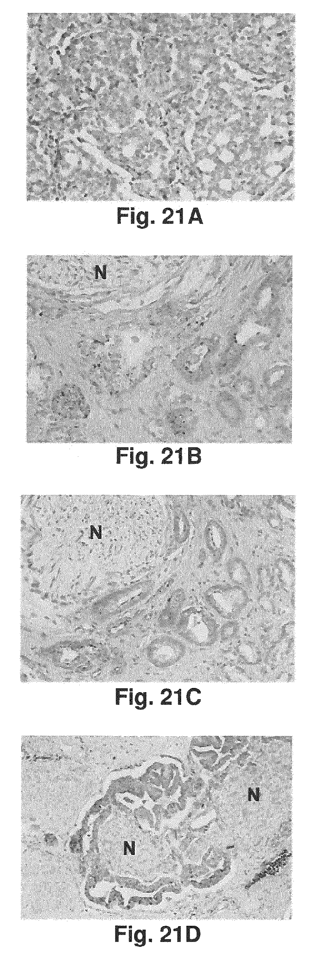

Metallothionein, CXADR and miR-224 were found to be expressed in the tumor epithelium **(****Figures 19A-19D****,** **Figures 20A-20D** and **Figures 21A-21D****).** The labeling pattern for metallothionein (epithelial, cytoplasmic, nuclear) and CXADR (epithelial, membranous, cytoplasmic) was consistent with that described by others. A lower expression of metallothionein and CXADR was observed in perineural cancer cells of 6 tumors (43%) and 7 tumors (50%), respectively, when compared with nonperineural cancer cells in the same tissues **(****Figures 19A-19D****,** **Figures 20A-20D****).** No difference was detected in the other tumors with the exception of one (7%) where the expression of metallothionein was scored to be higher in perineural cancer cells than nonperineural cancer cells. A marked increased expression of *miR-224* in perineural cancer cells was observed in 4 tumors (36%) **(****Figures 21A-21D****).** No such difference was seen in the other 7 tumors where *miR*-*224* expression was mostly low to undetectable in the tumor epithelium.

### DISCUSSION of EXAMPLE II

We investigated the gene expression profiles of PNI and non-PNI tumors and found significant differences in microRNA and mRNA expression between them. Most strikingly, unsupervised hierarchical cluster analysis based on the expression of 235 microRNAs yielded two main tumor clusters, one of which contained all non-PNI tumors. We could not achieve such a classification based on the expression of 13,000 protein-coding transcripts which is in agreement with other studies that could not find an mRNA expression signature associated with local invasion in prostate cancer.

Our findings show that microRNA expression could be a more distinctive feature of PNI tumors, when compared with non-PNI tumors, than mRNA expression. These findings are consistent with previous reports showing that microRNA expression profiles can be superior to mRNA expression profiles in classifying tumors by developmental lineage and differentiation state.

Nineteen microRNAs were found to be higher expressed in PNI tumors than non-PNI tumors. Of those, miR-10, miR-21, and miR-125b are candidate oncogenes. Furthermore, miR-21 and miR-224 are located in malignancy-associated chromosomal regions that were found to have an increased gene expression in human prostate cancer. A microRNA expression signature common to several human solid cancers, including prostate cancer, has been described. The shared microRNAs between that study and our PNI signature are miR-21, miR-24, and miR-30c. Most notable, however, is the overlap of the PNI signature with other microRNA signatures that were discovered under experimental conditions.

Hypoxia has been found to induce miR-24, miR-26, miR-27, and miR-181. Those microRNAs are also upregulated in PNI tumors. Even more prominent are the similarities between the PNI signature and an inflammation-induced microRNA signature in lungs of LPS-treated mice. Here, LPS induced miR-21, miR-27b, miR-100, and miR-224, among several other microRNAs. Thus, the observed PNI microRNA signature could be partly the result of a pro-inflammatory environment and hypoxia in the cancerous prostate.

To evaluate the possibility of confounding effects by tumor grade and stage in the PNI signature, we compared the list of differently expressed microRNAs between PNI and non-PNI tumors with the same lists comparing high with low Gleason score tumors and organ-confined tumors with tumors that showed extraprostatic extension. This additional analysis revealed that the PNI signature was not shared by these two contrasts. Instead, only very few microRNAs were found to be significantly differently expressed by tumor grade and stage. The heterogeneous nature of prostate tumors may have limited our ability to find a microRNA signature associated with these two prognostic factors. Alternatively, the PNI signature could be very distinct and unique to the transition of non-PNI to PNI and may specifically involve the interaction between nerve and cancer cells. This signature could also be a transient phenomenon of cancer cells and disappears when these cells disseminate from their perineural location. We analyzed the expression of miR-224, the most differently expressed microRNA by PNI status, in perineural and nonperineural cancer cells and found an increased expression of it in perineural cancer cells in a subset of the tumors. Although not all tumors showed upregulation of miR-224 in perineural cancer cells, the observation indicates that mechanisms by which cancer cells adhere to nerves could be involved in the induction of miR-224.

Analysis of the mRNA expression profile revealed 34 genes that were down-regulated in PNI tumors at a FDR threshold of ≤ 10%. Even though we observed genes that were higher expressed in PNI tumors than non-PNI tumors, e.g., *CRISP3, PSCA, BMP7,* or *BCL2,* their high FDR excluded them from our list of significantly differently expressed genes. Only two other studies, using a co-culture model of DU-145 prostate cancer cells with neuronal cells, examined the expression profile of mRNA associated with PNI. Those studies discovered that the genes encoding bystin and Pim-2 are upregulated in PNI. We did not detect an increase of the corresponding mRNAs in PNI tumors. Different methodologies may explain some of the differences among the gene lists generated in the various studies. In addition, our chip did not contain probesets for the gene encoding bystin.

Several of the 34 differently expressed genes were members of the metallothionein gene family. These genes are located in a gene cluster on chromosome 16q13 and have been found to be down-regulated in prostate cancer by promoter hypermethylation and reduced zinc availability. By immunohistochemistry, we could confirm that metallothionein expression is noticeably lower in perineural cancer cells when compared with nonperineural cancer cells in a subset of the prostate tumors. The down-regulation at the transition from a non-PNI tumor to a PNI tumor may indicate important changes in the metal metabolism of cancer cells that take place at this stage of the disease. Several other genes in our list of differently expressed genes encode proteins with mitochondrial localization, e.g., 4-aminobutyrate aminotransferase, ferrochelatase, and long chain acyl-coenzyme A dehydrogenase, among others. The aminobutyrate aminotransferase and the long chain acyl-coenzyme A dehydrogenase are key genes in the organic (carboxylic) acid metabolism (e.g., ketone body, fatty acid) of cells, whereas the ferrochelatase is involved in the biosynthesis of heme. Alterations in metabolism and in the genome of mitochondria are common events in prostate carcinogenesis. Our data show that some of these changes may occur at the transition into a PNI-positive tumor.

Other genes that were found to be down-regulated in PNI tumors were those encoding the spermine synthase, the v-MAF oncogene homolog (MAF), and CXADR. Spermine synthase is a key enzyme of the polyamine synthesis pathway that catalyzes the conversion of spermidine into spermine. A transcriptional dysregulation of the polyamine synthesis pathway in prostate cancer has been observed. Spermine is an endogenous inhibitor of prostate carcinoma cell growth. Therefore, down-regulation of the spermine synthase may allow increased growth and survival of prostate cancer cells in a perineural environment. MAF is an oncogene in lymphomas and myelomas, but it was found to be a candidate tumor suppressor gene in prostate cancer. CXADR has a crucial function in the uptake of adenoviruses into human cells. This receptor was found to be down-regulated in locally advanced prostate cancer when compared with normal prostate.

Because single gene effects are unlikely to cause PNI, we conducted a pathway analysis for the protein-coding genes that were differently expressed between PNI tumors and non-PNI tumors. This analysis revealed that the most significantly altered biological processes in PNI tumors, when compared to non-PNI tumors, are those that regulate cell and energy metabolism. Other altered biological processes related to neuronal functions, such as neurogenesis and the transmission of nerve impulse, and to the negative regulation of cell death. The latter is consistent with previous findings that prostate cancer cells in a perineural location show decreased apoptosis and increased survival.

We observed significant alterations in microRNA and mRNA expression at the transition from a non-PNI tumor to a PNI tumor. Unsupervised hierarchical clustering revealed that non-PNI tumors are more distinct from PNI tumors by their microRNA expression profile than by their mRNA expression profile. We also identified various genes and biological processes related to mitochondrial function and cell metabolism that could be functionally significant in PNI.

### EXAMPLES of USES and DEFINITIONS THEREOF

The practice of the present invention will employ, unless otherwise indicated, conventional methods of pharmacology, chemistry, biochemistry, recombinant DNA techniques and immunology, within the skill of the art. Such techniques are explained fully in the literature. See, e.g., Handbook of Experimental Immunology, Vols. I-IV (D. M. Weir and C. C. Blackwell eds., Blackwell Scientific Publications); A. L. Lehninger, Biochemistry (Worth Publishers, Inc., current addition); Sambrook, et al., Molecular Cloning: A Laboratory Manual (2nd Edition, 1989); Methods In Enzymology (S. Colowick and N. Kaplan eds., Academic Press, Inc.).

As such, the definitions herein are provided for further explanation and are not to be construed as limiting.

The articles "a" and "an" are used herein to refer to one or to more than one (i.e., to at least one) of the grammatical object of the article. By way of example, "an element" means one element or more than one element.

A "marker" and "biomarker" is a gene and/or protein and/or functional variants thereof o whose altered level of expression in a tissue or cell from its expression level in normal or healthy tissue or cell is associated with a disorder and/or disease state.

The "normal" level of expression of a marker is the level of expression of the marker in cells of a human subject or patient not afflicted with a disorder and/or disease state.

An "over-expression" or "significantly higher level of expression" of a marker refers to an expression level in a test sample that is greater than the standard error of the assay employed to assess expression, and in certain embodiments, at least twice, and in other embodiments, three, four, five or ten times the expression level of the marker in a control sample (e.g., sample from a healthy subject not having the marker associated disorder and/or disease state) and in certain embodiments, the average expression level of the marker in several control samples.

A "significantly lower level of expression" of a marker refers to an expression level in a test sample that is at least twice, and in certain embodiments, three, four, five or ten times lower than the expression level of the marker in a control sample (e.g., sample from a healthy subject not having the marker associated disorder and/or disease state) and in certain embodiments, the average expression level of the marker in several control samples.

A kit is any manufacture (e.g. a package or container) comprising at least one reagent, e.g., a probe, for specifically detecting the expression of a marker. The kit may be promoted, distributed or sold as a unit for performing the methods of the present invention.

"Proteins" encompass marker proteins and their fragments; variant marker proteins and their fragments; peptides and polypeptides comprising an at least 15 amino acid segment of a marker or variant marker protein; and fusion proteins comprising a marker or variant marker protein, or an at least 15 amino acid segment of a marker or variant marker protein.

The compositions, kits and methods described herein have the following non-limiting uses, among others:
1) assessing whether a subject is afflicted with a disorder and/or disease state;
2) assessing the stage of a disorder and/or disease state in a subject;
3) assessing the grade of a disorder and/or disease state in a subject;
4) assessing the nature of a disorder and/or disease state in a subject;
5) assessing the potential to develop a disorder and/or disease state in a subject;
6) assessing the histological type of cells associated with a disorder and/or disease state in a subject;
7) making antibodies, antibody fragments or antibody derivatives that are useful for treating a disorder and/or disease state in a subject;
8) assessing the presence of a disorder and/or disease state in a subject's cells;
9) assessing the efficacy of one or more test compounds for inhibiting a disorder and/or disease state in a subject;
10) assessing the efficacy of a therapy for inhibiting a disorder and/or disease state in a subject;
11) monitoring the progression of a disorder and/or disease state in a subject;
12) selecting a composition or therapy for inhibiting a disorder and/or disease state in a subject;
13) treating a subject afflicted with a disorder and/or disease state;
14) inhibiting a disorder and/or disease state in a subject;
15) assessing the harmful potential of a test compound; and
16) preventing the onset of a disorder and/or disease state in a subject at risk therefor.

### Screening Methods

Animal models can be created to enable screening of therapeutic agents useful for treating or preventing a disorder and/or disease state in a subject. Accordingly, the methods are useful for identifying therapeutic agents for treating or preventing a disorder and/or disease state in a subject. The methods comprise administering a candidate agent to an animal model made by the methods described herein, and assessing at least one response in the animal model as compared to a control animal model to which the candidate agent has not been administered. If at least one response is reduced in symptoms or delayed in onset, the candidate agent is an agent for treating or preventing the disease.

The candidate agents may be pharmacologic agents already known in the art or may be agents previously unknown to have any pharmacological activity. The agents may be naturally arising or designed in the laboratory. They may be isolated from microorganisms, animals or plants, or may be produced recombinantly, or synthesized by any suitable chemical method. They may be small molecules, nucleic acids, proteins, peptides or peptidomimetics. In certain embodiments, candidate agents are small organic compounds having a molecular weight of more than 50 and less than about 2,500 daltons. Candidate agents comprise functional groups necessary for structural interaction with proteins. Candidate agents are also found among biomolecules including, but not limited to: peptides, saccharides, fatty acids, steroids, purines, pyrimidines, derivatives, structural analogs or combinations thereof.

Candidate agents are obtained from a wide variety of sources including libraries of synthetic or natural compounds. There are, for example, numerous means available for random and directed synthesis of a wide variety of organic compounds and biomolecules, including expression of randomized oligonucleotides and oligopeptides. Alternatively, libraries of natural compounds in the form of bacterial, fungal, plant and animal extracts are available or readily produced. Additionally, natural or synthetically produced libraries and compounds are readily modified through conventional chemical, physical and biochemical means, and may be used to produce combinatorial libraries. In certain embodiments, the candidate agents can be obtained using any of the numerous approaches in combinatorial library methods art, including, by non-limiting example: biological libraries; spatially addressable parallel solid phase or solution phase libraries; synthetic library methods requiring deconvolution; the "one-bead one-compound" library method; and synthetic library methods using affinity chromatography selection.

In certain further embodiments, certain pharmacological agents may be subjected to directed or random chemical modifications, such as acylation, alkylation, esterification, amidification, etc. to produce structural analogs.

The same methods for identifying therapeutic agents for treating a disorder and/or disease state in a subject can also be used to validate lead compounds/agents generated from in vitro studies.

The candidate agent may be an agent that up- or down-regulates one or more of a disorder and/or disease state in a subject response pathway. In certain embodiments, the candidate agent may be an antagonist that affects such pathway.

### Methods for Treating a Disorder and/or Disease State

There is provided herein methods for treating, inhibiting, relieving or reversing a disorder and/or disease state response. In the methods described herein, an agent that interferes with a signaling cascade is administered to an individual in need thereof, such as, but not limited to, subjects in whom such complications are not yet evident and those who already have at least one such response.

In the former instance, such treatment is useful to prevent the occurrence of such response and/or reduce the extent to which they occur. In the latter instance, such treatment is useful to reduce the extent to which such response occurs, prevent their further development or reverse the response.

In certain embodiments, the agent that interferes with the response cascade may be an antibody specific for such response.

### Expression of Biomarker(s)

Expression of a marker can be inhibited in a number of ways, including, by way of a non-limiting example, an antisense oligonucleotide can be provided to the disease cells in order to inhibit transcription, translation, or both, of the marker(s). Alternately, a polynucleotide encoding an antibody, an antibody derivative, or an antibody fragment which specifically binds a marker protein, and operably linked with an appropriate promoter/regulator region, can be provided to the cell in order to generate intracellular antibodies which will inhibit the function or activity of the protein. The expression and/or function of a marker may also be inhibited by treating the disease cell with an antibody, antibody derivative or antibody fragment that specifically binds a marker protein. Using the methods described herein, a variety of molecules, particularly including molecules sufficiently small that they are able to cross the cell membrane, can be screened in order to identify molecules which inhibit expression of a marker or inhibit the function of a marker protein. The compound so identified can be provided to the subject in order to inhibit disease cells of the subject.

Any marker or combination of markers, as well as any certain markers in combination with the markers, may be used in the compositions, kits and methods described herein. In general, it is desirable to use markers for which the difference between the level of expression of the marker in disease cells and the level of expression of the same marker in normal system cells is as great as possible. Although this difference can be as small as the limit of detection of the method for assessing expression of the marker, it is desirable that the difference be at least greater than the standard error of the assessment method, and, in certain embodiments, a difference of at least 2-, 3-, 4-, 5-, 6-, 7-, 8-, 9-, 10-, 15-, 20-, 100-, 500-, 1000-fold or greater than the level of expression of the same marker in normal tissue.

It is recognized that certain marker proteins are secreted to the extracellular space surrounding the cells. These markers are used in certain embodiments of the compositions, kits and methods, owing to the fact that such marker proteins can be detected in a body fluid sample, which may be more easily collected from a human subject than a tissue biopsy sample. In addition, in vivo techniques for detection of a marker protein include introducing into a subject a labeled antibody directed against the protein. For example, the antibody can be labeled with a radioactive marker whose presence and location in a subject can be detected by standard imaging techniques.

In order to determine whether any particular marker protein is a secreted protein, the marker protein is expressed in, for example, a mammalian cell, such as a human cell line, extracellular fluid is collected, and the presence or absence of the protein in the extracellular fluid is assessed (e.g. using a labeled antibody which binds specifically with the protein).

It will be appreciated that subject samples containing such cells may be used in the methods described herein. In these embodiments, the level of expression of the marker can be assessed by assessing the amount (e.g., absolute amount or concentration) of the marker in a sample. The cell sample can, of course, be subjected to a variety of post-collection preparative and storage techniques (e.g., nucleic acid and/or protein extraction, fixation, storage, freezing, ultrafiltration, concentration, evaporation, centrifugation, etc.) prior to assessing the amount of the marker in the sample.

It will also be appreciated that the markers may be shed from the cells into, for example, the respiratory system, digestive system, the blood stream and/or interstitial spaces. The shed markers can be tested, for example, by examining the sputum, BAL, serum, plasma, urine, stool, etc.

The compositions, kits and methods can be used to detect expression of marker proteins having at least one portion which is displayed on the surface of cells which express it. For example, immunological methods may be used to detect such proteins on whole cells, or computer-based sequence analysis methods may be used to predict the presence of at least one extracellular domain (i.e., including both secreted proteins and proteins having at least one cell-surface domain). Expression of a marker protein having at least one portion which is displayed on the surface of a cell which expresses it may be detected without necessarily lysing the cell (e.g., using a labeled antibody which binds specifically with a cell-surface domain of the protein).

Expression of a marker may be assessed by any of a wide variety of methods for detecting expression of a transcribed nucleic acid or protein. Non-limiting examples of such methods include immunological methods for detection of secreted, cell-surface, cytoplasmic or nuclear proteins, protein purification methods, protein function or activity assays, nucleic acid hybridization methods, nucleic acid reverse transcription methods and nucleic acid amplification methods.

In a particular embodiment, expression of a marker is assessed using an antibody (e.g., a radio-labeled, chromophore-labeled, fluorophore-labeled or enzyme-labeled antibody), an antibody derivative (e.g., an antibody conjugated with a substrate or with the protein or ligand of a protein-ligand pair), or an antibody fragment (e.g., a single-chain antibody, an isolated antibody hypervariable domain, etc.) which binds specifically with a marker protein or fragment thereof, including a marker protein which has undergone all or a portion of its normal post-translational modification.

In another particular embodiment, expression of a marker is assessed by preparing mRNA/cDNA (i.e., a transcribed polynucleotide) from cells in a subject sample, and by hybridizing the mRNA/cDNA with a reference polynucleotide which is a complement of a marker nucleic acid, or a fragment thereof. cDNA can, optionally, be amplified using any of a variety of polymerase chain reaction methods prior to hybridization with the reference polynucleotide; preferably, it is not amplified. Expression of one or more markers can likewise be detected using quantitative PCR to assess the level of expression of the marker(s). Alternatively, any of the many methods of detecting mutations or variants (e.g., single nucleotide polymorphisms, deletions, etc.) of a marker may be used to detect occurrence of a marker in a subject.

In a related embodiment, a mixture of transcribed polynucleotides obtained from the sample is contacted with a substrate having fixed thereto a polynucleotide complementary to or homologous with at least a portion (e.g., at least 7, 10, 15, 20, 25, 30, 40, 50, 100, 500, or more nucleotide residues) of a marker nucleic acid. If polynucleotides complementary to or homologous with are differentially detectable on the substrate (e.g., detectable using different chromophores or fluorophores, or fixed to different selected positions), then the levels of expression of a plurality of markers can be assessed simultaneously using a single substrate (e.g., a "gene chip" microarray of polynucleotides fixed at selected positions). When a method of assessing marker expression is used which involves hybridization of one nucleic acid with another, it is desired that the hybridization be performed under stringent hybridization conditions.

In certain embodiments, the biomarker assays can be performed using mass spectrometry or surface plasmon resonance. In various embodiments, the method of identifying an agent active against a disorder and/or disease state in a subject can include one or more of: a) providing a sample of cells containing one or more markers or derivative thereof; b) preparing an extract from such cells; c) mixing the extract with a labeled nucleic acid probe containing a marker binding site; and, d) determining the formation of a complex between the marker and the nucleic acid probe in the presence or absence of the test agent. The determining step can include subjecting said extract/nucleic acid probe mixture to an electrophoretic mobility shift assay.

In certain embodiments, the determining step comprises an assay selected from an enzyme linked immunoabsorption assay (ELISA), fluorescence based assays and ultra high throughput assays, for example surface plasmon resonance (SPR) or fluorescence correlation spectroscopy (FCS) assays. In such embodiments, the SPR sensor is useful for direct real-time observation of biomolecular interactions since SPR is sensitive to minute refractive index changes at a metal-dielectric surface. SPR is a surface technique that is sensitive to changes of 10⁵ to 10⁻⁶ refractive index (RI) units within approximately 200 nm of the SPR sensor/sample interface. Thus, SPR spectroscopy is useful for monitoring the growth of thin organic films deposited on the sensing layer.

Because the compositions, kits, and methods rely on detection of a difference in expression levels of one or more markers, it is desired that the level of expression of the marker is significantly greater than the minimum detection limit of the method used to assess expression in at least one of normal cells and cancer-affected cells.

It is understood that by routine screening of additional subject samples using one or more of the markers, it will be realized that certain of the markers are over-expressed in cells of various types, including a specific disorder and/or disease state in a subject.

In addition, as a greater number of subject samples are assessed for expression of the markers and the outcomes of the individual subjects from whom the samples were obtained are correlated, it will also be confirmed that altered expression of certain of the markers are strongly correlated with a disorder and/or disease state in a subject and that altered expression of other markers are strongly correlated with other diseases. The compositions, kits, and methods are thus useful for characterizing one or more of the stage, grade, histological type, and nature of a disorder and/or disease state in a subject.

When the compositions, kits, and methods are used for characterizing one or more of the stage, grade, histological type, and nature of a disorder and/or disease state in a subject, it is desired that the marker or panel of markers is selected such that a positive result is obtained in at least about 20%, and in certain embodiments, at least about 40%, 60%, or 80%, and in substantially all subjects afflicted with a disorder and/or disease state of the corresponding stage, grade, histological type, or nature. The marker or panel of markers invention can be selected such that a positive predictive value of greater than about 10% is obtained for the general population (in a non-limiting example, coupled with an assay specificity greater than 80%).

When a plurality of markers are used in the compositions, kits, and methods, the level of expression of each marker in a subject sample can be compared with the normal level of expression of each of the plurality of markers in non-disorder and/or non-disease samples of the same type, either in a single reaction mixture (i.e. using reagents, such as different fluorescent probes, for each marker) or in individual reaction mixtures corresponding to one or more of the markers. In one embodiment, a significantly increased level of expression of more than one of the plurality of markers in the sample, relative to the corresponding normal levels, is an indication that the subject is afflicted with a disorder and/or disease state. When a plurality of markers is used, 2, 3, 4, 5, 8, 10, 12, 15, 20, 30, or 50 or more individual markers can be used; in certain embodiments, the use of fewer markers may be desired.

In order to maximize the sensitivity of the compositions, kits, and methods (i.e. by interference attributable to cells of system origin in a subject sample), it is desirable that the marker used therein be a marker which has a restricted tissue distribution, e.g., normally not expressed in a non-system tissue.

It is recognized that the compositions, kits, and methods will be of particular utility to subjects having an enhanced risk of developing a disorder and/or disease state in a subject and their medical advisors. Subjects recognized as having an enhanced risk of developing a disorder and/or disease include, for example, subjects having a familial history of such disorder or disease.

The level of expression of a marker in normal human system tissue can be assessed in a variety of ways. In one embodiment, this normal level of expression is assessed by assessing the level of expression of the marker in a portion of system cells which appear to be normal and by comparing this normal level of expression with the level of expression in a portion of the system cells which is suspected of being abnormal. Alternately, and particularly as further information becomes available as a result of routine performance of the methods described herein, population-average values for normal expression of the markers may be used. In other embodiments, the 'normal' level of expression of a marker may be determined by assessing expression of the marker in a subject sample obtained from a non-afflicted subject, from a subject sample obtained from a subject before the suspected onset of a disorder and/or disease state in the subject, from archived subject samples, and the like.

There is also provided herein compositions, kits, and methods for assessing the presence of disorder and/or disease state cells in a sample (e.g. an archived tissue sample or a sample obtained from a subject). These compositions, kits, and methods are substantially the same as those described above, except that, where necessary, the compositions, kits, and methods are adapted for use with samples other than subject samples. For example, when the sample to be used is a parafinized, archived human tissue sample, it can be necessary to adjust the ratio of compounds in the compositions, in the kits, or the methods used to assess levels of marker expression in the sample.

### Kits nnd Rengents

The kits are useful for assessing the presence of disease cells (e.g. in a sample such as a subject sample). The kit comprises a plurality of reagents, each of which is capable of binding specifically with a marker nucleic acid or protein. Suitable reagents for binding with a marker protein include antibodies, antibody derivatives, antibody fragments, and the like. Suitable reagents for binding with a marker nucleic acid (e.g. a genomic DNA, an MRNA, a spliced MRNA, a cDNA, or the like) include complementary nucleic acids. For example, the nucleic acid reagents may include oligonucleotides (labeled or non-labeled) fixed to a substrate, labeled oligonucleotides not bound with a substrate, pairs of PCR primers, molecular beacon probes, and the like.

The kits may optionally comprise additional components useful for performing the methods described herein. By way of example, the kit may comprise fluids (e.g. SSC buffer) suitable for annealing complementary nucleic acids or for binding an antibody with a protein with which it specifically binds, one or more sample compartments, an instructional material which describes performance of the method, a sample of normal system cells, a sample of cancer-related disease cells, and the like.

### Methods of Producing Antibodies

There is also provided herein a method of making an isolated hybridoma which produces an antibody useful for assessing whether a subject is afflicted with a disorder and/or disease state. In this method, a protein or peptide comprising the entirety or a segment of a marker protein is synthesized or isolated (e.g. by purification from a cell in which it is expressed or by transcription and translation of a nucleic acid encoding the protein or peptide in vivo or in vitro). A vertebrate, for example, a mammal such as a mouse, rat, rabbit, or sheep, is immunized using the protein or peptide. The vertebrate may optionally (and preferably) be immunized at least one additional time with the protein or peptide, so that the vertebrate exhibits a robust immune response to the protein or peptide. Splenocytes are isolated from the immunized vertebrate and fused with an immortalized cell line to form hybridomas, using any of a variety of methods. Hybridomas formed in this manner are then screened using standard methods to identify one or more hybridomas which produce an antibody which specifically binds with the marker protein or a fragment thereof. There is also provided herein hybridomas made by this method and antibodies made using such hybridomas.

### Methods of Assessing Efficacy

There is also provided herein a method of assessing the efficacy of a test compound for inhibiting disease cells. As described above, differences in the level of expression of the markers correlate with the abnormal state of the subject's cells. Although it is recognized that changes in the levels of expression of certain of the markers likely result from the abnormal state of such cells, it is likewise recognized that changes in the levels of expression of other of the markers induce, maintain, and promote the abnormal state of those cells. Thus, compounds which inhibit a disorder and/or disease state in a subject will cause the level of expression of one or more of the markers to change to a level nearer the normal level of expression for that marker (i.e. the level of expression for the marker in normal cells).

This method thus comprises comparing expression of a marker in a first cell sample and maintained in the presence of the test compound and expression of the marker in a second cell sample and maintained in the absence of the test compound. A significantly reduced expression of a marker in the presence of the test compound is an indication that the test compound inhibits a related disease. The cell samples may, for example, be aliquots of a single sample of normal cells obtained from a subject, pooled samples of normal cells obtained from a subject, cells of a normal cell line, aliquots of a single sample of related disease cells obtained from a subject, pooled samples of related disease cells obtained from a subject, cells of a related disease cell line, or the like.

In one embodiment, the samples are cancer-related disease cells obtained from a subject and a plurality of compounds believed to be effective for inhibiting various cancer-related diseases are tested in order to identify the compound which is likely to best inhibit the cancer-related disease in the subject.

This method may likewise be used to assess the efficacy of a therapy for inhibiting a related disease in a subject. In this method, the level of expression of one or more markers in a pair of samples (one subjected to the therapy, the other not subjected to the therapy) is assessed. As with the method of assessing the efficacy of test compounds, if the therapy induces a significantly lower level of expression of a marker then the therapy is efficacious for inhibiting a cancer-related disease. As above, if samples from a selected subject are used in this method, then alternative therapies can be assessed in vitro in order to select a therapy most likely to be efficacious for inhibiting a cancer-related disease in the subject.

As described herein, the abnormal state of human cells is correlated with changes in the levels of expression of the markers. There is also provided a method for assessing the harmful potential of a test compound. This method comprises maintaining separate aliquots of human cells in the presence and absence of the test compound. Expression of a marker in each of the aliquots is compared. A significantly higher level of expression of a marker in the aliquot maintained in the presence of the test compound (relative to the aliquot maintained in the absence of the test compound) is an indication that the test compound possesses a harmful potential. The relative harmful potential of various test compounds can be assessed by comparing the degree of enhancement or inhibition of the level of expression of the relevant markers, by comparing the number of markers for which the level of expression is enhanced or inhibited, or by comparing both. Various aspects are described in further detail in the following subsections.

### Isolated Proteins and Antibodies

One aspect pertains to isolated marker proteins and biologically active portions thereof, as well as polypeptide fragments suitable for use as immunogens to raise antibodies directed against a marker protein or a fragment thereof. In one embodiment, the native marker protein can be isolated from cells or tissue sources by an appropriate purification scheme using standard protein purification techniques. In another embodiment, a protein or peptide comprising the whole or a segment of the marker protein is produced by recombinant DNA techniques. Alternative to recombinant expression, such protein or peptide can be synthesized chemically using standard peptide synthesis techniques.

An "isolated" or "purified" protein or biologically active portion thereof is substantially free of cellular material or other contaminating proteins from the cell or tissue source from which the protein is derived, or substantially free of chemical precursors or other chemicals when chemically synthesized. The language "substantially free of cellular material" includes preparations of protein in which the protein is separated from cellular components of the cells from which it is isolated or recombinantly produced. Thus, protein that is substantially free of cellular material includes preparations of protein having less than about 30%, 20%, 10%, or 5% (by dry weight) of heterologous protein (also referred to herein as a "contaminating protein").

When the protein or biologically active portion thereof is recombinantly produced, it is also preferably substantially free of culture medium, i.e., culture medium represents less than about 20%, 10%, or 5% of the volume of the protein preparation. When the protein is produced by chemical synthesis, it is preferably substantially free of chemical precursors or other chemicals, i.e., it is separated from chemical precursors or other chemicals which are involved in the synthesis of the protein. Accordingly such preparations of the protein have less than about 30%, 20%, 10%, 5% (by dry weight) of chemical precursors or compounds other than the polypeptide of interest.

Biologically active portions of a marker protein include polypeptides comprising amino acid sequences sufficiently identical to or derived from the amino acid sequence of the marker protein, which include fewer amino acids than the full length protein, and exhibit at least one activity of the corresponding full-length protein. Typically, biologically active portions comprise a domain or motif with at least one activity of the corresponding full-length protein. A biologically active portion of a marker protein can be a polypeptide which is, for example, 10, 25, 50, 100 or more amino acids in length. Moreover, other biologically active portions, in which other regions of the marker protein are deleted, can be prepared by recombinant techniques and evaluated for one or more of the functional activities of the native form of the marker protein. In certain embodiments, useful proteins are substantially identical (e.g., at least about 40%, and in certain embodiments, 50%, 60%, 70%, 80%, 90%, 95%, or 99%) to one of these sequences and retain the functional activity of the corresponding naturally-occurring marker protein yet differ in amino acid sequence due to natural allelic variation or mutagenesis.

In addition, libraries of segments of a marker protein can be used to generate a variegated population of polypeptides for screening and subsequent selection of variant marker proteins or segments thereof.

### Predictive Medicine

There is also provided herein uses of the animal models and markers in the field of predictive medicine in which diagnostic assays, prognostic assays, pharmacogenomics, and monitoring clinical trials are used for prognostic (predictive) purposes to thereby treat an individual prophylactically. Accordingly, there is also provided herein diagnostic assays for determining the level of expression of one or more marker proteins or nucleic acids, in order to determine whether an individual is at risk of developing a particular disorder and/or disease. Such assays can be used for prognostic or predictive purposes to thereby prophylactically treat an individual prior to the onset of the disorder and/or disease.

In another aspect, the methods are useful for at least periodic screening of the same individual to see if that individual has been exposed to chemicals or toxins that change his/her expression patterns.

Yet another aspect pertains to monitoring the influence of agents (e.g., drugs or other compounds) administered either to inhibit a disorder and/or disease or to treat or prevent any other disorder (e.g., in order to understand any system effects that such treatment may have) on the expression or activity of a marker in clinical trials.

### Pharmaceutical Compositions

The compounds may be in a formulation for administration topically, locally or systemically in a suitable pharmaceutical carrier. Remington's Pharmaceutical Sciences, 15th Edition by E. W. Martin (Mark Publishing Company, 1975), discloses typical carriers and methods of preparation. The compound may also be encapsulated in suitable biocompatible microcapsules, microparticles or microspheres formed of biodegradable or non-biodegradable polymers or proteins or liposomes for targeting to cells. Such systems are well known to those skilled in the art and may be optimized for use with the appropriate nucleic acid.

Various methods for nucleic acid delivery are described, for example in Sambrook et al., 1989, Molecular Cloning: A Laboratory Manual, Cold Spring Harbor Laboratory, New York; and Ausubel et al., 1994, Current Protocols in Molecular Biology, John Wiley & Sons, New York. Such nucleic acid delivery systems comprise the desired nucleic acid, by way of example and not by limitation, in either "naked" form as a "naked" nucleic acid, or formulated in a vehicle suitable for delivery, such as in a complex with a cationic molecule or a liposome forming lipid, or as a component of a vector, or a component of a pharmaceutical composition. The nucleic acid delivery system can be provided to the cell either directly, such as by contacting it with the cell, or indirectly, such as through the action of any biological process.

Formulations for topical administration may include ointments, lotions, creams, gels, drops, suppositories, sprays, liquids and powders. Conventional pharmaceutical carriers, aqueous, powder or oily bases, or thickeners can be used as desired.

Formulations suitable for parenteral administration, such as, for example, by intraarticular (in the joints), intravenous, intramuscular, intradermal, intraperitoneal, and subcutaneous routes, include aqueous and non-aqueous, isotonic sterile injection solutions, which can contain antioxidants, buffers, bacteriostats, and solutes that render the formulation isotonic with the blood of the intended recipient, and aqueous and non-aqueous sterile suspensions, solutions or emulsions that can include suspending agents, solubilizers, thickening agents, dispersing agents, stabilizers, and preservatives. Formulations for injection may be presented in unit dosage form, e.g., in ampules or in multi-dose containers, with an added preservative. Those of skill in the art can readily determine the various parameters for preparing and formulating the compositions without resort to undue experimentation. The compound can be used alone or in combination with other suitable components.

In general, methods of administering compounds, including nucleic acids, are well known in the art. In particular, the routes of administration already in use for nucleic acid therapeutics, along with formulations in current use, provide preferred routes of administration and formulation for the nucleic acids selected will depend of course, upon factors such as the particular formulation, the severity of the state of the subject being treated, and the dosage required for therapeutic efficacy. As generally used herein, an "effective amount" is that amount which is able to treat one or more symptoms of the disorder, reverse the progression of one or more symptoms of the disorder, halt the progression of one or more symptoms of the disorder, or prevent the occurrence of one or more symptoms of the disorder in a subject to whom the formulation is administered, as compared to a matched subject not receiving the compound. The actual effective amounts of compound can vary according to the specific compound or combination thereof being utilized, the particular composition formulated, the mode of administration, and the age, weight, condition of the individual, and severity of the symptoms or condition being treated.

Any acceptable method known to one of ordinary skill in the art may be used to administer a formulation to the subject. The administration may be localized (i.e., to a particular region, physiological system, tissue, organ, or cell type) or systemic, depending on the condition being treated.

### Pharmacogenomics

The markers are also useful as pharmacogenomic markers. As used herein, a "pharmacogenomic marker" is an objective biochemical marker whose expression level correlates with a specific clinical drug response or susceptibility in a subject. The presence or quantity of the pharmacogenomic marker expression is related to the predicted response of the subject and more particularly the subject's tumor to therapy with a specific drug or class of drugs. By assessing the presence or quantity of the expression of one or more pharmacogenomic markers in a subject, a drug therapy which is most appropriate for the subject, or which is predicted to have a greater degree of success, may be selected.

### Monitoring Clinical Trials

Monitoring the influence of agents (e.g., drug compounds) on the level of expression of a marker can be applied not only in basic drug screening, but also in clinical trials. For example, the effectiveness of an agent to affect marker expression can be monitored in clinical trials of subjects receiving treatment for a cancer-related disease.

In one non-limiting embodiment, the present invention provides a method for monitoring the effectiveness of treatment of a subject with an agent (e.g., an agonist, antagonist, peptidomimetic, protein, peptide, nucleic acid, small molecule, or other drug candidate) comprising the steps of:
i) obtaining a pre-administration sample from a subject prior to administration of the agent;
ii) detecting the level of expression of one or more selected markers in the pre-administration sample;
iii) obtaining one or more post-administration samples from the subject;
iv) detecting the level of expression of the marker(s) in the post-administration samples;
v) comparing the level of expression of the marker(s) in the pre-administration sample with the level of expression of the marker(s) in the post-administration sample or samples; and
vi) altering the administration of the agent to the subject accordingly.

For example, increased expression of the marker gene(s) during the course of treatment may indicate ineffective dosage and the desirability of increasing the dosage. Conversely, decreased expression of the marker gene(s) may indicate efficacious treatment and no need to change dosage.

### Electronic Apparatus Readable Media, Systems, Arrays and Methods of Using Same

As used herein, "electronic apparatus readable media" refers to any suitable medium for storing, holding or containing data or information that can be read and accessed directly by an electronic apparatus. Such media can include, but are not limited to: magnetic storage media, such as floppy discs, hard disc storage medium, and magnetic tape; optical storage media such as compact disc; electronic storage media such as RAM, ROM, EPROM, EEPROM and the like; and general hard disks and hybrids of these categories such as magnetic/optical storage media. The medium is adapted or configured for having recorded thereon a marker as described herein.

As used herein, the term "electronic apparatus" is intended to include any suitable computing or processing apparatus or other device configured or adapted for storing data or information. Examples of electronic apparatus suitable for use with the present invention include stand-alone computing apparatus; networks, including a local area network (LAN), a wide area network (WAN) Internet, Intranet, and Extranet; electronic appliances such as personal digital assistants (PDAs), cellular phone, pager and the like; and local and distributed processing systems.

As used herein, "recorded" refers to a process for storing or encoding information on the electronic apparatus readable medium. Those skilled in the art can readily adopt any method for recording information on media to generate materials comprising the markers described herein.

A variety of software programs and formats can be used to store the marker information of the present invention on the electronic apparatus readable medium. Any number of data processor structuring formats (e.g., text file or database) may be employed in order to obtain or create a medium having recorded thereon the markers. By providing the markers in readable form, one can routinely access the marker sequence information for a variety of purposes. For example, one skilled in the art can use the nucleotide or amino acid sequences in readable form to compare a target sequence or target structural motif with the sequence information stored within the data storage means. Search means are used to identify fragments or regions of the sequences which match a particular target sequence or target motif.

Thus, there is also provided herein a medium for holding instructions for performing a method for determining whether a subject has a cancer-related disease or a pre-disposition to a cancer-related disease, wherein the method comprises the steps of determining the presence or absence of a marker and based on the presence or absence of the marker, determining whether the subject has a cancer-related disease or a pre-disposition to a cancer-related disease and/or recommending a particular treatment for a cancer-related disease or pre-cancer-related disease condition.

There is also provided herein an electronic system and/or in a network, a method for determining whether a subject has a cancer-related disease or a pre-disposition to a cancer-related disease associated with a marker wherein the method comprises the steps of determining the presence or absence of the marker, and based on the presence or absence of the marker, determining whether the subject has a particular disorder and/or disease or a pre-disposition to such disorder and/or disease, and/or recommending a particular treatment for such disease or disease and/or such pre-cancer-related disease condition. The method may further comprise the step of receiving phenotypic information associated with the subject and/or acquiring from a network phenotypic information associated with the subject.

Also provided herein is a network, a method for determining whether a subject has a disorder and/or disease or a pre-disposition to a disorder and/or disease associated with a marker, the method comprising the steps of receiving information associated with the marker, receiving phenotypic information associated with the subject, acquiring information from the network corresponding to the marker and/or disorder and/or disease, and based on one or more of the phenotypic information, the marker, and the acquired information, determining whether the subject has a disorder and/or disease or a pre-disposition thereto. The method may further comprise the step of recommending a particular treatment for the disorder and/or disease or pre-disposition thereto.

There is also provided herein a business method for determining whether a subject has a disorder and/or disease or a pre-disposition thereto, the method comprising the steps of receiving information associated with the marker, receiving phenotypic information associated with the subject, acquiring information from the network corresponding to the marker and/or a disorder and/or disease, and based on one or more of the phenotypic information, the marker, and the acquired information, determining whether the subject has a disorder and/or disease or a pre-disposition thereto. The method may further comprise the step of recommending a particular treatment therefor.

There is also provided herein an array that can be used to assay expression of one or more genes in the array. In one embodiment, the array can be used to assay gene expression in a tissue to ascertain tissue specificity of genes in the array. In this manner, up to about 7000 or more genes can be simultaneously assayed for expression. This allows a profile to be developed showing a battery of genes specifically expressed in one or more tissues.

In addition to such qualitative determination, there is provided herein the quantitation of gene expression. Thus, not only tissue specificity, but also the level of expression of a battery of genes in the tissue is ascertainable. Thus, genes can be grouped on the basis of their tissue expression per se and level of expression in that tissue. This is useful, for example, in ascertaining the relationship of gene expression between or among tissues. Thus, one tissue can be perturbed and the effect on gene expression in a second tissue can be determined. In this context, the effect of one cell type on another cell type in response to a biological stimulus can be determined.

Such a determination is useful, for example, to know the effect of cell-cell interaction at the level of gene expression. If an agent is administered therapeutically to treat one cell type but has an undesirable effect on another cell type, the method provides an assay to determine the molecular basis of the undesirable effect and thus provides the opportunity to co-administer a counteracting agent or otherwise treat the undesired effect. Similarly, even within a single cell type, undesirable biological effects can be determined at the molecular level. Thus, the effects of an agent on expression of other than the target gene can be ascertained and counteracted.

In another embodiment, the array can be used to monitor the time course of expression of one or more genes in the array. This can occur in various biological contexts, as disclosed herein, for example development of a disorder and/or disease, progression thereof, and processes, such as cellular transformation associated therewith.

The array is also useful for ascertaining the effect of the expression of a gene or the expression of other genes in the same cell or in different cells. This provides, for example, for a selection of alternate molecular targets for therapeutic intervention if the ultimate or downstream target cannot be regulated.

The array is also useful for ascertaining differential expression patterns of one or more genes in normal and abnormal cells. This provides a battery of genes that could serve as a molecular target for diagnosis or therapeutic intervention.

### Surrogate Markers

The markers may serve as surrogate markers for one or more disorders or disease states or for conditions leading up thereto. As used herein, a "surrogate marker" is an objective biochemical marker which correlates with the absence or presence of a disease or disorder, or with the progression of a disease or disorder. The presence or quantity of such markers is independent of the disease. Therefore, these markers may serve to indicate whether a particular course of treatment is effective in lessening a disease state or disorder. Surrogate markers are of particular use when the presence or extent of a disease state or disorder is difficult to assess through standard methodologies, or when an assessment of disease progression is desired before a potentially dangerous clinical endpoint is reached.

The markers are also useful as pharmacodynamic markers. As used herein, a "pharmacodynamic marker" is an objective biochemical marker which correlates specifically with drug effects. The presence or quantity of a pharmacodynamic marker is not related to the disease state or disorder for which the drug is being administered; therefore, the presence or quantity of the marker is indicative of the presence or activity of the drug in a subject. For example, a pharmacodynamic marker may be indicative of the concentration of the drug in a biological tissue, in that the marker is either expressed or transcribed or not expressed or transcribed in that tissue in relationship to the level of the drug. In this fashion, the distribution or uptake of the drug may be monitored by the pharmacodynamic marker. Similarly, the presence or quantity of the pharmacodynamic marker may be related to the presence or quantity of the metabolic product of a drug, such that the presence or quantity of the marker is indicative of the relative breakdown rate of the drug in vivo.

Pharmacodynamic markers are of particular use in increasing the sensitivity of detection of drug effects, particularly when the drug is administered in low doses. Since even a small amount of a drug may be sufficient to activate multiple rounds of marker transcription or expression, the amplified marker may be in a quantity which is more readily detectable than the drug itself. Also, the marker may be more easily detected due to the nature of the marker itself; for example, using the methods described herein, antibodies may be employed in an immune-based detection system for a protein marker, or marker-specific radiolabeled probes may be used to detect a mRNA marker. Furthermore, the use of a pharmacodynamic marker may offer mechanism-based prediction of risk due to drug treatment beyond the range of possible direct observations.

### Protocols for Testing

The method of testing for a disorder and/or disease may comprise, for example measuring the expression level of each marker gene in a biological sample from a subject over time and comparing the level with that of the marker gene in a control biological sample.

When the marker gene is one of the genes described herein and the expression level is differentially expressed (for examples, higher or lower than that in the control), the subject is judged to be affected with a disorder and/or disease. When the expression level of the marker gene falls within the permissible range, the subject is unlikely to be affected therewith.

The standard value for the control may be pre-determined by measuring the expression level of the marker gene in the control, in order to compare the expression levels. For example, the standard value can be determined based on the expression level of the above-mentioned marker gene in the control. For example, in certain embodiments, the permissible range is taken as ± 2S.D. based on the standard value. Once the standard value is determined, the testing method may be performed by measuring only the expression level in a biological sample from a subject and comparing the value with the determined standard value for the control.

Expression levels of marker genes include transcription of the marker genes to mRNA, and translation into proteins. Therefore, one method of testing for a disorder and/or disease is performed based on a comparison of the intensity of expression of mRNA corresponding to the marker genes, or the expression level of proteins encoded by the marker genes.

The measurement of the expression levels of marker genes in the testing for a disorder and/or disease can be carried out according to various gene analysis methods. Specifically, one can use, for example, a hybridization technique using nucleic acids that hybridize to these genes as probes, or a gene amplification technique using DNA that hybridize to the marker genes as primers.

The probes or primers used for the testing can be designed based on the nucleotide sequences of the marker genes. The identification numbers for the nucleotide sequences of the respective marker genes are described herein.

Further, it is to be understood that genes of higher animals generally accompany polymorphism in a high frequency. There are also many molecules that produce isoforms comprising mutually different amino acid sequences during the splicing process. Any gene associated with a cancer-related disease that has an activity similar to that of a marker gene is included in the marker genes, even if it has nucleotide sequence differences due to polymorphism or being an isoform.

It is also to be understood that the marker genes can include homologs of other species in addition to humans. Thus, unless otherwise specified, the expression "marker gene" refers to a homolog of the marker gene unique to the species or a foreign marker gene which has been introduced into an individual.

Also, it is to be understood that a "homolog of a marker gene" refers to a gene derived from a species other than a human, which can hybridize to the human marker gene as a probe under stringent conditions. Such stringent conditions are known to one skilled in the art who can select an appropriate condition to produce an equal stringency experimentally or empirically.

A polynucleotide comprising the nucleotide sequence of a marker gene or a nucleotide sequence that is complementary to the complementary strand of the nucleotide sequence of a marker gene and has at least 15 nucleotides, can be used as a primer or probe. Thus, a "complementary strand" means one strand of a double stranded DNA with respect to the other strand and which is composed of A:T (U for RNA) and G:C base pairs.

In addition, "complementary" means not only those that are completely complementary to a region of at least 15 continuous nucleotides, but also those that have a nucleotide sequence homology of at least 40% in certain instances, 50% in certain instances, 60% in certain instances, 70% in certain instances, 80% in certain instances, 90% in certain instances, and 95% in certain instances, or higher. The degree of homology between nucleotide sequences can be determined by an algorithm, BLAST, etc.

Such polynucleotides are useful as a probe to detect a marker gene, or as a primer to amplify a marker gene. When used as a primer, the polynucleotide comprises usually 15 bp to 100 bp, and in certain embodiments 15 bp to 35 bp of nucleotides. When used as a probe, a DNA comprises the whole nucleotide sequence of the marker gene (or the complementary strand thereof), or a partial sequence thereof that has at least 15 bp nucleotides. When used as a primer, the 3' region must be complementary to the marker gene, while the 5' region can be linked to a restriction enzyme-recognition sequence or a tag.

"Polynucleotides" may be either DNA or RNA. These polynucleotides may be either synthetic or naturally-occurring. Also, DNA used as a probe for hybridization is usually labeled. Those skilled in the art readily understand such labeling methods. Herein, the term "oligonucleotide" means a polynucleotide with a relatively low degree of polymerization. Oligonucleotides are included in polynucleotides.

Tests for a disorder and/or disease using hybridization techniques can be performed using, for example, Northern hybridization, dot blot hybridization, or the DNA microarray technique. Furthermore, gene amplification techniques, such as the RT-PCR method may be used. By using the PCR amplification monitoring method during the gene amplification step in RT-PCR, one can achieve a more quantitative analysis of the expression of a marker gene.

In the PCR gene amplification monitoring method, the detection target (DNA or reverse transcript of RNA) is hybridized to probes that are labeled with a fluorescent dye and a quencher which absorbs the fluorescence. When the PCR proceeds and Taq polymerase degrades the probe with its 5'-3' exonuclease activity, the fluorescent dye and the quencher draw away from each other and the fluorescence is detected. The fluorescence is detected in real time. By simultaneously measuring a standard sample in which the copy number of a target is known, it is possible to determine the copy number of the target in the subject sample with the cycle number where PCR amplification is linear. Also, one skilled in the art recognizes that the PCR amplification monitoring method can be carried out using any suitable method.

The method of testing for a cancer-related disease can be also carried out by detecting a protein encoded by a marker gene. Hereinafter, a protein encoded by a marker gene is described as a "marker protein." For such test methods, for example, the Western blotting method, the immunoprecipitation method, and the ELISA method may be employed using an antibody that binds to each marker protein.

Antibodies used in the detection that bind to the marker protein may be produced by any suitable technique. Also, in order to detect a marker protein, such an antibody may be appropriately labeled. Alternatively, instead of labeling the antibody, a substance that specifically binds to the antibody, for example, protein A or protein G, may be labeled to detect the marker protein indirectly. More specifically, such a detection method can include the ELISA method.

A protein or a partial peptide thereof used as an antigen may be obtained, for example, by inserting a marker gene or a portion thereof into an expression vector, introducing the construct into an appropriate host cell to produce a transformant, culturing the transformant to express the recombinant protein, and purifying the expressed recombinant protein from the culture or the culture supernatant. Alternatively, the amino acid sequence encoded by a gene or an oligopeptide comprising a portion of the amino acid sequence encoded by a full-length cDNA are chemically synthesized to be used as an immunogen.

Furthermore, a test for a cancer-related disease can be performed using as an index not only the expression level of a marker gene but also the activity of a marker protein in a biological sample. Activity of a marker protein means the biological activity intrinsic to the protein. Various methods can be used for measuring the activity of each protein.

Even if a subject is not diagnosed as being affected with a disorder and/or disease in a routine test in spite of symptoms suggesting these diseases, whether or not such a subject is suffering from a disorder and/or disease can be easily determined by performing a test according to the methods described herein.

More specifically, in certain embodiments, when the marker gene is one of the genes described herein, an increase or decrease in the expression level of the marker gene in a subject whose symptoms suggest at least a susceptibility to a disorder and/or disease indicates that the symptoms are primarily caused thereby.

In addition, the tests are useful to determine whether a disorder and/or disease is improving in a subject. In other words, the methods described herein can be used to judge the therapeutic effect of a treatment therefor. Furthermore, when the marker gene is one of the genes described herein, an increase or decrease in the expression level of the marker gene in a subject, who has been diagnosed as being affected thereby, implies that the disease has progressed more.

The severity and/or susceptibility to a disorder and/or disease may also be determined based on the difference in expression levels. For example, when the marker gene is one of the genes described herein, the degree of increase in the expression level of the marker gene is correlated with the presence and/or severity of a disorder and/or disease.

### Animal Models

Animal models for a disorder and/or disease where the expression level of one or more marker genes or a gene functionally equivalent to the marker gene has been elevated in the animal model can also be made. A "functionally equivalent gene" as used herein generally is a gene that encodes a protein having an activity similar to a known activity of a protein encoded by the marker gene. A representative example of a functionally equivalent gene includes a counterpart of a marker gene of a subject animal, which is intrinsic to the animal.

The animal model is useful for detecting physiological changes due to a disorder and/or disease. In certain embodiments, the animal model is useful to reveal additional functions of marker genes and to evaluate drugs whose targets are the marker genes.

An animal model can be created by controlling the expression level of a counterpart gene or administering a counterpart gene. The method can include creating an animal model by controlling the expression level of a gene selected from the group of genes described herein. In another embodiment, the method can include creating an animal model by administering the protein encoded by a gene described herein, or administering an antibody against the protein. It is to be also understood, that in certain other embodiments, the marker can be over-expressed such that the marker can then be measured using appropriate methods. In another embodiment, an animal model can be created by introducing a gene selected from such groups of genes, or by administering a protein encoded by such a gene. In another embodiment, a disorder and/or disease can be induced by suppressing the expression of a gene selected from such groups of genes or the activity of a protein encoded by such a gene. An antisense nucleic acid, a ribozyme, or an RNAi can be used to suppress the expression. The activity of a protein can be controlled effectively by administering a substance that inhibits the activity, such as an antibody.

The animal model is useful to elucidate the mechanism underlying a disorder and/or disease and also to test the safety of compounds obtained by screening. For example, when an animal model develops the symptoms of a particular disorder and/or disease, or when a measured value involved in certain a disorder and/or disease alters in the animal, a screening system can be constructed to explore compounds having activity to alleviate the disease.

As used herein, the expression "an increase in the expression level" refers to any one of the following: where a marker gene introduced as a foreign gene is expressed artificially; where the transcription of a marker gene intrinsic to the subject animal and the translation thereof into the protein are enhanced; or where the hydrolysis of the protein, which is the translation product, is suppressed.

As used herein, the expression "a decrease in the expression level" refers to either the state in which the transcription of a marker gene of the subject animal and the translation thereof into the protein are inhibited, or the state in which the hydrolysis of the protein, which is the translation product, is enhanced. The expression level of a gene can be determined, for example, by a difference in signal intensity on a DNA chip. Furthermore, the activity of the translation productthe protein--can be determined by comparing with that in the normal state.

It is also within the contemplated scope that the animal model can include transgenic animals, including, for example animals where a marker gene has been introduced and expressed artificially; marker gene knockout animals; and knock-in animals in which another gene has been substituted for a marker gene. A transgenic animal, into which an antisense nucleic acid of a marker gene, a ribozyme, a polynucleotide having an RNAi effect, or a DNA functioning as a decoy nucleic acid or such has been introduced, can be used as the transgenic animal. Such transgenic animals also include, for example, animals in which the activity of a marker protein has been enhanced or suppressed by introducing a mutation(s) into the coding region of the gene, or the amino acid sequence has been modified to become resistant or susceptible to hydrolysis. Mutations in an amino acid sequence include substitutions, deletions, insertions, and additions.

### Examples of Expression

In addition, the expression itself of a marker gene can be controlled by introducing a mutation(s) into the transcriptional regulatory region of the gene. Those skilled in the art understand such amino acid substitutions. Also, the number of amino acids that are mutated is not particularly restricted, as long as the activity is maintained. Normally, it is within 50 amino acids, in certain nonlimiting embodiments, within 30 amino acids, within 10 amino acids, or within 3 amino acids. The site of mutation may be any site, as long as the activity is maintained.

In yet another aspect, there is provided herein screening methods for candidate compounds for therapeutic agents to treat a particular disorder and/or disease. One or more marker genes are selected from the group of genes described herein. A therapeutic agent for a cancer-related disease can be obtained by selecting a compound capable of increasing or decreasing the expression level of the marker gene(s).

It is to be understood that the expression "a compound that increases the expression level of a gene" refers to a compound that promotes any one of the steps of gene transcription, gene translation, or expression of a protein activity. On the other hand, the expression "a compound that decreases the expression level of a gene", as used herein, refers to a compound that inhibits any one of these steps.

In particular aspects, the method of screening for a therapeutic agent for a disorder and/or disease can be carried out either in vivo or in vitro. This screening method can be performed, for example, by:
1) administering a candidate compound to an animal subject;
2) measuring the expression level of a marker gene(s) in a biological sample from the animal subject; or
3) selecting a compound that increases or decreases the expression level of a marker gene(s) as compared to that in a control with which the candidate compound has not been contacted.

In still another aspect, there is provided herein a method to assess the efficacy of a candidate compound for a pharmaceutical agent on the expression level of a marker gene(s) by contacting an animal subject with the candidate compound and monitoring the effect of the compound on the expression level of the marker gene(s) in a biological sample derived from the animal subject. The variation in the expression level of the marker gene(s) in a biological sample derived from the animal subject can be monitored using the same technique as used in the testing method described above. Furthermore, based on the evaluation, a candidate compound for a pharmaceutical agent can be selected by screening.

### Certain Nucleobase Sequences

Nucleobase sequences of mature miRNAs and their corresponding stem-loop sequences described herein are the sequences found in miRBase, an online searchable database of miRNA sequences and annotation, found at http://microrna.sanger.ac.uk/. Entries in the miRBase Sequence database represent a predicted hairpin portion of a miRNA transcript (the stem-loop), with information on the location and sequence of the mature miRNA sequence. The miRNA stem-loop sequences in the database are not strictly precursor miRNAs (pre-miRNAs), and may in some instances include the pre-miRNA and some flanking sequence from the presumed primary transcript. The miRNA nucleobase sequences described herein encompass any version of the miRNA, including the sequences described in Release 10.0 of the miRBase sequence database and sequences described in any earlier Release of the miRBase sequence database. A sequence database release may result in the re-naming of certain miRNAs. A sequence database release may result in a variation of a mature miRNA sequence. The compounds that may encompass such modified oligonucleotides may be complementary to any nucleobase sequence version of the miRNAs described herein.

It is understood that any nucleobase sequence set forth herein is independent of any modification to a sugar moiety, an internucleoside linkage, or a nucleobase. It is further understood that a nucleobase sequence comprising U's also encompasses the same nucleobase sequence wherein 'U' is replaced by 'T' at one or more positions having 'U'. Conversely, it is understood that a nucleobase sequence comprising T's also encompasses the same nucleobase sequence wherein 'T' is replaced by 'U'' at one or more positions having 'T'.

In certain embodiments, a modified oligonucleotide has a nucleobase sequence that is complementary to a miRNA or a precursor thereof, meaning that the nucleobase sequence of a modified oligonucleotide is a least 60%, 65%, 70%, 75%, 80%, 85%, 90%, 95%, 97%, 98% or 99% identical to the complement of a miRNA or precursor thereof over a region of 8, 9, 10, 11, 12, 13, 14, 15, 16, 17, 18, 19, 20, 21, 22, 23, 24, 25, 30, 35, 40, 45, 50, 55, 60, 65, 70, 75, 80, 85, 90, 95, 100 or more nucleobases, or that the two sequences hybridize under stringent hybridization conditions. Accordingly, in certain embodiments the nucleobase sequence of a modified oligonucleotide may have one or more mismatched basepairs with respect to its target miRNA or target miRNA precursor sequence, and is capable of hybridizing to its target sequence. In certain embodiments, a modified oligonucleotide has a nucleobase sequence that is 100% complementary to a miRNA or a precursor thereof. In certain embodiments, the nucleobase sequence of a modified oligonucleotide has full-length complementary to a miRNA.

### miRNA (miR) Therapies

In some embodiments, the present invention provides microRNAs that inhibit the expression of one or more genes in a subject. MicroRNA expression profiles can serve as a new class of cancer biomarkers.

Included herein are methods of inhibiting gene expression and/or activity using one or more MiRs. In some embodiments, the miR(s) inhibit the expression of a protein. In other embodiments, the miRNA(s) inhibits gene activity (e.g., cell invasion activity).

The miRNA can be isolated from cells or tissues, recombinantly produced, or synthesized in vitro by a variety of techniques well known to one of ordinary skill in the art. In one embodiment, miRNA is isolated from cells or tissues. Techniques for isolating miRNA from cells or tissues are well known to one of ordinary skill in the art. For example, miRNA can be isolated from total RNA using the mirVana miRNA isolation kit from Ambion, Inc. Another technique utilizes the flashIPAGE™ Fractionator System (Ambion, Inc.) for PAGE purification of small nucleic acids.

For the use of miRNA therapeutics, it is understood by one of ordinary skill in the art that nucleic acids administered in vivo are taken up and distributed to cells and tissues.

The nucleic acid may be delivered in a suitable manner which enables tissue-specific uptake of the agent and/or nucleic acid delivery system. The formulations described herein can supplement treatment conditions by any known conventional therapy, including, but not limited to, antibody administration, vaccine administration, administration of cytotoxic agents, natural amino acid polypeptides, nucleic acids, nucleotide analogues, and biologic response modifiers. Two or more combined compounds may be used together or sequentially.

Certain embodiments of the invention provide pharmaceutical compositions containing (a) one or more nucleic acid or small molecule compounds and (b) one or more other chemotherapeutic agents.

### Additional Useful Definitions

"Subject" means a human or non-human animal selected for treatment or therapy. "Subject suspected of having" means a subject exhibiting one or more clinical indicators of a disorder, disease or condition.

"Preventing" or "prevention" refers to delaying or forestalling the onset, development or progression of a condition or disease for a period of time, including weeks, months, or years. "Treatment" or "treat" means the application of one or more specific procedures used for the cure or amelioration of a disorder and/or disease. In certain embodiments, the specific procedure is the administration of one or more pharmaceutical agents.

"Amelioration" means a lessening of severity of at least one indicator of a condition or disease. In certain embodiments, amelioration includes a delay or slowing in the progression of one or more indicators of a condition or disease. The severity of indicators may be determined by subjective or objective measures which are known to those skilled in the art.

"Subject in need thereof" means a subject identified as in need of a therapy or treatment.

"Administering" means providing a pharmaceutical agent or composition to a subject, and includes, but is not limited to, administering by a medical professional and self-administering.

"Parenteral administration" means administration through injection or infusion. Parenteral administration includes, but is not limited to, subcutaneous administration, intravenous administration, intramuscular administration, intraarterial administration, and intracranial administration. "Subcutaneous administration" means administration just below the skin.

"Improves function" means the changes function toward normal parameters. In certain embodiments, function is assessed by measuring molecules found in a subject's bodily fluids. Pharmaceutical composition" means a mixture of substances suitable for administering to an individual that includes a pharmaceutical agent. For example, a pharmaceutical composition may comprise a modified oligonucleotide and a sterile aqueous solution.

"Target nucleic acid," "target RNA," "target RNA transcript" and "nucleic acid target" all mean a nucleic acid capable of being targeted by antisense compounds. Targeting" means the process of design and selection of nucleobase sequence that will hybridize to a target nucleic acid and induce a desired effect. "Targeted to" means having a nucleobase sequence that will allow hybridization to a target nucleic acid to induce a desired effect. In certain embodiments, a desired effect is reduction of a target nucleic acid.

"Modulation" means to a perturbation of function or activity. In certain embodiments, modulation means an increase in gene expression. In certain embodiments, modulation means a decrease in gene expression.

"Expression" means any functions and steps by which a gene's coded information is converted into structures present and operating in a cell.

"Region" means a portion of linked nucleosides within a nucleic acid. In certain embodiments, a modified oligonucleotide has a nucleobase sequence that is complementary to a region of a target nucleic acid. For example, in certain such embodiments a modified oligonucleotide is complementary to a region of a miRNA stem-loop sequence. In certain such embodiments, a modified oligonucleotide is 100% identical to a region of a miRNA sequence.

"Segment" means a smaller or sub-portion of a region.

"Nucleobase sequence" means the order of contiguous nucleobases, in a 5' to 3' orientation, independent of any sugar, linkage, and/or nucleobase modification.

"Contiguous nucleobases" means nucleobases immediately adjacent to each other in a nucleic acid.

"Nucleobase complementarity" means the ability of two nucleobases to pair non-covalently via hydrogen bonding. "Complementary" means a first nucleobase sequence is at least 60%, 65%, 70%, 75%, 80%, 85%, 90%, 95%, 97%, 98% or 99% identical, or is 100% identical, to the complement of a second nucleobase sequence over a region of 8, 9, 10, 11, 12, 13, 14, 15, 16, 17, 18, 19, 20, 21, 22, 23, 24, 25, 30, 35, 40, 45, 50, 55, 60, 65, 70, 75, 80, 85, 90, 95, 100 or more nucleobases, or that the two sequences hybridize under stringent hybridization conditions. In certain embodiments a modified oligonucleotide that has a nucleobase sequence which is 100% complementary to a miRNA, or precursor thereof, may not be 100% complementary to the miRNA, or precursor thereof, over the entire length of the modified oligonucleotide.

"Complementarity" means the nucleobase pairing ability between a first nucleic acid and a second nucleic acid. "Full-length complementarity" means each nucleobase of a first nucleic acid is capable of pairing with each nucleobase at a corresponding position in a second nucleic acid. For example, in certain embodiments, a modified oligonucleotide wherein each nucleobase has complementarity to a nucleobase in an miRNA has full-length complementarity to the miRNA.

"Percent complementary" means the number of complementary nucleobases in a nucleic acid divided by the length of the nucleic acid. In certain embodiments, percent complementarity of a modified oligonucleotide means the number of nucleobases that are complementary to the target nucleic acid, divided by the number of nucleobases of the modified oligonucleotide. In certain embodiments, percent complementarity of a modified oligonucleotide means the number of nucleobases that are complementary to a miRNA, divided by the number of nucleobases of the modified oligonucleotide.

"Percent region bound" means the percent of a region complementary to an oligonucleotide region. Percent region bound is calculated by dividing the number of nucleobases of the target region that are complementary to the oligonucleotide by the length of the target region. In certain embodiments, percent region bound is at least 80%, at least 85%, at least 90%, at least 95%, at least 96%, at least 97%, at least 98%, at least 99%, or 100%.

"Percent identity" means the number of nucleobases in first nucleic acid that are identical to nucleobases at corresponding positions in a second nucleic acid, divided by the total number of nucleobases in the first nucleic acid.

"Substantially identical" used herein may mean that a first and second nucleobase sequence are at least 60%, 65%, 70%, 75%, 80%, 85%, 90%, 95%, 97%, 98% or 99% identical, or 100% identical, over a region of 8, 9, 10, 11, 12, 13, 14, 15, 16, 17, 18, 19, 20, 21, 22, 23, 24, 25, 30, 35, 40, 45, 50, 55, 60, 65, 70, 75, 80, 85, 90, 95, 100 or more nucleobases.

"Hybridize" means the annealing of complementary nucleic acids that occurs through nucleobase complementarity.

"Mismatch" means a nucleobase of a first nucleic acid that is not capable of pairing with a nucleobase at a corresponding position of a second nucleic acid.

"Non-complementary nucleobase" means two nucleobases that are not capable of pairing through hydrogen bonding.

"Identical" means having the same nucleobase sequence.

"miRNA" or "miR" means a non-coding RNA between 18 and 25 nucleobases in length which hybridizes to and regulates the expression of a coding RNA. In certain embodiments, a miRNA is the product of cleavage of a pre-miRNA by the enzyme Dicer. Examples of miRNAs are found in the miRNA database known as miRBase (http://microrna.sanger.ac.uk/).

"Pre-miRNA" or "pre-miR" means a non-coding RNA having a hairpin structure, which contains a miRNA. In certain embodiments, a pre-miRNA is the product of cleavage of a pri-miR by the double-stranded RNA-specific ribonuclease known as Drosha.

"Stem-loop sequence" means an RNA having a hairpin structure and containing a mature miRNA sequence. Pre-miRNA sequences and stem-loop sequences may overlap. Examples of stem-loop sequences are found in the miRNA database known as miRBase (microrna.sanger.ac.uk/.

"miRNA precursor" means a transcript that originates from a genomic DNA and that comprises a non-coding, structured RNA comprising one or more miRNA sequences. For example, in certain embodiments a miRNA precursor is a pre-miRNA. In certain embodiments, a miRNA precursor is a pri-miRNA.

"Antisense compound" means a compound having a nucleobase sequence that will allow hybridization to a target nucleic acid. In certain embodiments, an antisense compound is an oligonucleotide having a nucleobase sequence complementary to a target nucleic acid.

"Oligonucleotide" means a polymer of linked nucleosides, each of which can be modified or unmodified, independent from one another. "Naturally occurring internucleoside linkage" means a 3' to 5' phosphodiester linkage between nucleosides. "Natural nucleobase" means a nucleobase that is unmodified relative to its naturally occurring form. "miR antagonist"+ means an agent designed to interfere with or inhibit the activity of a miRNA. In certain embodiments, a miR antagonist comprises an antisense compound targeted to a miRNA. In certain embodiments, a miR antagonist comprises a modified oligonucleotide having a nucleobase sequence that is complementary to the nucleobase sequence of a miRNA, or a precursor thereof. In certain embodiments, an miR antagonist comprises a small molecule, or the like that interferes with or inhibits the activity of an miRNA.

## Claims

1. Use of an isolated miR-32 gene product comprising a miR antagonist for increasing the responsiveness of prostate cancer cells to apoptotic stimuli, wherein the miR antagonist comprises an oligonucleotide sequence complementary to miR-32, or a precursor thereof.

2. Use of the isolated miR-32 gene product according to claim 1, in a pharmaceutical composition, wherein the pharmaceutical composition further comprises at least one anticancer agent selected from one or more of doxorubicin and etoposide.

3. Use of the isolated miR-32 gene product according to claim 1 or 2, wherein the prostate cancer cells are mammalian.

4. Use of the isolated miR-32 gene product according to claim 3, wherein the prostate cancer cells are human.

## Patentansprüche

1. Verwendung eines isolierten miR-32-Genprodukts, das einen miR-Antagonisten umfasst, zur Steigerung des Ansprechens von Prostatakrebszellen auf apoptotische Stimuli, wobei der miR-Antagonist eine Oligonukleotidsequenz umfasst, die zu miR-32 oder einem Vorläufer davon komplementär ist.

2. Verwendung des isolierten miR-32-Genprodukts nach Anspruch 1 in einer pharmazeutischen Zusammensetzung, wobei die pharmazeutische Zusammensetzung weiterhin mindestens ein Krebsmittel umfasst, das aus einem oder mehreren von Doxorubicin und Etoposid ausgewählt ist.

3. Verwendung des isolierten miR-32-Genprodukts nach Anspruch 1 oder 2, wobei die Prostatakrebszellen von einem Säugetier stammen.

4. Verwendung des isolierten miR-32-Genprodukts nach Anspruch 3, wobei die Prostatakrebszellen von einem Menschen stammen.

## Revendications

1. Utilisation d'un produit génique miR-32 isolé comprenant un antagoniste de miR pour l'augmentation de la sensibilité de cellules cancéreuses de la prostate à des stimuli apoptotiques, dans laquelle l'antagoniste de miR comprend une séquence oligonucléotidique complémentaire à miR-32 ou à un précurseur de celui-ci.

2. Utilisation du produit génique miR-32 isolé selon la revendication 1, dans une composition pharmaceutique, dans laquelle la composition pharmaceutique comprend en outre au moins un agent anticancéreux choisi parmi un ou plusieurs agents anticancéreux parmi la doxorubicine et l'étoposide.

3. Utilisation du produit génique miR-32 isolé selon la revendication 1 ou 2, dans laquelle les cellules cancéreuses de la prostate sont de mammifère.

4. Utilisation du produit génique miR-32 isolé selon la revendication 3, dans laquelle les cellules cancéreuses de la prostate sont humaines.
